# EUROPÄISCHE PATENTSCHRIFT

(11) **EP 3 052 477 B1**
(45) Veröffentlichungstag und Bekanntmachung des Hinweises auf die Patenterteilung: **24.10.2018**
(21) Anmeldenummer: 14761793.0
(22) Anmeldetag: 05.09.2014
(51) Int. Cl.: C07D 221/10, C07D 401/04, C07D 401/10, C07D 401/12, C07D 401/14, C07D 405/10, C07D 405/12, C07D 405/14, C07D 409/14, C07D 413/04, C07D 417/04, C07D 471/04, C07D 487/04, C09K 11/06, H05B 33/14

(54) **TRIARYLAMIN-SUBSTITUIERTE BENZO[H]CHINOLIN-DERIVATE ALS MATERIALIEN FÜR ELEKTRONISCHE VORRICHTUNGEN**
TRIARYLAMINE SUBSTITUTED BENZO[H]QUINOLINE DERIVATIVES AS MATERIALS FOR ELECTRONIC DEVICES
DÉRIVÉS BENZO[H]QUINOLINE SUBSTITUÉS PAR TRIARYLAMINE EN TANT QUE MATÉRIAUX POUR DISPOSITIFS ÉLECTRONIQUES

(30) Priorität: 04.10.2013 EP 13004785
(43) Veröffentlichungstag der Anmeldung: 10.08.2016
(73) Patentinhaber: Merck Patent GmbH, 64293 Darmstadt (DE)
(72) Erfinder: PARHAM, Amir Hossain, 60486 Frankfurt am Main (DE); STOESSEL, Philipp, 60389 Frankfurt am Main (DE); EBERLE, Thomas, 76829 Landau (DE); JATSCH, Anja, 60489 Frankfurt am Main (DE); KROEBER, Jonas Valentin, 60311 Frankfurt am Main (DE); PFLUMM, Christof, 64291 Darmstadt (DE); MARTYNOVA, Irina, 64347 Griesheim (DE); VOGES, Frank, 67098 Bad Duerkheim (DE); STIEBER, Frank, 64683 Einhausen (DE)
(86) Internationale Anmeldenummer: PCT/EP2014/002416
(87) Internationale Veröffentlichungsnummer: WO 2015/049022

(56) Entgegenhaltungen:
- WO-A2-2010/036036
- JP-A- 2003 282 270

## Beschreibung

Die vorliegende Anmeldung betrifft heteroaromatische Analoga des Phenanthrolins, gemäß einer unten definierten Formel (I). Die Verbindungen eignen sich unter anderem als Funktionsmaterialien in elektronischen Vorrichtungen, insbesondere in organischen Elektrolumineszenzvorrichtungen (OLEDs).

Unter elektronischen Vorrichtungen im Sinne dieser Anmeldung werden sogenannte organische elektronische Vorrichtungen verstanden (organic electronic devices), welche organische Verbindungen als Funktionsmaterialien enthalten. Insbesondere werden darunter OLEDs verstanden.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion, wie beispielsweise lochinjizierende Schichten, Lochtransportschichten, Elektronenblockierschichten und emittierende Schichten. Zur Verwendung in diesen Schichten werden kontinuierlich neue Materialien mit lochtransportierenden Eigenschaften gesucht, wie beispielsweise Lochinjektionsmaterialien, Lochtransportmaterialien, Elektronenblockiermaterialien und Matrixmaterialien zur Verwendung in Kombination mit Emittermaterialien, insbesondere phosphoreszierenden Emittermaterialien.

Hierfür werden im Stand der Technik unter anderem Mono-Triarylamine (vgl. JP 1995/053955, WO 2006/123667 und JP 2010/222268) und Bis- oder höherwertige Amine (vgl. US 7504163 und US 2005/0184657) beschrieben. Weiterhin sind hierfür Carbazol-Verbindungen bekannt, wie beispielsweise Bis-carbazolyl-biphenyl (CBP) und die in WO 2008/086851, US 2005/0221124 A1, EP 2202818 A1 und WO 2013/060418 beschriebenen Verbindungen. Weiterhin sind hierfür verbrückte Triarylamin-Verbindungen bekannt, wie beispielsweise in WO 2007/031165, WO 2010/083871, WO 2011/088877, WO 2011/107186 und WO 2011/128017 beschrieben. Weiterhin offenbaren WO 2010/036036 A2 und JP 2003-282270 A Benzochinolin-Derivate zur Verwendung in OLEDs.

Es besteht jedoch unverändert Bedarf an alternativen Verbindungen für diese Verwendung.

Es wurde nun gefunden, dass Arylamine, verbrückte Arylamine und Carbazol-Derivate, welche dadurch gekennzeichnet sind, dass sie eine Benzo[h]chinolin-Einheit aufweisen, hervorragend zur Verwendung in OLEDs geeignet sind.

Bevorzugt weisen diese Verbindungen eine oder mehrere vorteilhafte Eigenschaften gewählt aus sehr guten lochleitenden Eigenschaften und hoher Temperatur- und Oxidationsstabilität auf. Insbesondere weisen die Verbindungen ein energetisch relativ hochliegendes HOMO auf, verglichen mit anderen Verbindungen ähnlicher Struktur, und sind daher hervorragende Lochleiter. Bei der Verwendung in OLEDs werden mit den Verbindungen bevorzugt eine hohe Lebensdauer und eine geringe Betriebsspannung erzielt.

Gegenstand der vorliegenden Anmeldung sind somit Verbindungen der Formel (I)

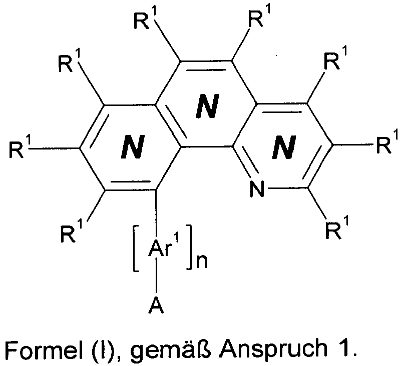

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von den oben unter Aryl- und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt umfasst die Verbindung der Formel (I) zusätzlich zur Gruppe A keine weitere Arylaminogruppe, keine weitere verbrückte Arylaminogruppe und keine weitere Carbazolgruppe. Unter verbrückten Arylaminogruppen sind dabei Gruppen entsprechend Gruppen der Formel (A-1) oder (A-2) zu verstehen, in denen wenigstens eine Brücke X vorhanden ist, die keine Einfachbindung darstellt.

Weiterhin ist es bevorzugt, dass die Verbindung der Formel (I) als Substituent R¹, R², R³ und R⁴ keine kondensierte Aryl- oder Heteroarylgruppe mit mehr als 14 aromatischen Ringatomen aufweist, besonders bevorzugt keine kondensierte Aryl- oder Heteroarylgruppe mit mehr als 10 aromatischen Ringatomen aufweist.

Für die Verbindungen gemäß Formel (I) ist es bevorzugt, dass in den Benzolringen, in deren Mitte ein N gezeichnet ist, keines oder genau ein oder zwei Ringglieder -CR¹= durch -N= ersetzt sind, besonders bevorzugt keines oder genau eines, ganz besonders bevorzugt keines.

Gemäß einer bevorzugten Ausführungsform der Erfindung sind im mittleren der drei Benzolringe in Formel (I) genau eine oder genau zwei Ringglieder -CR¹= durch -N= ersetzt, und in den beiden äußeren Ringen ist kein Ringglied -CR¹= durch -N= ersetzt.

Gemäß einer alternativen bevorzugten Ausführungsform der Erfindung ist in allen drei Benzolringen in Formel (I), in deren Mitte ein N gezeichnet ist, kein Ringglied -CR¹= durch N ersetzt.

Es ist bevorzugt, dass n gleich 0, 1 oder 2 ist, besonders bevorzugt gleich 0 oder 1, ganz besonders bevorzugt gleich 0.

Ganz besonders bevorzugt ist die Einheit-(Ar¹)ₙ- bei jedem Auftreten gleich oder verschieden gewählt aus Gruppen gemäß den folgenden Formeln (L-1) bis (L-18):

| | |
|---|---|
| 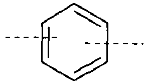 | 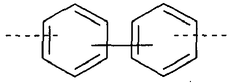 |
| Formel (L-1) | Formel (L-2) |
| 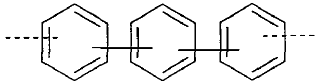 | 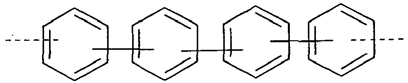 |
| Formel (L-3) | Formel (L-4) |
| | |
| 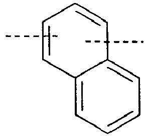 | 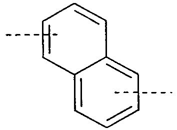 |
| Formel (L-5) | Formel (L-6) |
| | |
| 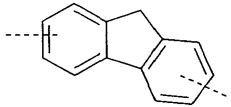 | 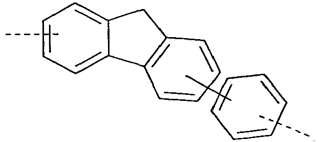 |
| Formel (L-7) | Formel (L-8) |
| | |
| 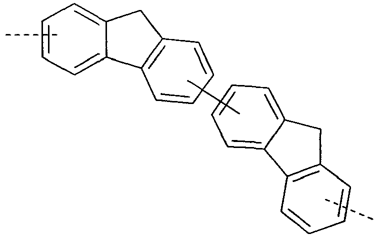 | 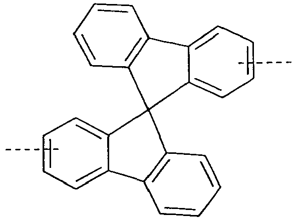 |
| Formel (L-9) | Formel (L-10) |
| | |
| 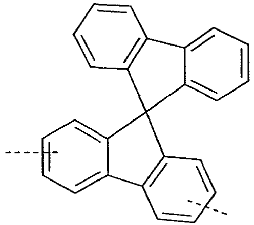 | 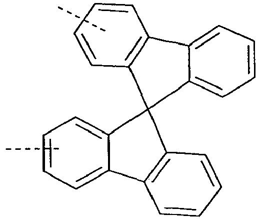 |
| Formel (L-11) | Formel (L-12) |
| 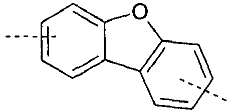 | 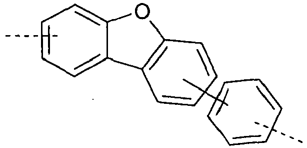 |
| Formel (L-13) | Formel (L-14) |
| | |
| 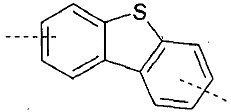 | 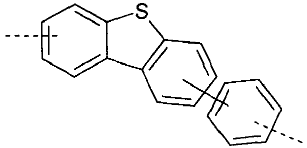 |
| Formel (L-15) | Formel (L-16) |
| | |
| 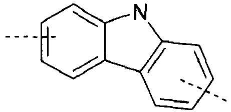 | 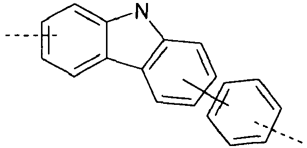 |
| Formel (L-17) | Formel (L-18) |

wobei die Gruppen an den freien Positionen jeweils mit Resten R¹ substituiert sein können und wobei die gestrichelten Linien die Bindungen an den Rest der Verbindung kennzeichnen.

R² ist bevorzugt bei jedem Auftreten gleich oder verschieden H, D, F, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, - R³C=CR³-, Si(R³)₂, C=O, C=NR³, -NR³-, -O-, -S-, -C(=O)O- oder-C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring bilden können.

Gemäß einer bevorzugten Ausführungsform bilden zwei Gruppen R², die an dasselbe Atom einer Gruppe X binden, beispielsweise an das C-Atom einer Gruppe X, die C(R²)₂ ist, miteinander einen Ring. Besonders bevorzugt bilden sie in diesem Fall einen Cycloalkylring, beispielsweise einen Cyclopentyl- oder einen Cyclohexylring.

R³ ist bevorzugt bei jedem Auftreten gleich oder verschieden H, D, F, CN, Si(R⁴)₃, N(R⁴)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, - R⁴C=CR⁴-, Si(R⁴)₂, C=O, C=NR⁴, -NR⁴-, -O-, -S-, -C(=O)O- oder - C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Für Gruppen der Formel (A-2) ist es bevorzugt, dass keine, genau eine oder genau zwei Indices i gleich 1 sind, und die anderen Indices i gleich 0 sind.

Weiterhin ist es bevorzugt, dass nicht mehr als zwei Gruppen X in einer Gruppe der Formel (A-2) vorliegen, die eine Einfachbindung darstellen.

Es ist bevorzugt, dass X bei jedem Auftreten gleich oder verschieden gewählt ist aus einer Einfachbindung oder einer Gruppe gewählt aus C(R²)₂, -C(R²)₂-C(R²)₂-, -C(R²)=C(R²)-, C=O, NR², O und S. Ganz besonders bevorzugt ist X bei jedem Auftreten gleich oder verschieden gewählt aus einer Einfachbindung oder einer Gruppe gewählt aus C(R²)₂, C=O, NR², O und S.

Bevorzugt ist Ar² bei jedem Auftreten gleich oder verschieden gewählt aus Benzol, Biphenyl, Terphenyl, Quaterphenyl, Fluoren, Spirobifluoren, Indenofluoren, Carbazol, Indenocarbazol, Indolocarbazol, Dibenzofuran und Dibenzothiophen, die jeweils mit einem oder mehreren Resten R¹ substituiert sein können.

Bevorzugte Ausführungsformen der Gruppen der Formel (A-1) sind die folgenden Formeln (A-1-1) bis (A-1-30)

| | |
|---|---|
| 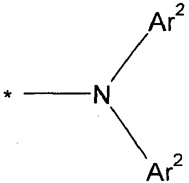 | 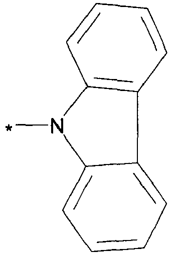 |
| Formel (A-1-1) | Formel (A-1-2) |
| 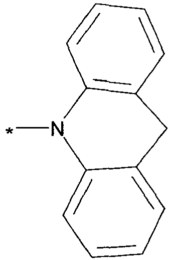 | 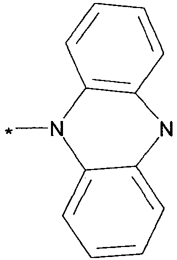 |
| Formel (A-1-3) | Formel (A-1-4) |
| | |
| 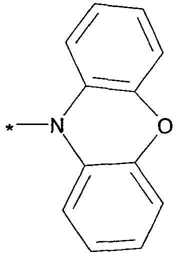 | 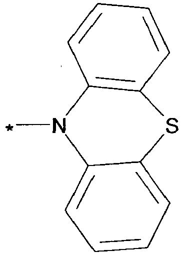 |
| Formel (A-1-5) | Formel (A-1-6) |
| | |
| 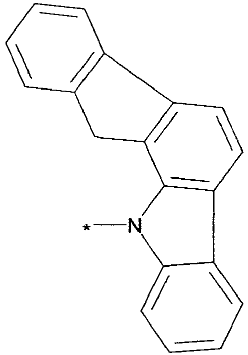 | 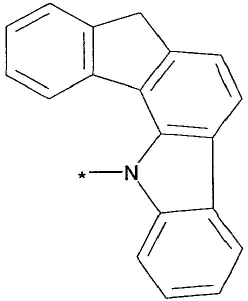 |
| Formel (A-1-7) | Formel (A-1-8) |
| 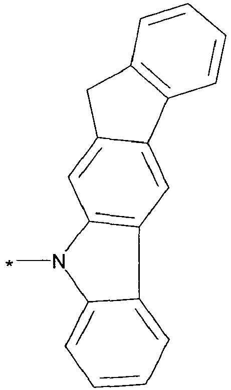 | 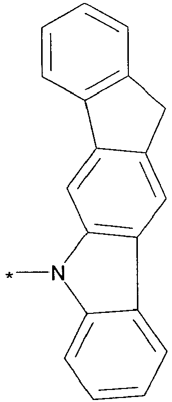 |
| Formel (A-1-9) | Formel (A-1-10) |
| | |
| 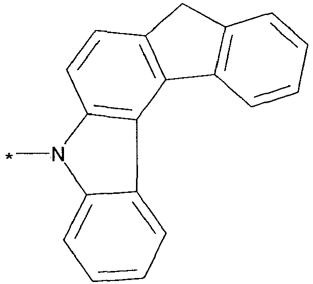 | 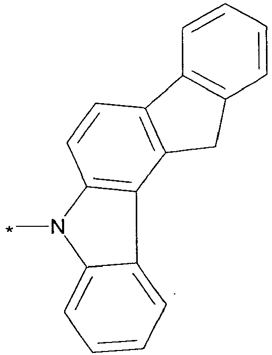 |
| Formel (A-1-11) | Formel (A-1-12) |
| | |
| 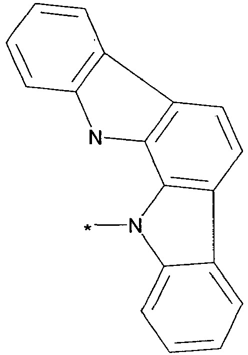 | 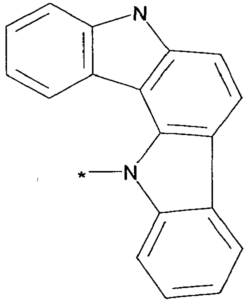 |
| Formel (A-1-13) | Formel (A-1-14) |
| 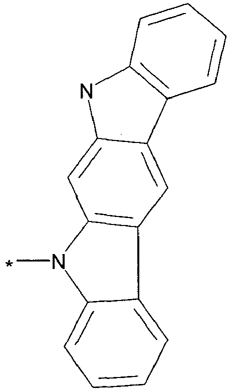 | 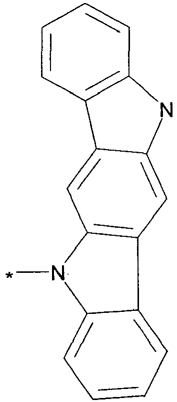 |
| Formel (A-1-15) | Formel (A-1-16) |
| | |
| 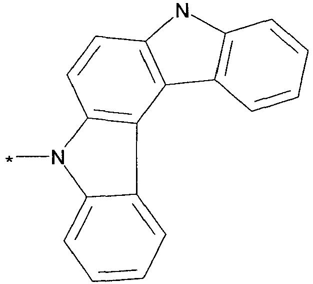 | 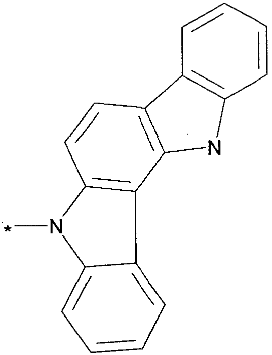 |
| Formel (A-1-17) | Formel (A-1-18) |
| 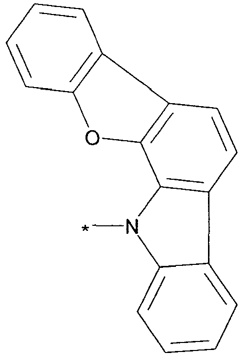 | 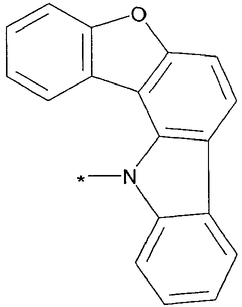 |
| Formel (A-1-19) | Formel (A-1-20) |
| | |
| 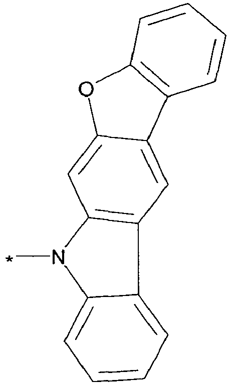 | 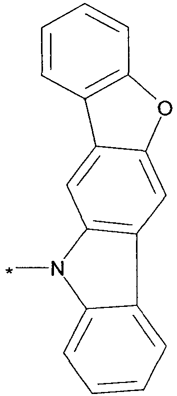 |
| Formel (A-1-21) | Formel (A-1-22) |
| | |
| 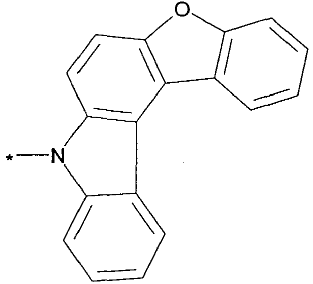 | 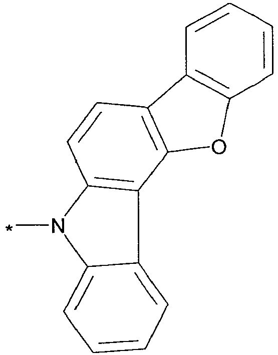 |
| Formel (A-1-23) | Formel (A-1-24) |
| 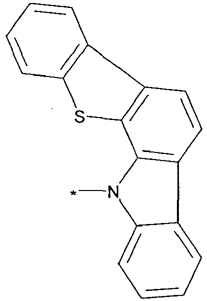 | 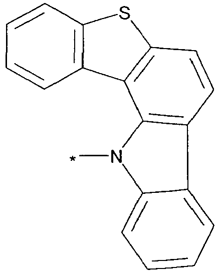 |
| Formel (A-1-25) | Formel (A-1-26) |
| | |
| 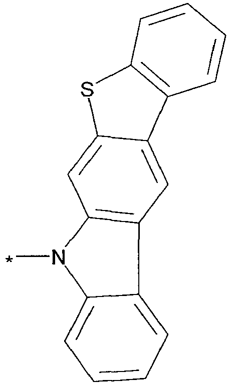 | 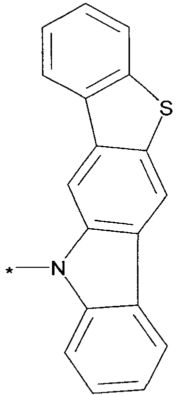 |
| Formel (A-1-27) | Formel (A-1-28) |
| | |
| 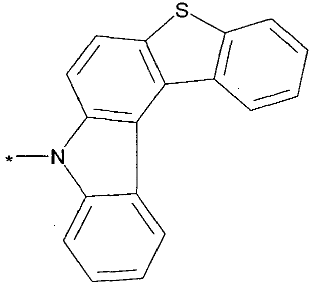 | 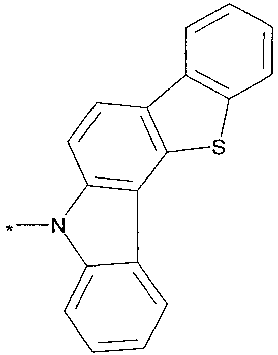 |
| Formel (A-1-29) | Formel (A-1-30) |

welche an den freien Positionen jeweils mit Resten R¹ substituiert sein können. Für die Formel (A-1-1) ist es bevorzugt, dass Ar² bei jedem Auftreten gleich oder verschieden gewählt ist aus Benzol, Biphenyl, Terphenyl, Quaterphenyl, Fluoren, Spirobifluoren, Indenofluoren, Carbazol, Indenocarbazol, Indolocarbazol, Dibenzofuran und Dibenzothiophen, die jeweils mit einem oder mehreren Resten R¹ substituiert sein können.

Bevorzugte Ausführungsformen der Gruppen der Formel (A-2) sind die folgenden Formeln (A-2-1) bis (A-2-59)

| | |
|---|---|
| 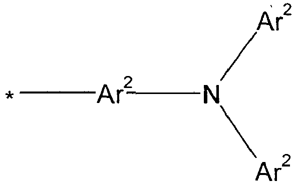 | 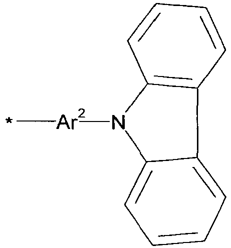 |
| Formel (A-2-1) | Formel (A-2-2) |
| | |
| 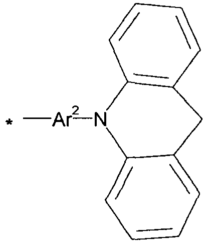 | 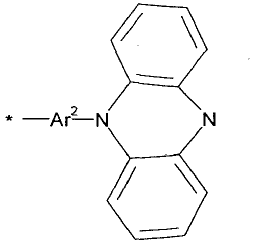 |
| Formel (A-2-3) | Formel (A-2-4) |
| 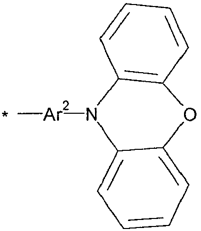 | 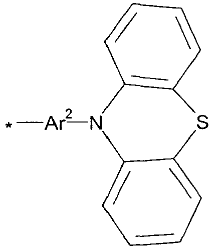 |
| Formel (A-2-5) | Formel (A-2-6) |
| | |
| 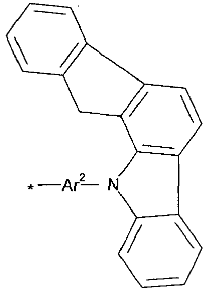 | 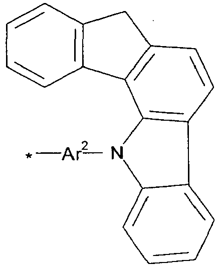 |
| Formel (A-2-7) | Formel (A-2-8) |
| | |
| 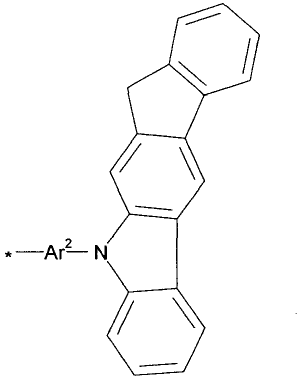 | 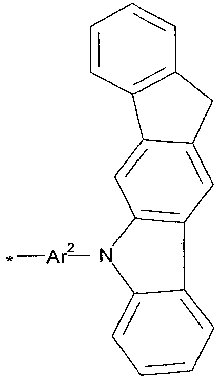 |
| Formel (A-2-9) | Formel (A-2-10) |
| 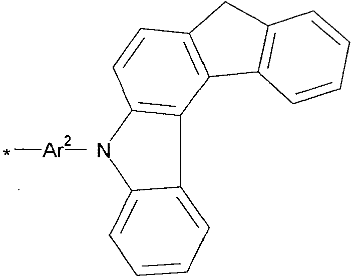 | 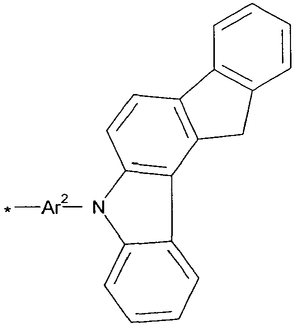 |
| Formel (A-2-11) | Formel (A-2-12) |
| | |
| 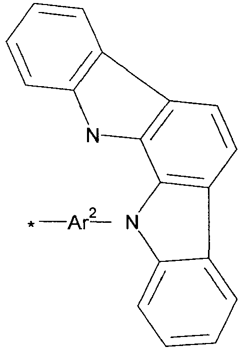 | 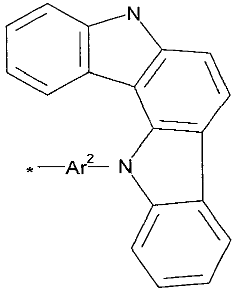 |
| Formel (A-2-13) | Formel (A-2-14) |
| | |
| 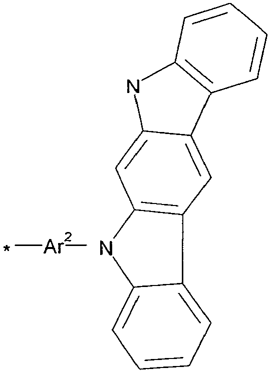 | 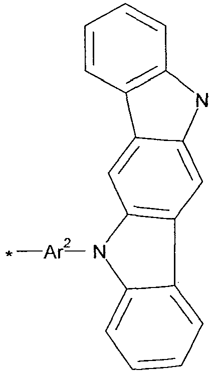 |
| Formel (A-2-15) | Formel (A-2-16) |
| 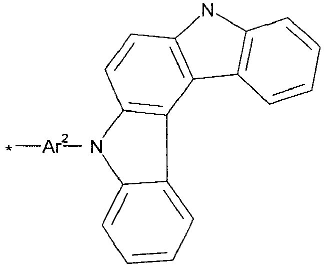 | 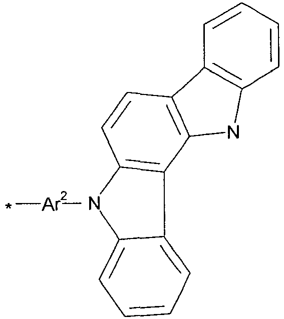 |
| Formel (A-2-17) | Formel (A-2-18) |
| | |
| 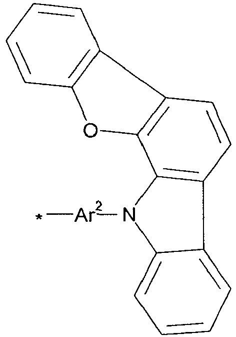 | 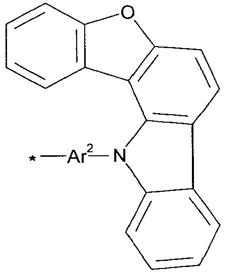 |
| Formel (A-2-19) | Formel (A-2-20) |
| | |
| 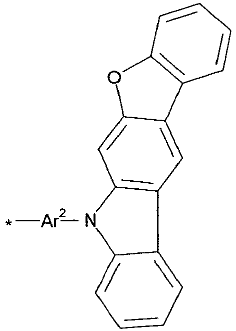 | 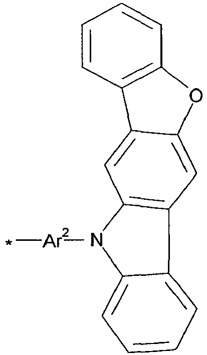 |
| Formel (A-2-21) | Formel (A-2-22) |
| 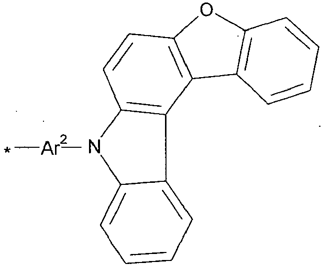 | 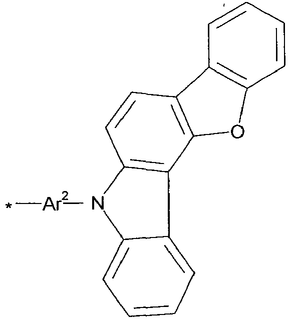 |
| Formel (A-2-23) | Formel (A-2-24) |
| | |
| 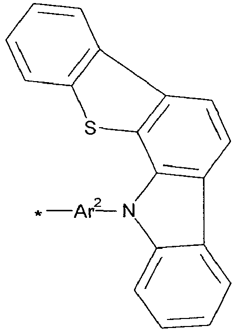 | 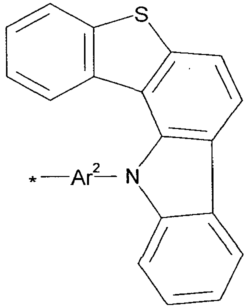 |
| Formel (A-2-25) | Formel (A-2-26) |
| | |
| 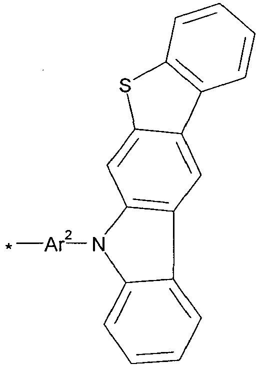 | 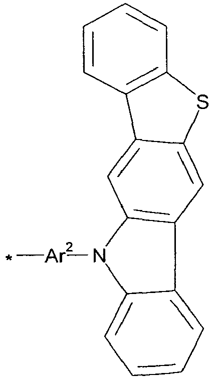 |
| Formel (A-2-27) | Formel (A-2-28) |
| 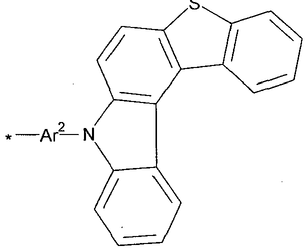 | 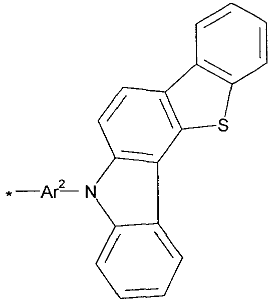 |
| Formel (A-2-29) | Formel (A-2-30) |
| | |
| 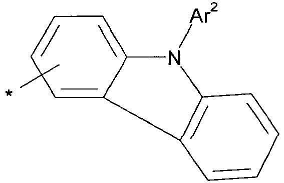 | 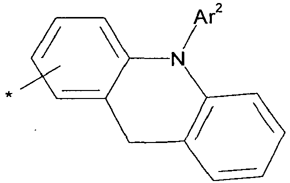 |
| Formel (A-2-31) | Formel (A-2-32) |
| | |
| 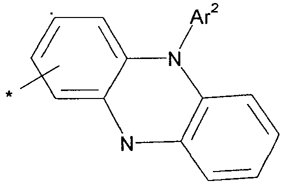 | 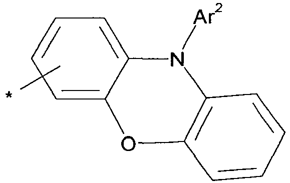 |
| Formel (A-2-33) | Formel (A-2-34) |
| | |
| 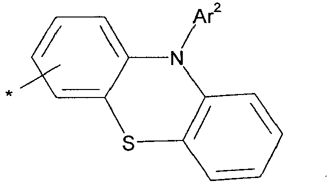 | 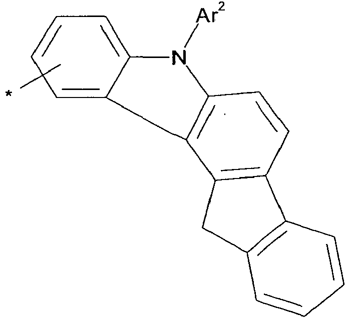 |
| Formel (A-2-35) | Formel (A-2-36) |
| 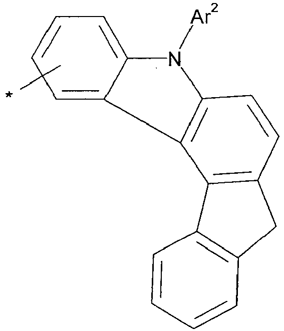 | 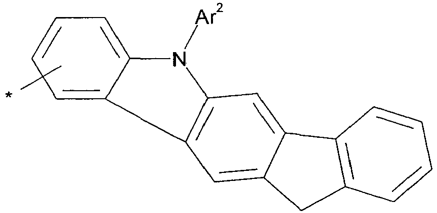 |
| Formel (A-2-37) | Formel (A-2-38) |
| | |
| 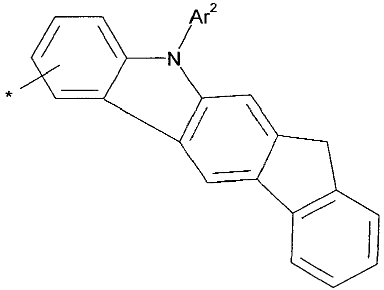 | 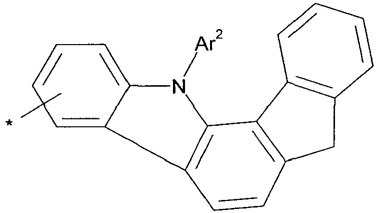 |
| Formel (A-2-39) | Formel (A-2-40) |
| | |
| 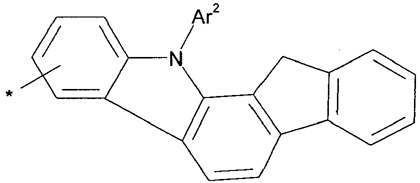 | 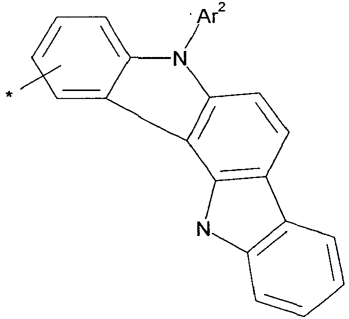 |
| Formel (A-2-41) | Formel (A-2-42) |
| 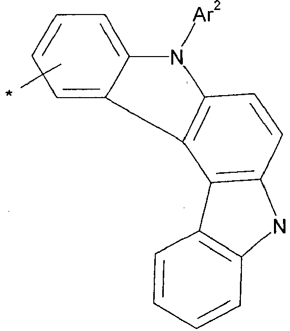 | 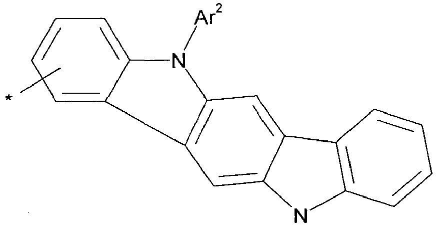 |
| Formel (A-2-43) | Formel (A-2-44) |
| | |
| 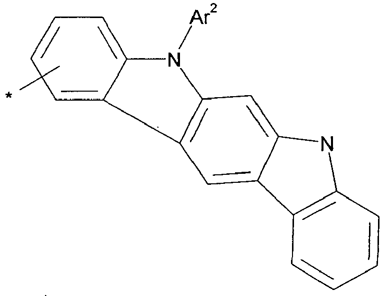 | 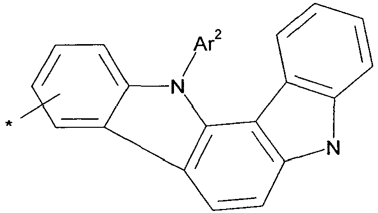 |
| Formel (A-2-45) | Formel (A-2-46) |
| | |
| 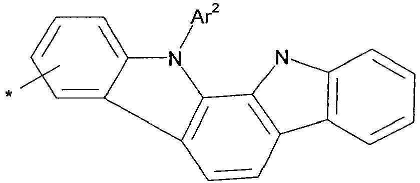 | 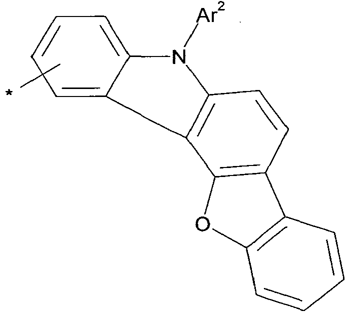 |
| Formel (A-2-47) | Formel (A-2-48) |
| 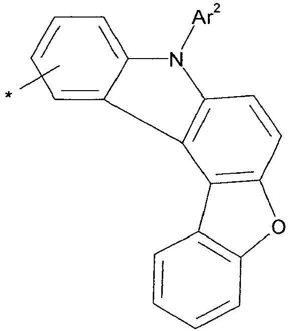 | 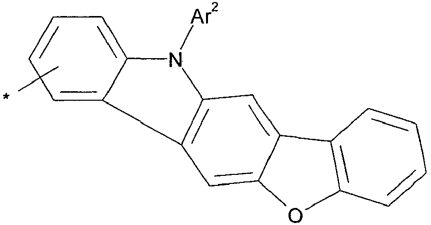 |
| Formel (A-2-49) | Formel (A-2-50) |
| | |
| 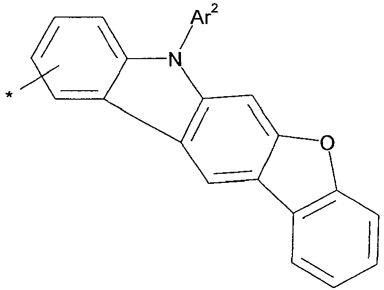 | 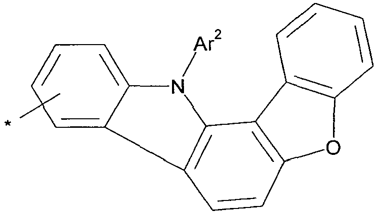 |
| Formel (A-2-51) | Formel (A-2-52) |
| | |
| 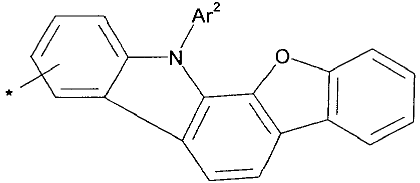 | 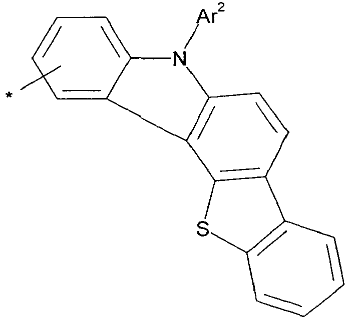 |
| Formel (A-2-53) | Formel (A-2-54) |
| 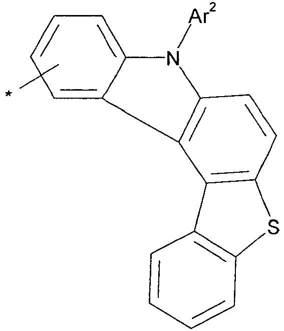 | 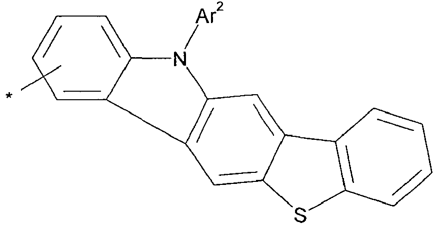 |
| Formel (A-2-55) | Formel (A-2-56) |
| | |
| 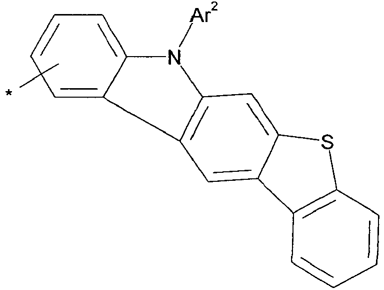 | 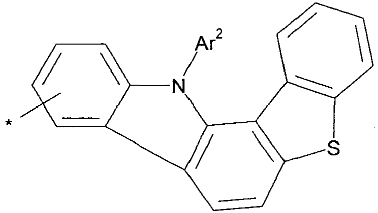 |
| Formel (A-2-57) | Formel (A-2-58) |
| | |
| 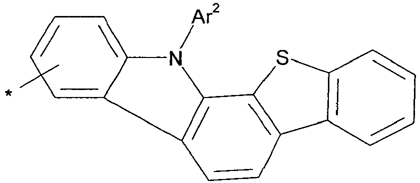 | |
| Formel (A-2-59) | |

welche an den freien Positionen jeweils mit Resten R¹ substituiert sein können. Für Ar² ist es dabei bevorzugt, dass es bei jedem Auftreten gleich oder verschieden gewählt ist aus Benzol, Biphenyl, Terphenyl, Quaterphenyl, Fluoren, Spirobifluoren, Indenofluoren, Carbazol, Indenocarbazol, Indolocarbazol, Dibenzofuran und Dibenzothiophen, die jeweils mit einem oder mehreren Resten R¹ substituiert sein können.

Bevorzugte Verbindungen der Formel (I) entsprechen einer der Formeln (I-1) bis (I-3)

| | |
|---|---|
| 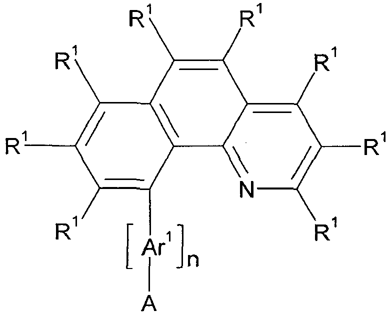 | 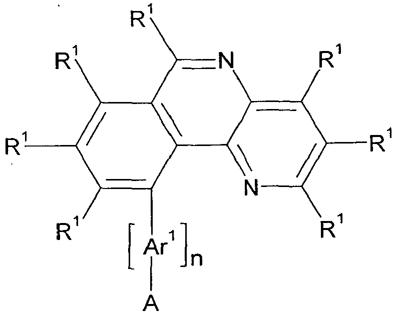 |
| Formel (I-1) | Formel (I-2) |
| | |
| 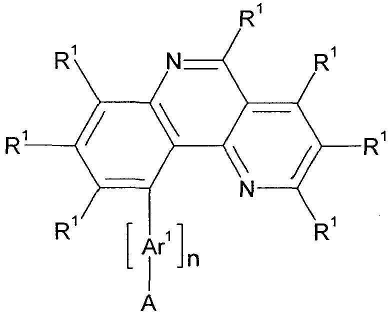 | |
| Formel (I-3) | |

wobei die auftretenden Gruppen und Indices wie oben definiert sind. Dabei sind die oben aufgeführten bevorzugten Ausführungsformen der Gruppen und Indices bevorzugt.

Insbesondere ist es für Formeln (I-1) bis (I-3) bevorzugt, dass die Gruppe A gewählt ist aus den bevorzugten Ausführungsformen der Gruppen der Formeln (A-1) und (A-2), insbesondere aus den Gruppen der Formeln (I-1-1) bis (I-1-30) und (I-2-1) bis (I-2-59).

Nochmals insbesondere ist es für Formeln (I-1) bis (I-3) bevorzugt, dass die Einheit -[Ar¹]ₙ- gewählt ist aus den oben aufgeführten Formeln (L-1) bis (L-18).

Nochmals insbesondere ist es für Formeln (I-1) bis (I-3) bevorzugt, dass n gleich 0 ist.

Unter den Formeln (I-1) bis (I-3) ist besonders bevorzugt Formel (I-1).

Im Folgenden sind Beispiele für Verbindungen der Formel (I) aufgeführt.

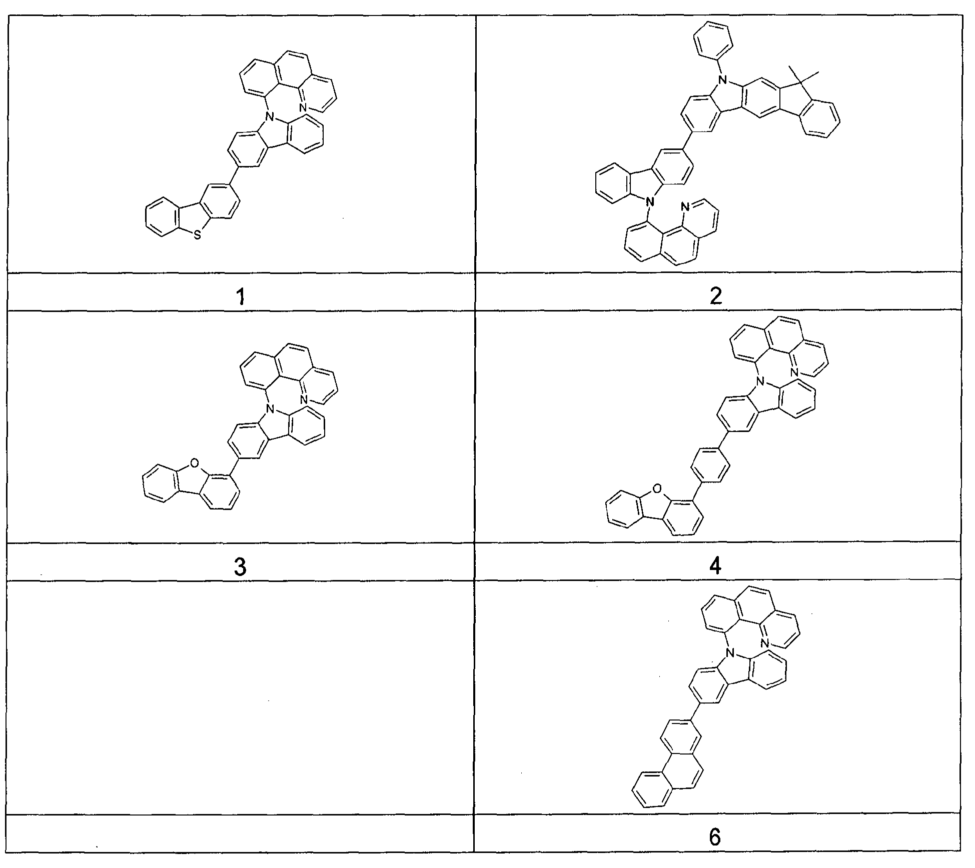

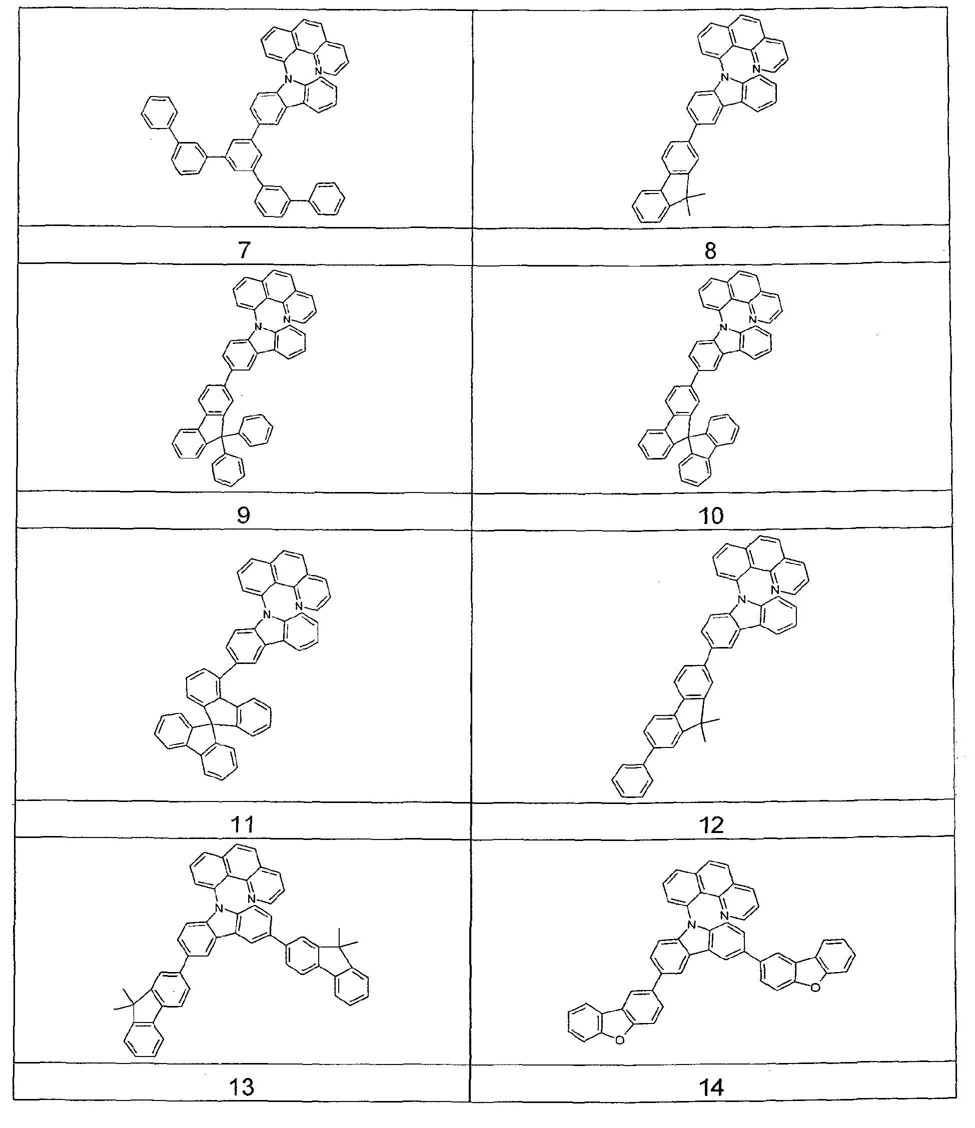

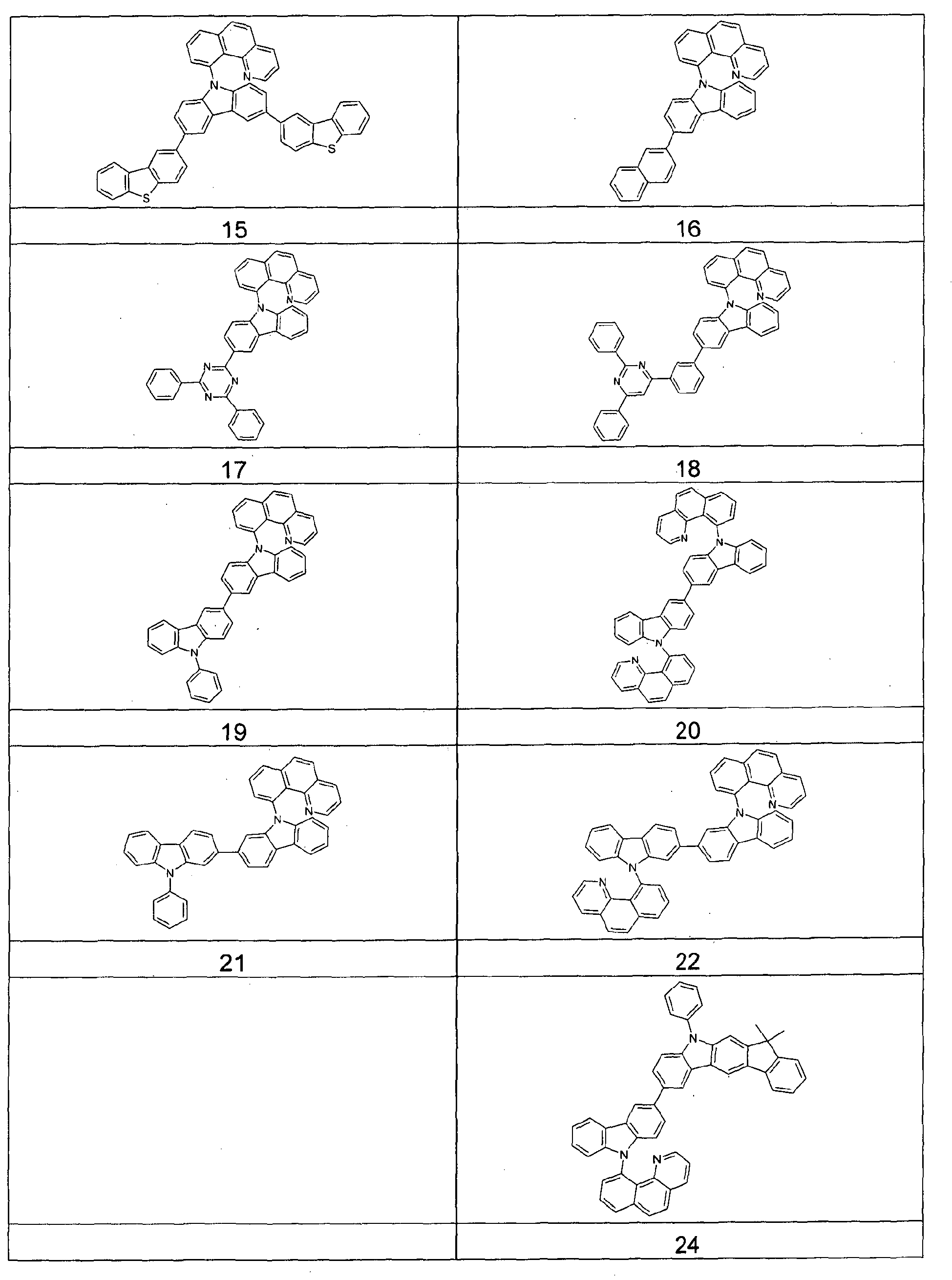

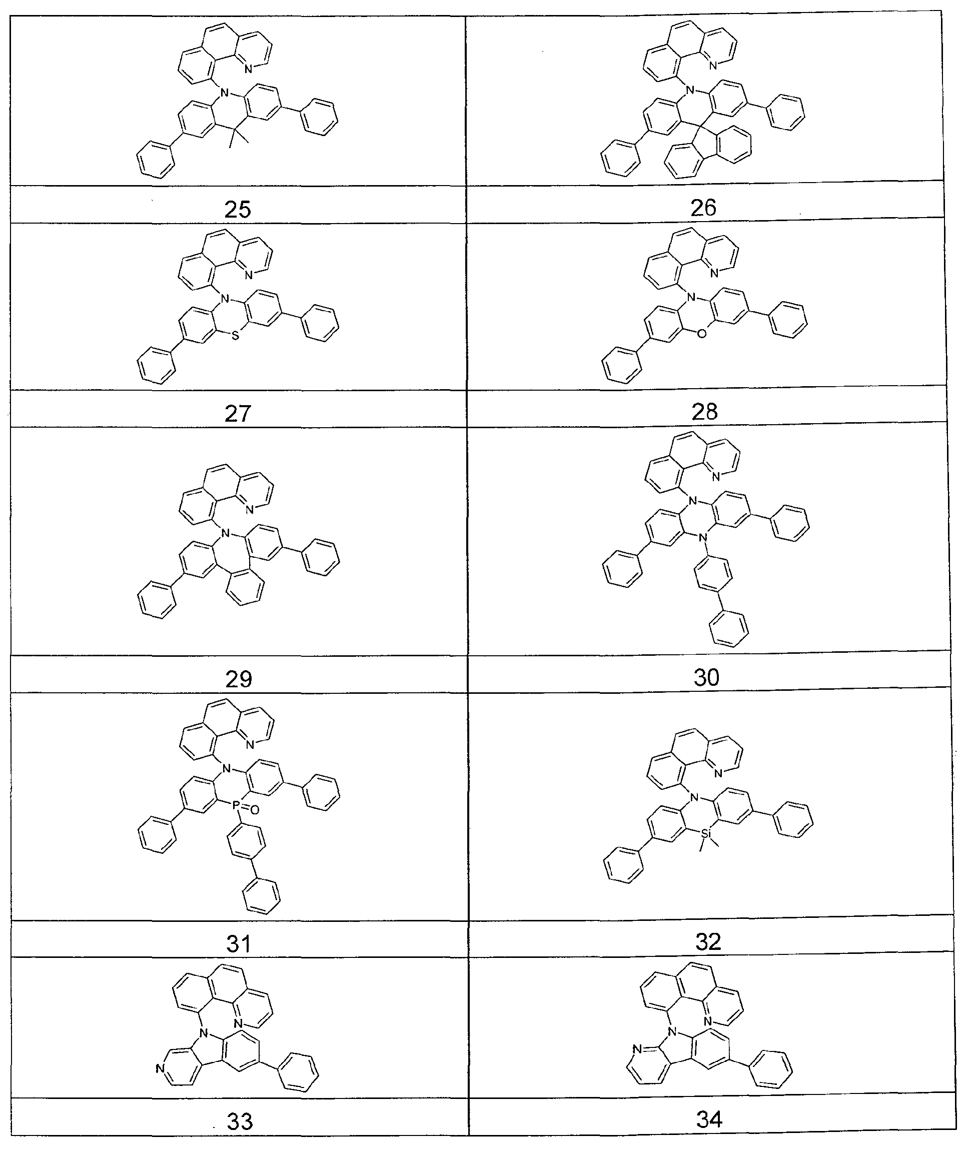

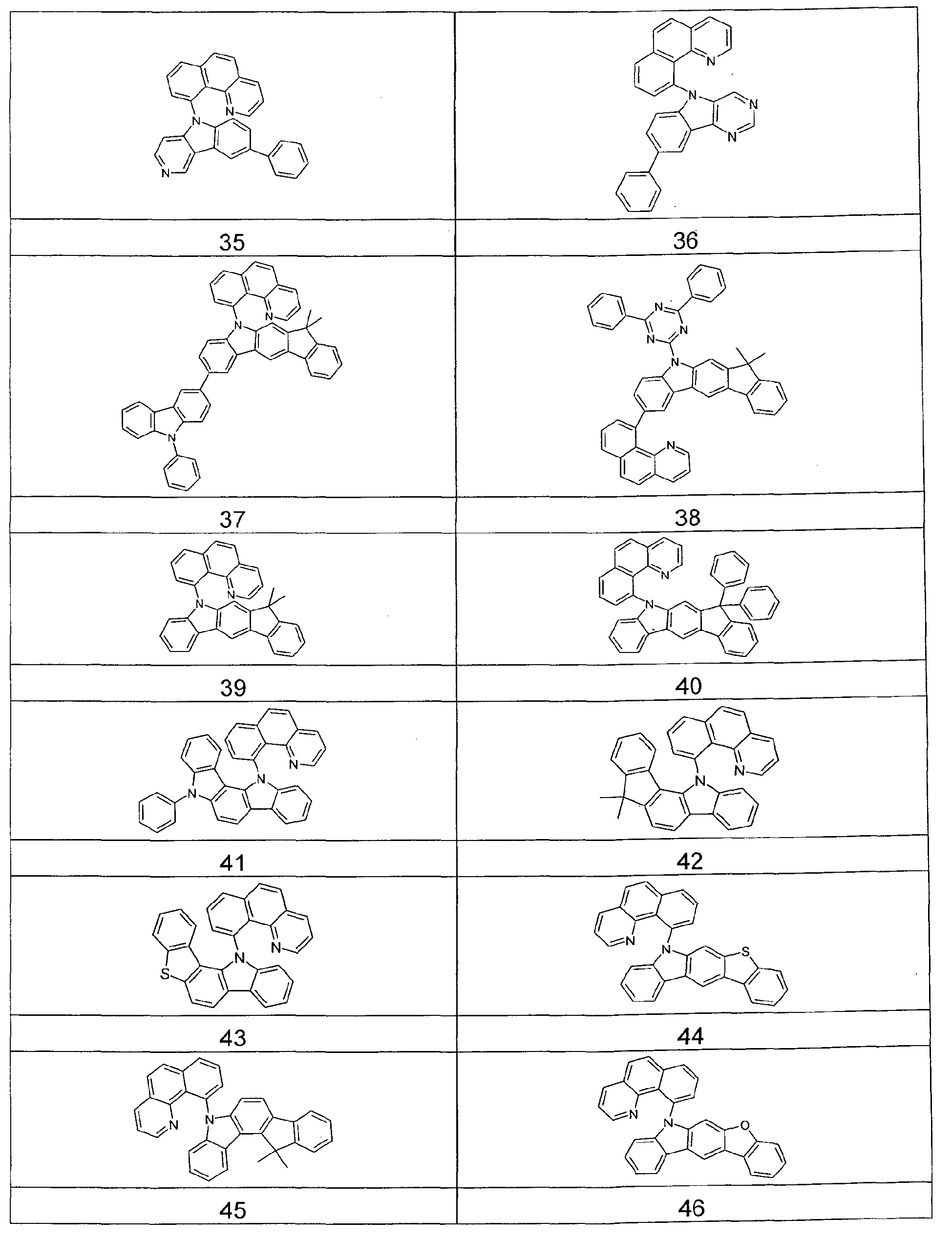

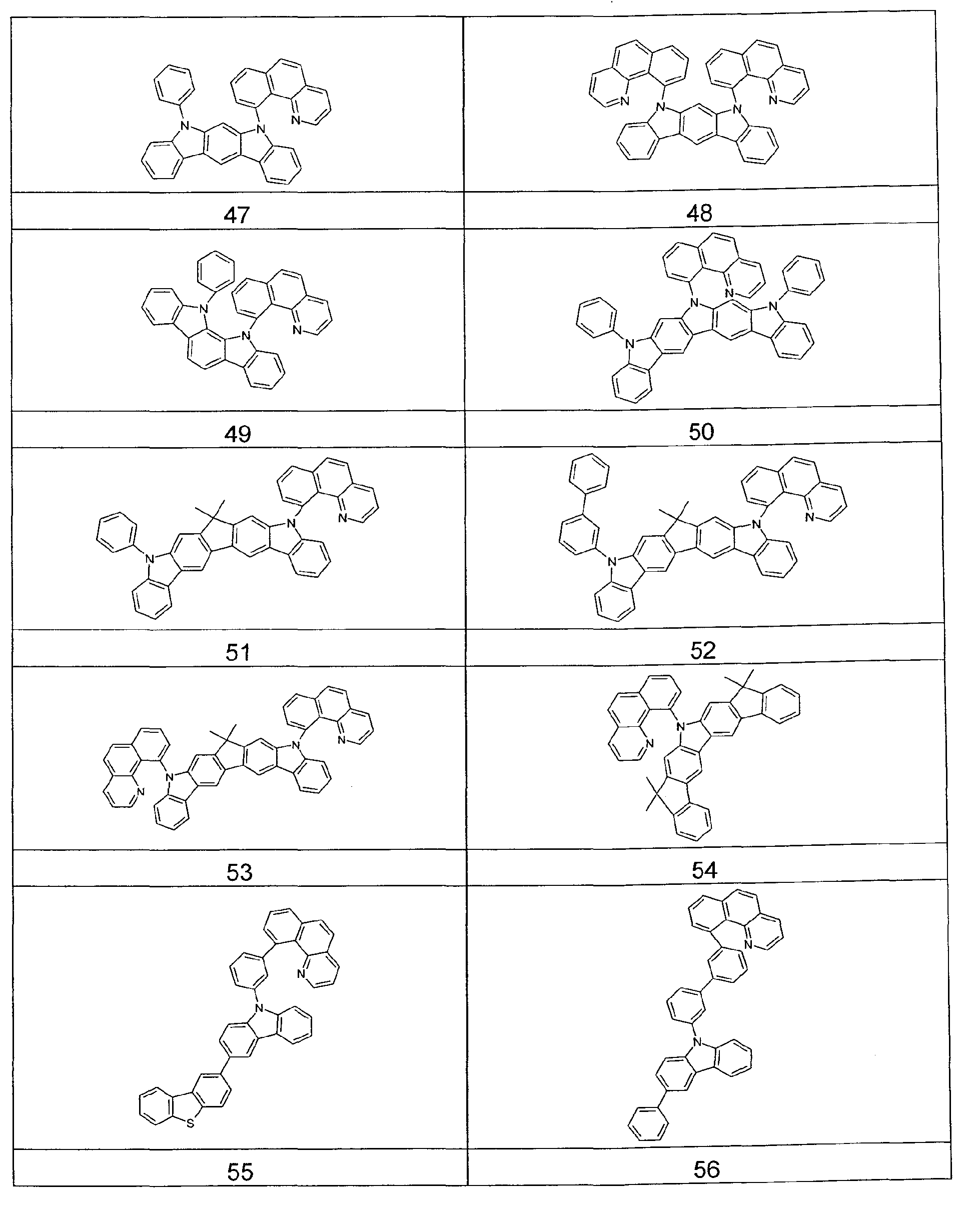

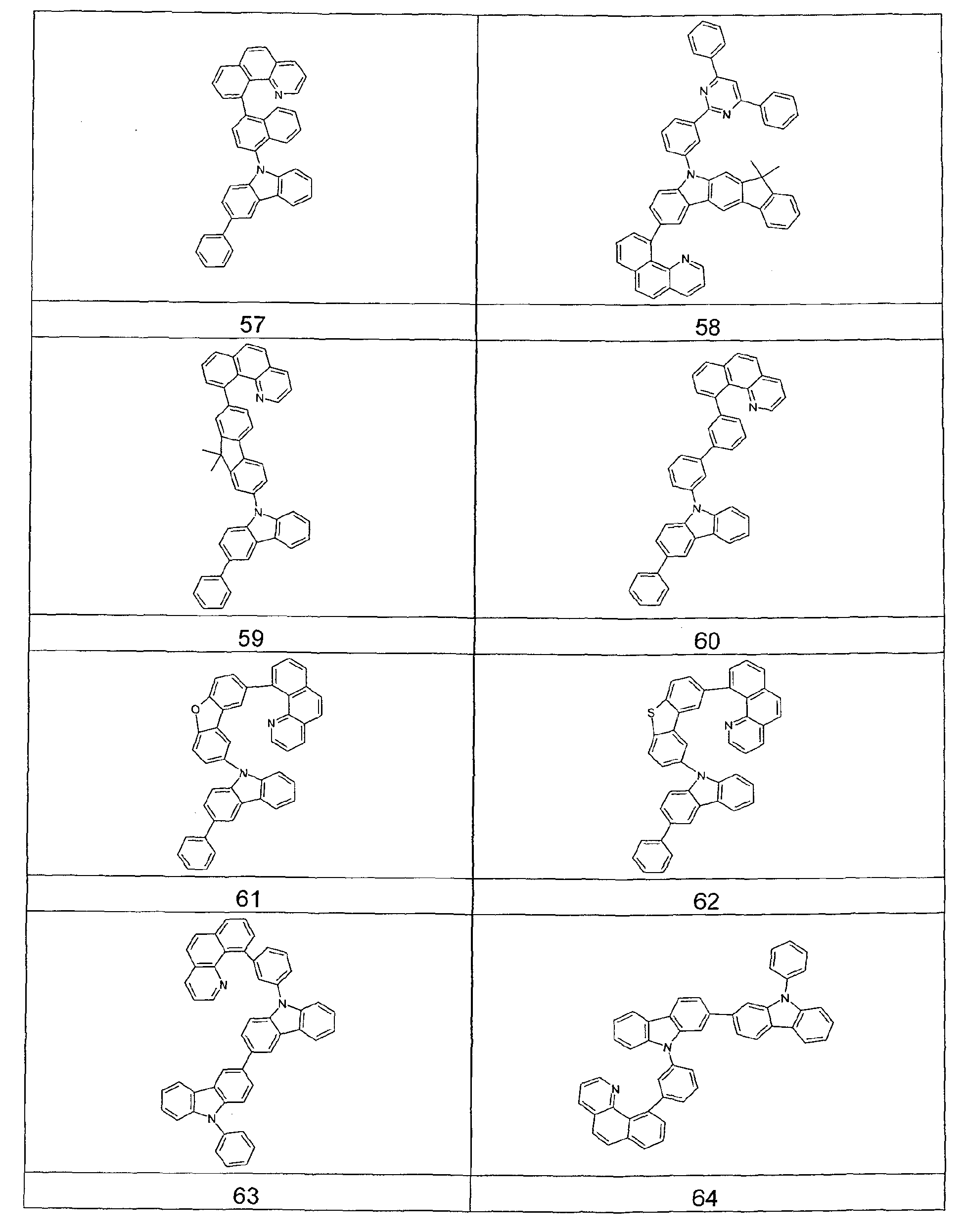

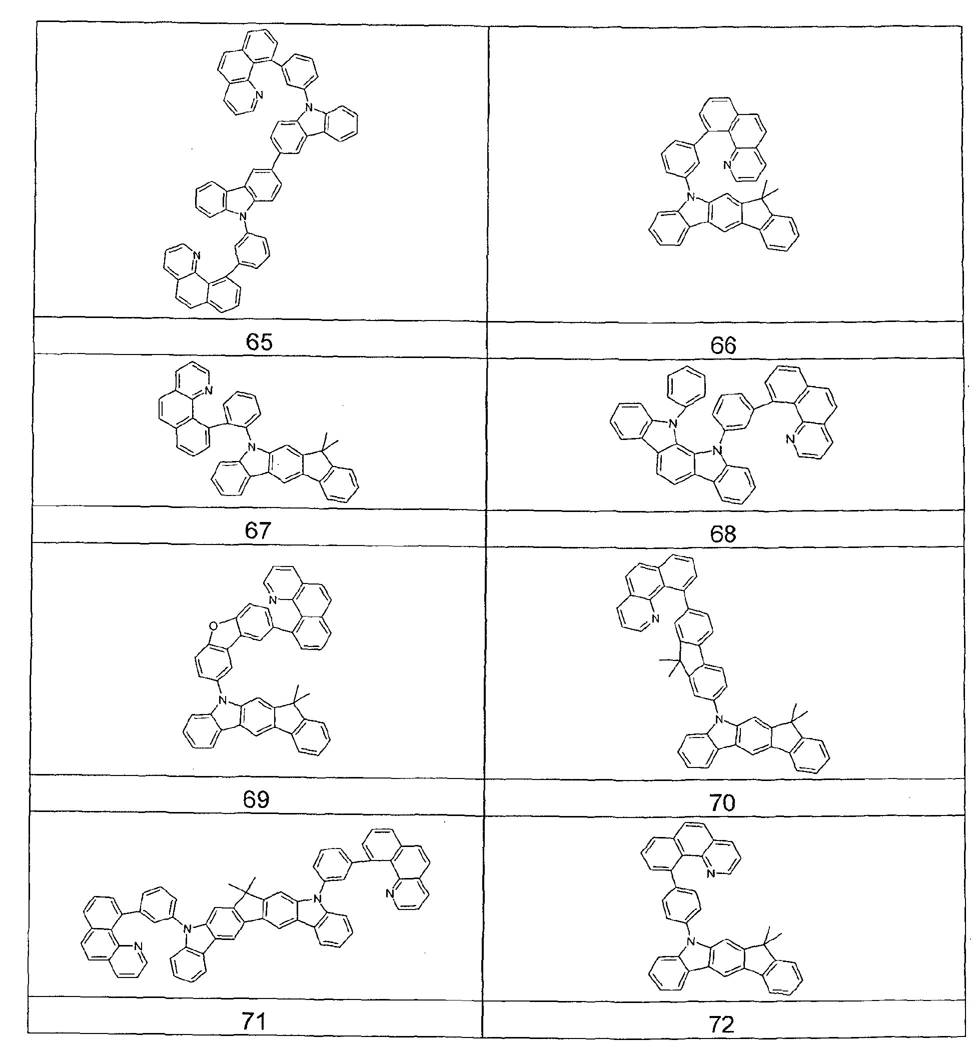

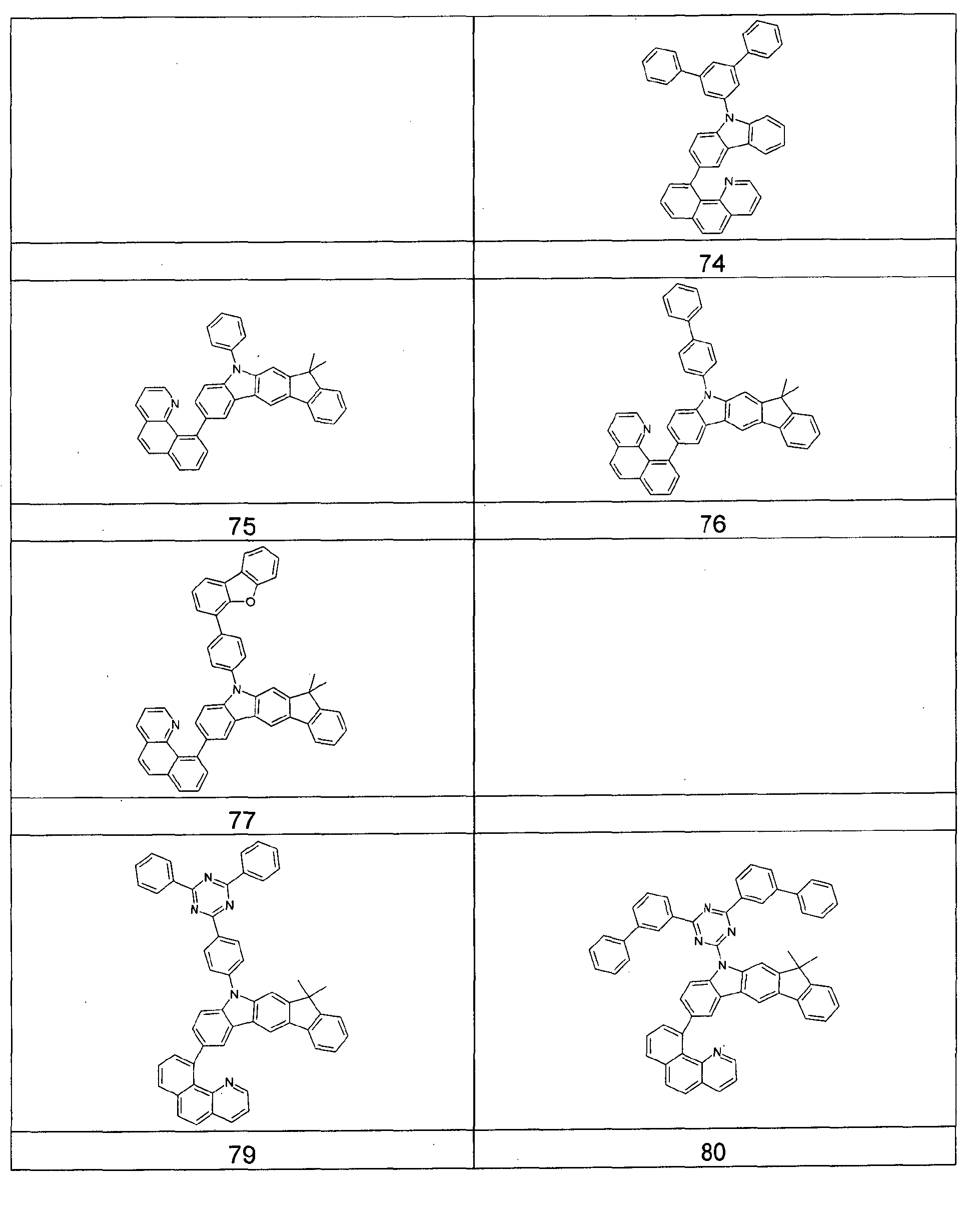

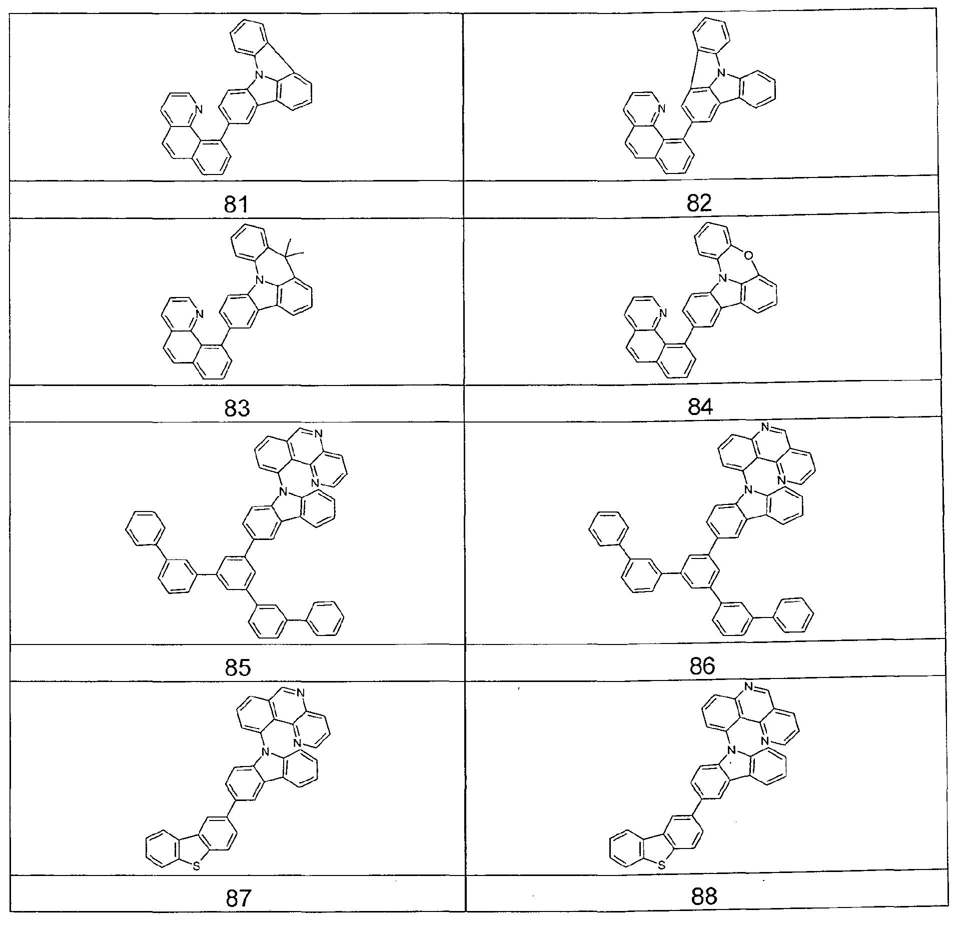

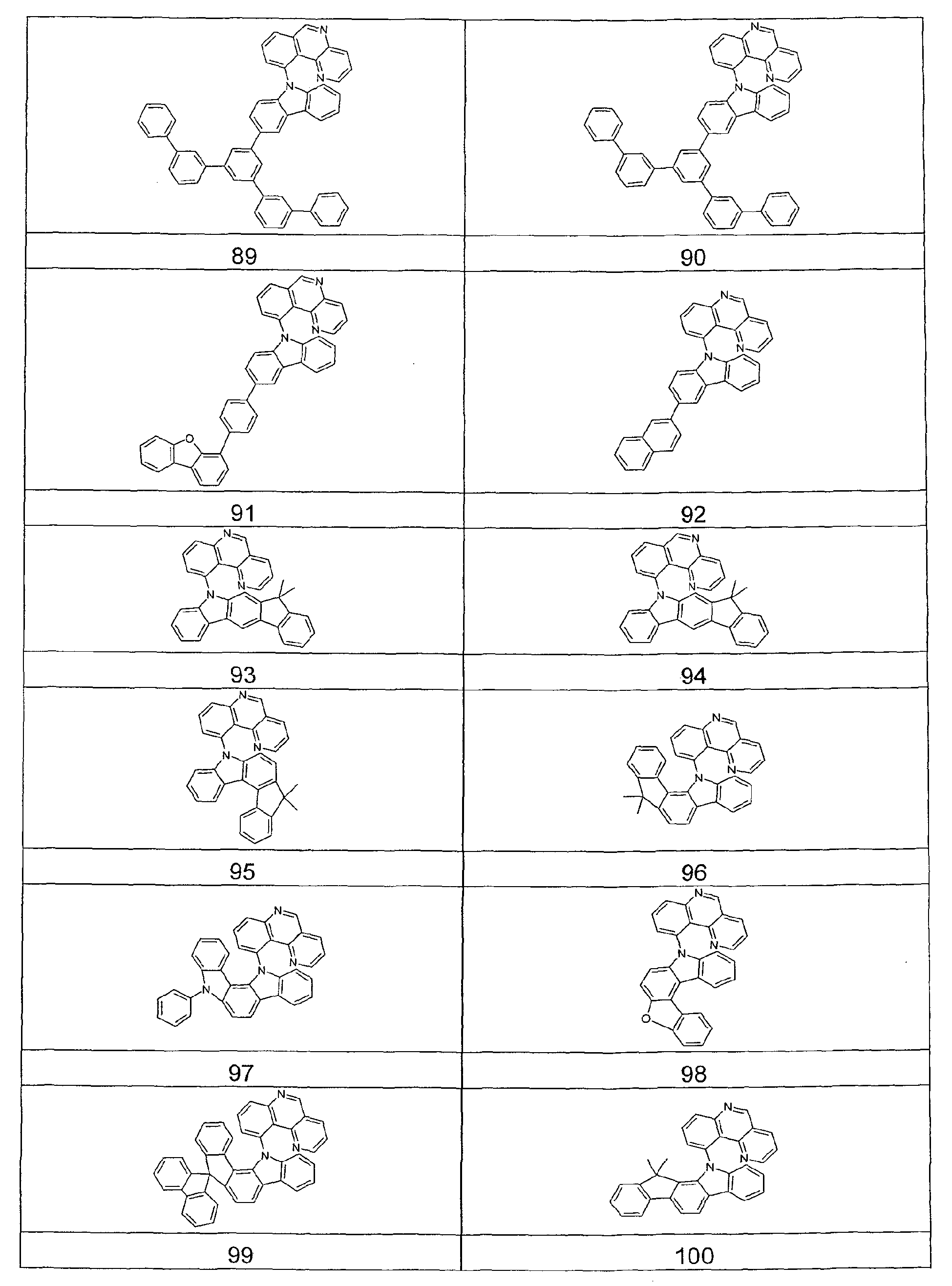

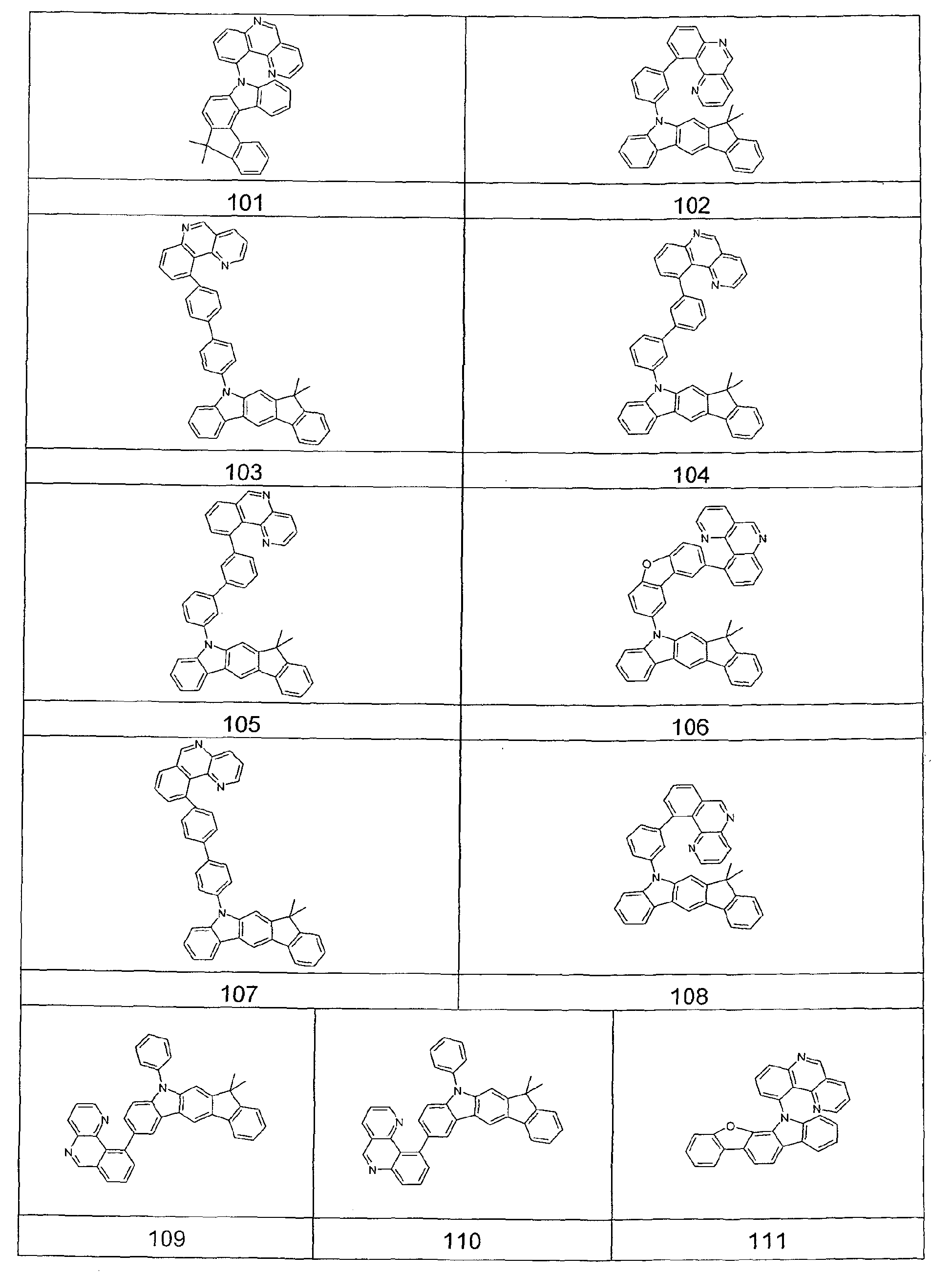

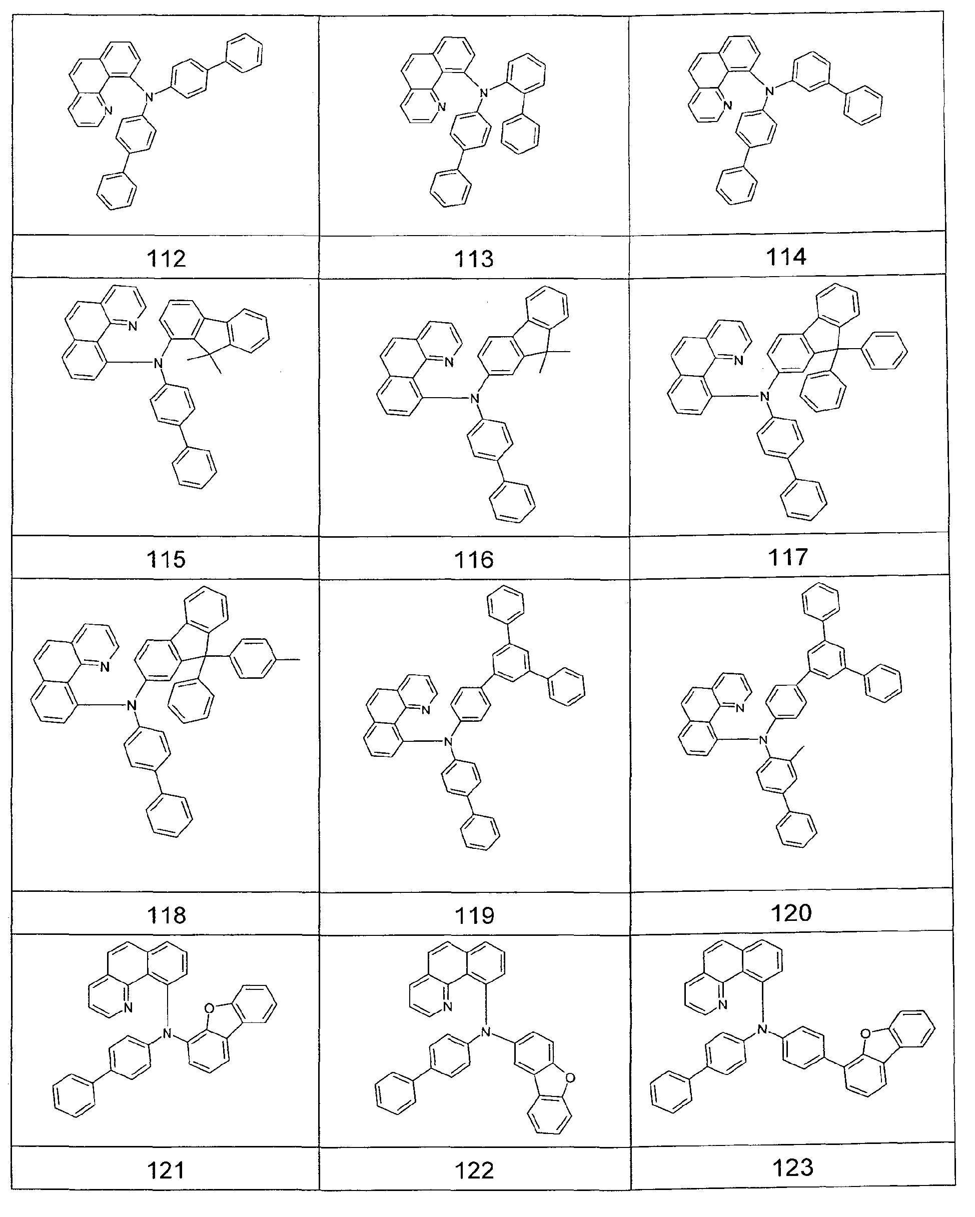

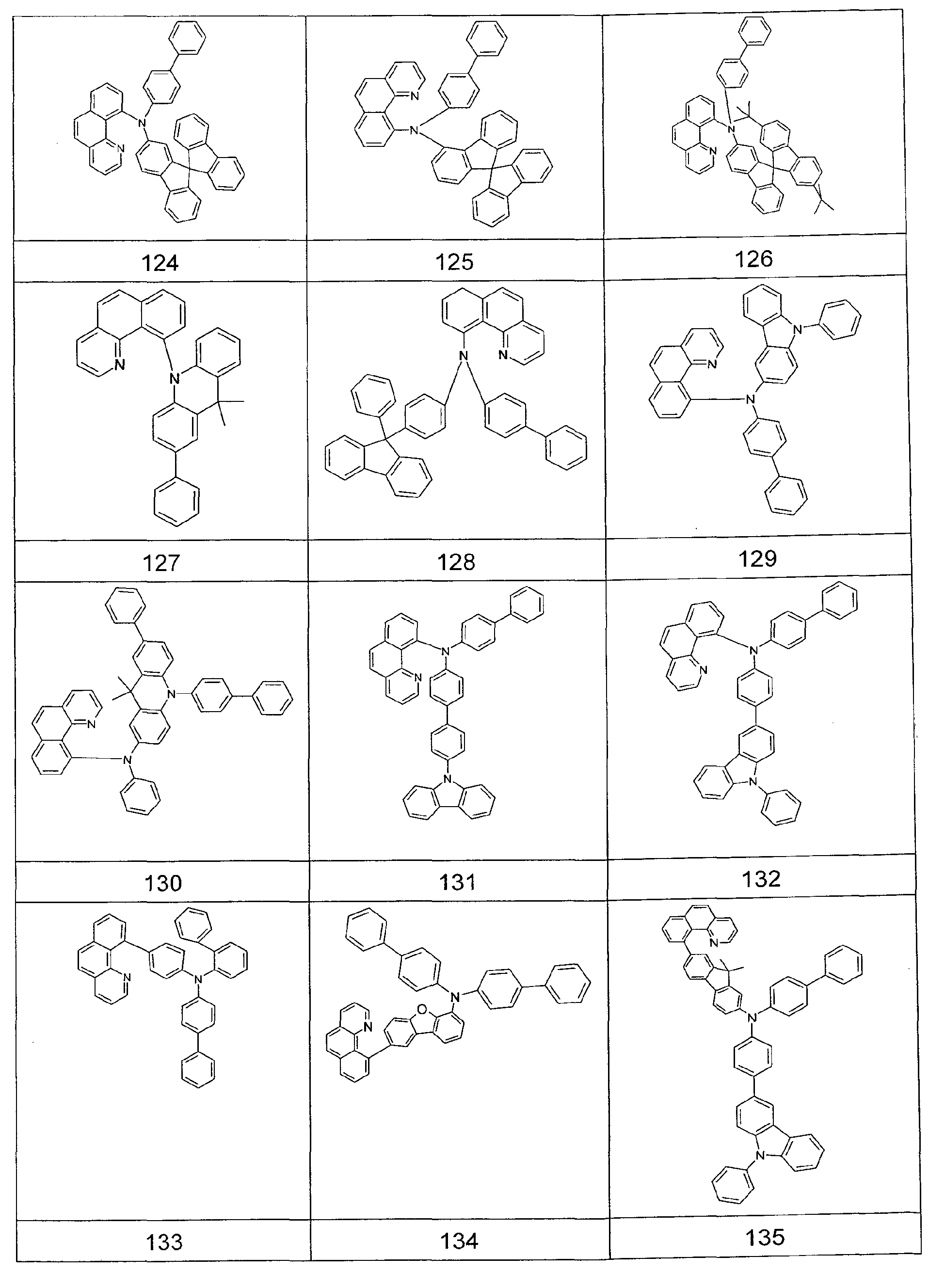

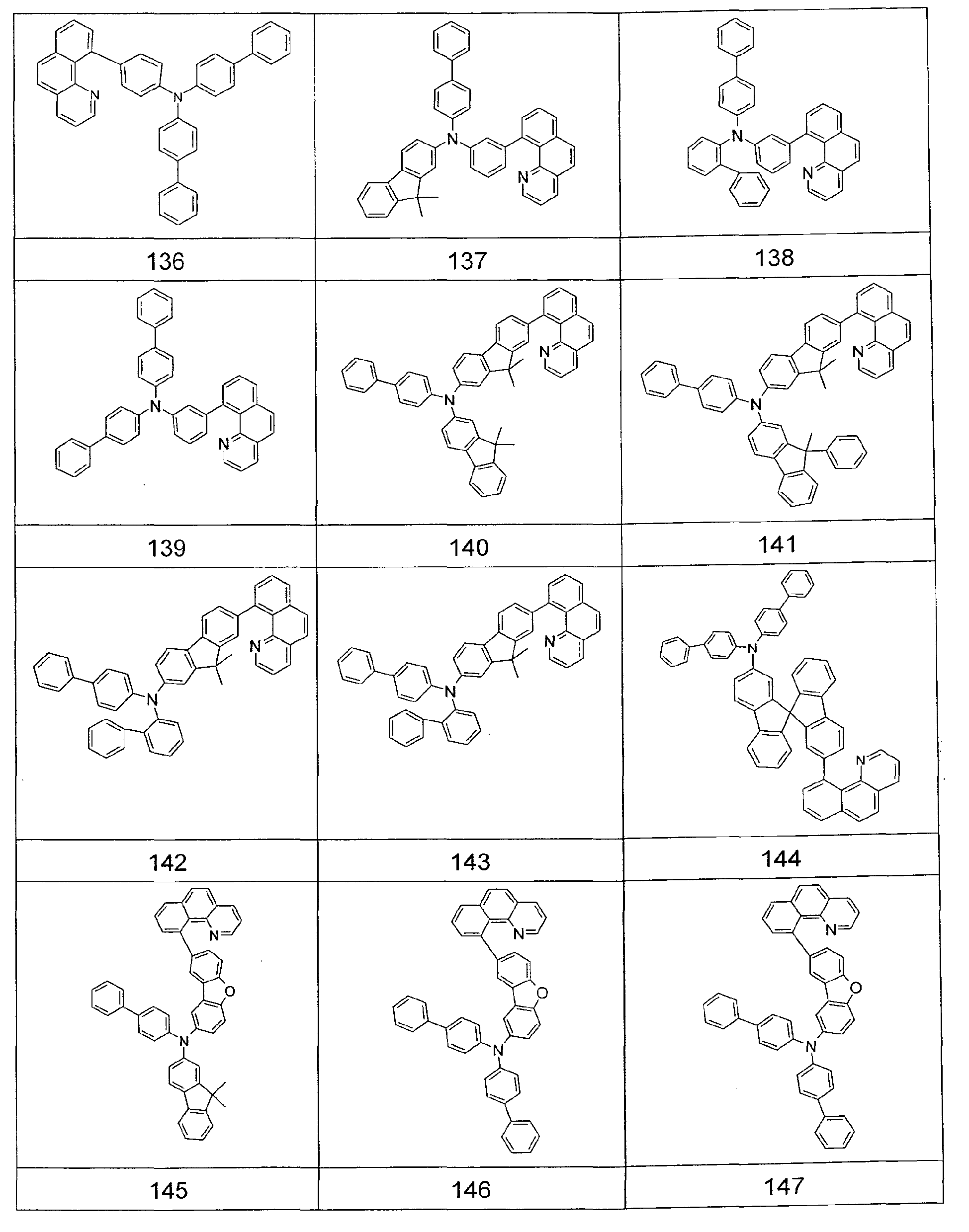

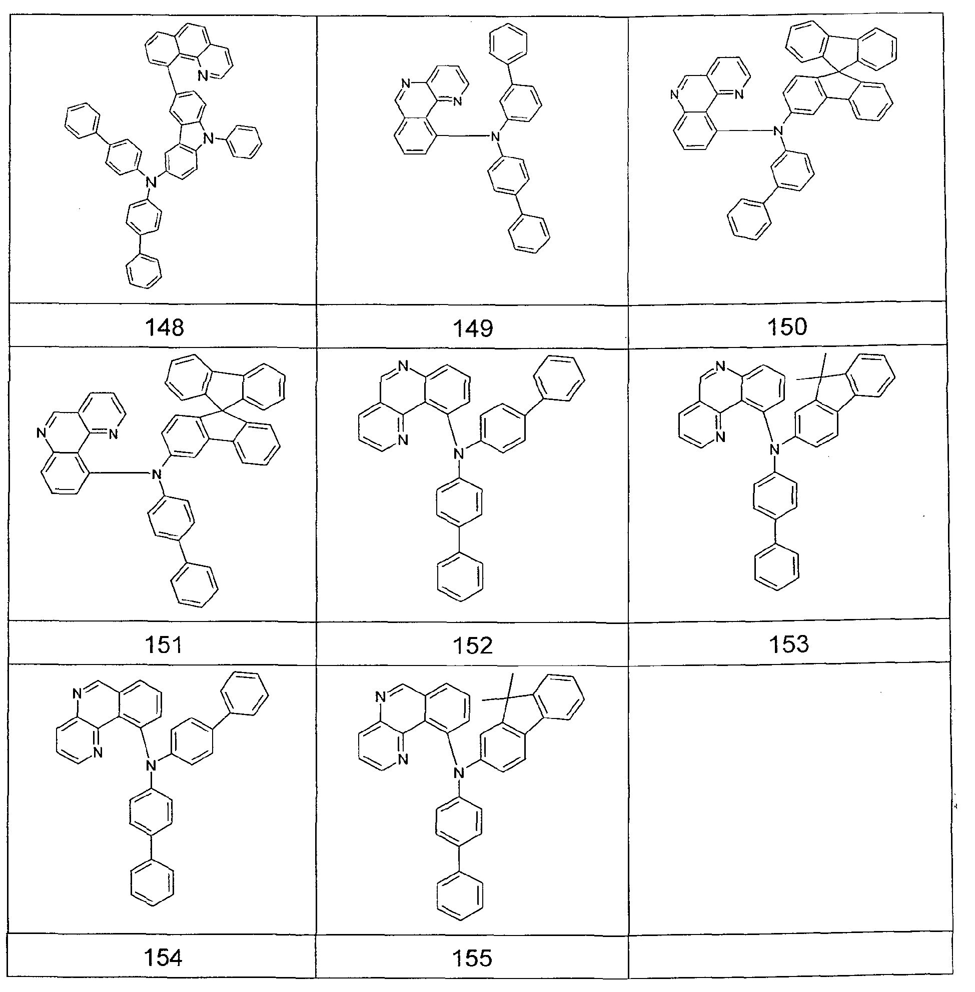

Die Synthese der erfindungsgemäßen Verbindungen kann gemäß im Stand der Technik bekannten Verfahren und Reaktionstypen, beispielsweise Halogenierung, Buchwald-Kupplung und Suzuki-Kupplung erfolgen.

Schema 1 zeigt einen bevorzugter Syntheseweg zur Herstellung der erfindungsgemäßen Verbindungen. Zur Synthese der erfindungsgemäßen Verbindungen wird die Benzo[h]chinolin-Verbindung A in einer Buchwald-Kupplung mit einem Amin B der Formel Ar-NH-Ar umgesetzt. In diesem und in den folgenden Schemata sind die Verbindungen unsubstituiert gezeigt. Sie können aber auch mit beliebigen Substituenten versehen sein.

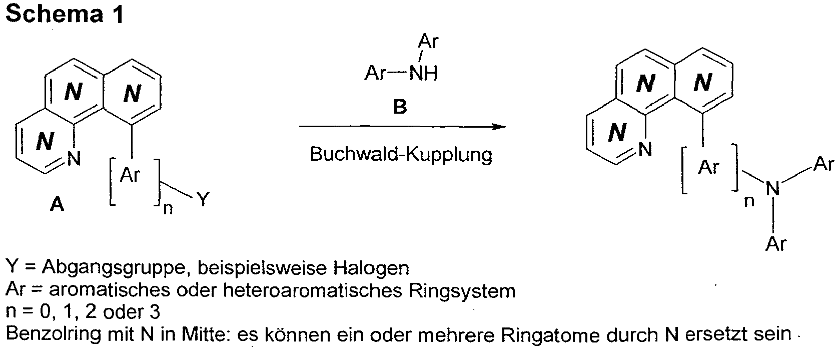

Ein anderer bevorzugter Syntheseweg zur Herstellung der erfindungsgemäßen Verbindungen wird im Schema 2 dargestellt. Der Syntheseweg umfasst zwei Kupplungsreaktionen: zunächst wird die Benzo[h]chinolin-Verbindung A in einer ersten Buchwald-Kupplung mit einem Amin C der Formel Ar-NH₂ umgesetzt. Schließlich erfolgt eine zweite Buchwald-Kupplung mit einer Verbindung D, beispielsweise mit einer Bromaryl-Verbindung.

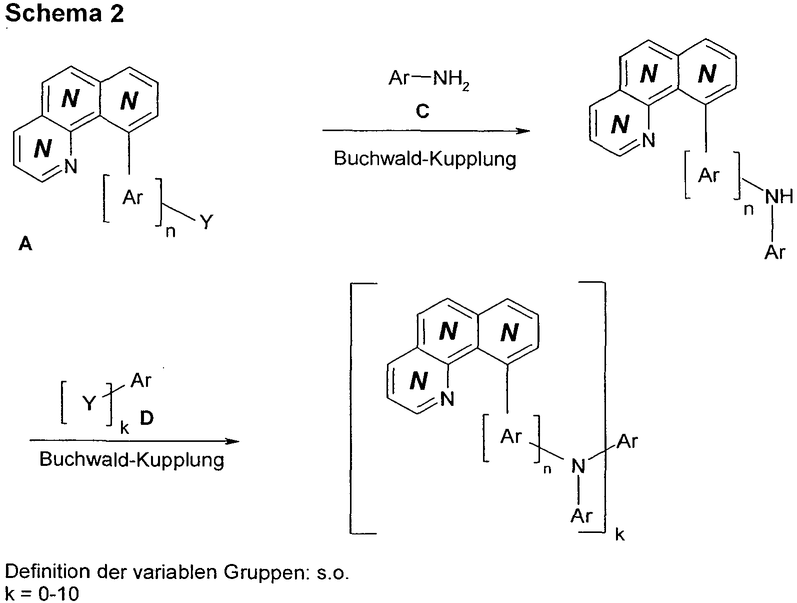

Synthesewege für die Ausgangsverbindungen A welche in der Synthese der erfindungsgemäßen Verbindungen eingesetzt werden, sind dem Fachmann bekannt. Weiterhin werden in den Ausführungsbeispielen einige explizite Syntheseverfahren im Detail dargestellt.

Ein weiteres geeignetes Syntheseverfahren zur Herstellung von Verbindungen der Formel (I), insbesondere solchen mit Carbazol-Substitutenten, ist allgemein im folgenden Schema 3 gezeigt. Carbazol-Gruppen (Verbindungen G) werden darin über Ullmann- oder Buchwald-Kupplungen in die Verbindungen eingeführt. Weiterhin besteht die Möglichkeit, Arylgruppen, die mit Carbazol- oder Diarylaminogruppen substituiert sind (Verbindungen H), über Suzuki-Kupplungen einzuführen.

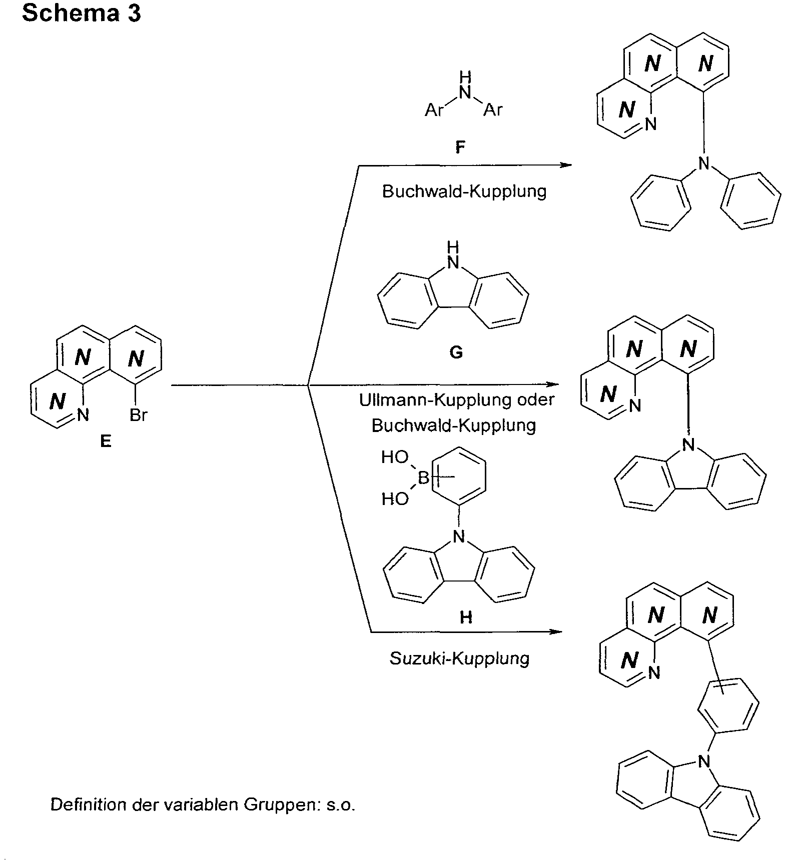

Weiterer Gegenstand der vorliegenden Erfindung ist somit ein Verfahren zur Herstellung einer Verbindung gemäß Formel (I), dadurch gekennzeichnet, dass eine Verbindung einer Formel (Int-I) 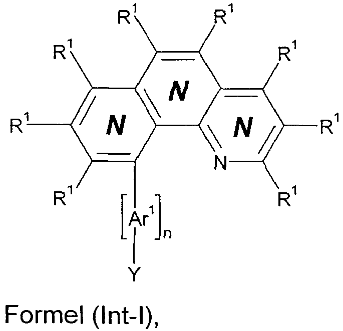 wobei die auftretenden variablen Gruppen und Indices in der Verbindung der Formel (Int-I) wie für die Verbindung der Formel (I) definiert sind, und in den Benzolringen, in deren Mitte ein N gezeichnet ist, jeweils ein oder mehrere Ringglieder -CR¹= durch -N= ersetzt sein können, und wobei Y eine beliebige reaktive Gruppe darstellt,
in einer Kupplungsreaktion weiter umgesetzt wird.

Die Kupplungsreaktion ist dabei bevorzugt eine übergangsmetallkatalysierte Kupplungsreaktion, besonders bevorzugt eine Pd- oder Cukatalysierte Kupplungsreaktion. Ganz besonders bevorzugt ist sie eine Buchwald-Kupplung oder eine Ullmann-Kupplung. Bevorzugt findet die Kupplungsreaktion mit dem Stickstoffatom einer Arylaminverbindung oder einer Carbazolverbindung statt.

Die Gruppe Y ist bevorzugt gewählt aus I, Br, Cl, einem O-Tosylat, einem O-Triflat, einem O-Sulfonat, Boronsäure, einem Boronsäureester, einer teilfluorierten Silylgruppe, und einer Diazoniumgruppe. Besonders bevorzugt ist die Gruppe Y gewählt aus I, Br und Cl.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Die Verbindungen können, unter anderem abhängig von der Substitution, in unterschiedlichen Funktionen und/oder Schichten eingesetzt werden. Bevorzugt werden die Verbindungen als Matrixmaterialien, besonders bevorzugt als Matrixmaterialien für phosphoreszierende Emitter in einer emittierenden Schicht, oder als Lochtransportmaterialien in einer Lochtransportschicht eingesetzt. Gemäß einer alternativen Ausführungsform der Erfindung können die Verbindungen auch als Elektronentransportmaterialien in einer Elektronentransportschicht eingesetzt werden.

Ein weiterer Gegenstand der Erfindung ist die Verwendung der Verbindungen gemäß Formel (I) in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Gegenstand der Erfindung ist weiterhin eine elektronische Vorrichtung, enthaltend Anode, Kathode sowie mindestens eine organische Schicht, wobei die organische Schicht mindestens eine Verbindung der Formel (I) enthält. Dabei ist die elektronische Vorrichtung bevorzugt ausgewählt aus den oben genannten Vorrichtungen und besonders bevorzugt eine organische Elektrolumineszenzvorrichtung (OLED).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer), Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt. Die in den jeweiligen Schichten und Funktionen bevorzugt eingesetzten Verbindungen werden in späteren Abschnitten explizit offenbart.

Die Abfolge der Schichten der organischen Elektrolumineszenzvorrichtung ist bevorzugt wie folgt:
- Anode
- Lochinjektionsschicht
- Lochtransportschicht
- optional 1, 2 oder 3 weitere Lochtransportschichten
- emittierende Schicht
- Elektronentransportschicht
- Elektroneninjektionsschicht
- Kathode.
Dabei müssen nicht alle der genannten Schichten vorhanden sein, und/oder es können zusätzlich weitere Schichten vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in der Lochtransportschicht oder in einer anderen Schicht vorhanden sein.

In weiß emittierenden OLEDs gemäß der vorliegenden Anmeldung können anstelle mehrerer farbig emittierender Emitterverbindungen auch eine einzelne Emitterverbindung verwendet werden, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Emitter eingesetzt wird. Dabei kann die Verbindung in unterschiedlichen Schichten, bevorzugt in einer Lochtransportschicht oder in einer emittierenden Schicht verwendet werden.

Vom Begriff phosphoreszierende Emitter sind gemäß der vorliegenden Anmeldung Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, wie einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen. Weitere Beispiele für geeignete phosphoreszierende Emitter können der in einem späteren Abschnitt folgenden Tabelle entnommen werden.

In einer bevorzugten Ausführungsform der vorliegenden Erfindung werden die Verbindungen der Formel (I) als Matrixmaterial in einer emittierenden Schicht in Kombination mit einem oder mehreren fluoreszierenden oder phosphoreszierenden Emittern, vorzugsweise phosphoreszierenden Emittern, eingesetzt. Bevorzugt weisen die Verbindungen der Formel (I) in diesem Fall eine Gruppe X auf, die eine Einfachbindung darstellt, so dass die Verbindungen eine Carbazolgruppe enthalten.

Der Anteil des Matrixmaterials in der emittierenden Schicht beträgt in diesem Fall zwischen 50.0 und 99.9 %, bevorzugt zwischen 80.0 und 99.5 % und besonders bevorzugt für fluoreszierende emittierende Schichten zwischen 92.0 und 99.5 % sowie für phosphoreszierende emittierende Schichten zwischen 85.0 und 97.0 %. Entsprechend beträgt der Anteil des Emitters zwischen 0.1 und 50.0 %, bevorzugt zwischen 0.5 und 20.0 % und besonders bevorzugt für fluoreszierende emittierende Schichten zwischen 0.5 und 8.0 % sowie für phosphoreszierende emittierende Schichten zwischen 3.0 und 15.0 %.

Unter Angaben der Anteile von Verbindungen in % wird dabei in solchen Fällen, in denen die Verbindung aus der Gasphase aufgebracht werden, Volumenprozent verstanden, und in solchen Fällen, in denen die Verbindungen aus flüssiger Phase aufgebracht werden, Gewichtsprozent verstanden.

Eine emittierende Schicht einer organischen Elektrolumineszenzvorrichtung kann auch Systeme umfassend mehrere Matrixmaterialien (Mixed-Matrix-Systeme) und/oder mehrere Emitter enthalten. Auch in diesem Fall sind die Emitter im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Emittermaterials.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Emitterverbindungen umfassen. Die Emitterverbindungen zusammen haben erfindungsgemäß einen Anteil von 0.1 bis 50.0 % an der Gesamtmischung und bevorzugt einen Anteil von 0.5 bis 20.0 % an der Gesamtmischung. Entsprechend haben die Matrixverbindungen zusammen einen Anteil von 50.0 bis 99.9 % an der Gesamtmischung und bevorzugt einen Anteil von 80.0 bis 99.5 % an der Gesamtmischung.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitter im mixed-Matrix-System eingesetzt wird.

Bevorzugte phosphoreszierende Emitter zur Verwendung in Mixed-Matrix-Systemen enthaltend die erfindungsgemäßen Verbindungen sind die in einer folgenden Tabelle aufgeführten phosphoreszierenden Emitter.

In einer weiteren bevorzugten Ausführungsform der Erfindung werden die Verbindungen gemäß Formel (I) als Lochtransportmaterial eingesetzt. Die Verbindungen werden dann bevorzugt in einer Lochtransportschicht, einer Elektronenblockierschicht oder einer Lochinjektionsschicht eingesetzt. Bevorzugt weisen die Verbindungen der Formel (I) in diesem Fall keine Gruppe X auf, d.h. die Verbindungen weisen Gruppen A auf, die Arylaminogruppen darstellen.

Eine Lochtransportschicht gemäß der vorliegenden Anmeldung ist eine Schicht mit lochtransportierender Funktion; welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

In einer weiteren Ausführungsform der Erfindung wird die Verbindung gemäß Formel (I) als Elektronentransportmaterial in einer Elektronentransportschicht oder Elektroneninjektionsschicht oder Lochblockierschicht eingesetzt. Hierfür ist es bevorzugt, dass die Verbindung der Formel (I) eine Gruppe X aufweist, die eine Einfachbindung darstellt, so dass die Verbindung eine Carbazolgruppe enthält. Bevorzugt enthält die Verbindung bei dieser Verwendung zusätzlich eine oder mehrere elektronenziehende Gruppen, beispielsweise elektronenarme Heteroarylgruppen wie Pyrimidingruppen, Triazingruppen oder Benzimidazolgruppen.

Im Folgenden werden die in den erfindungsgemäßen elektronischen Vorrichtungen bevorzugt eingesetzten weiteren Funktionsmaterialien aufgeführt.

Die in der folgenden Tabelle aufgeführten Verbindungen stellen besonders geeignete phosphoreszierende Emitter dar.

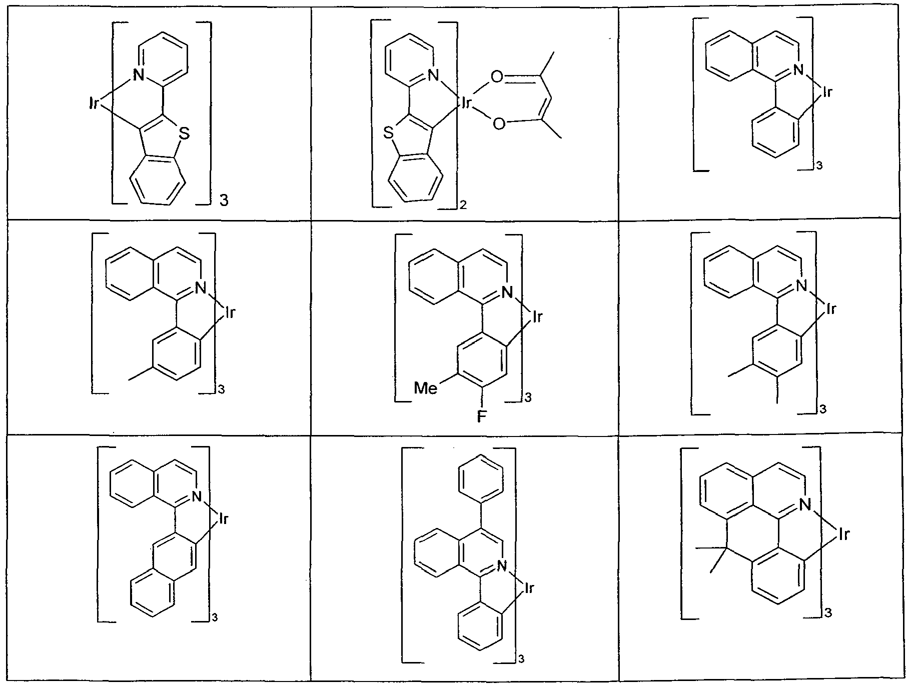

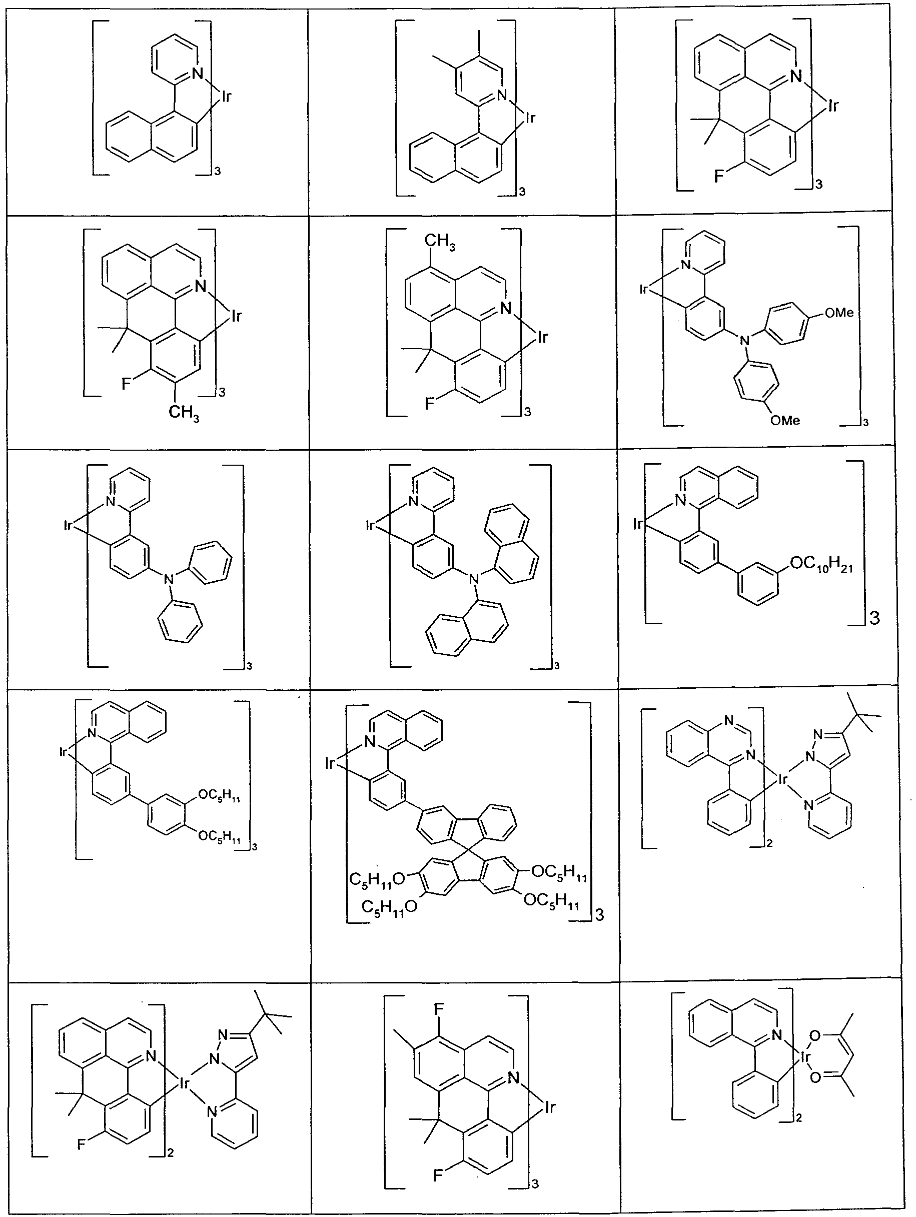

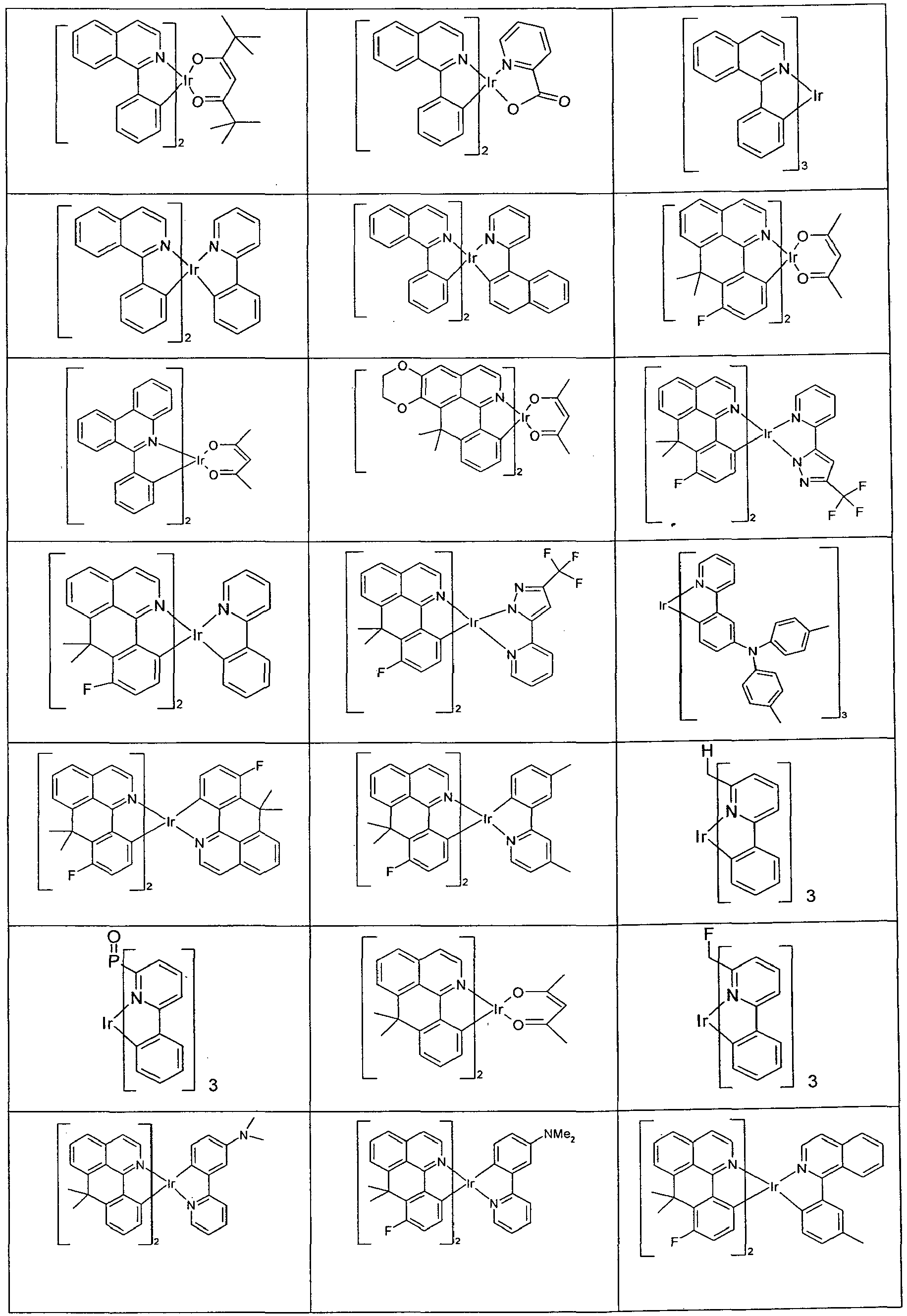

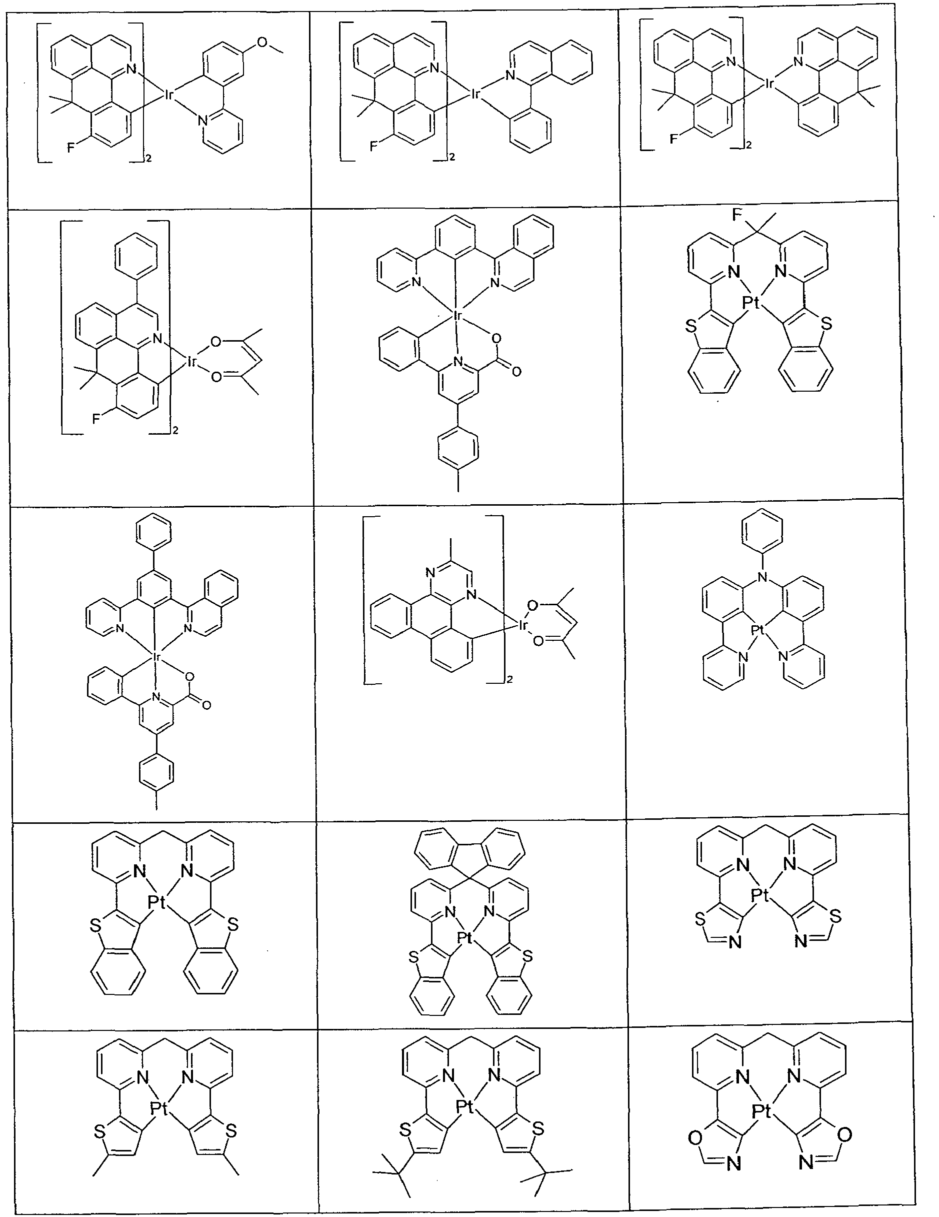

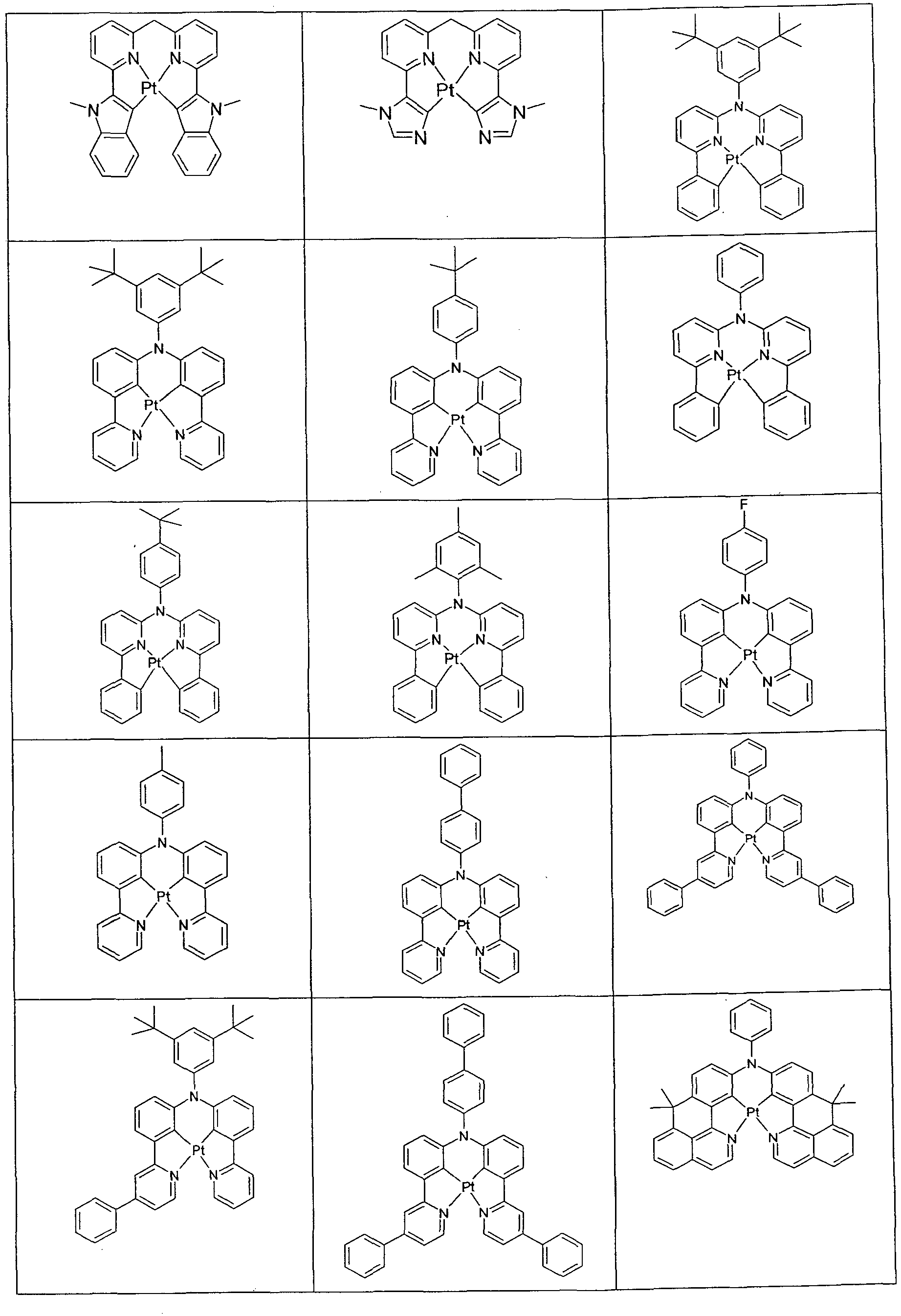

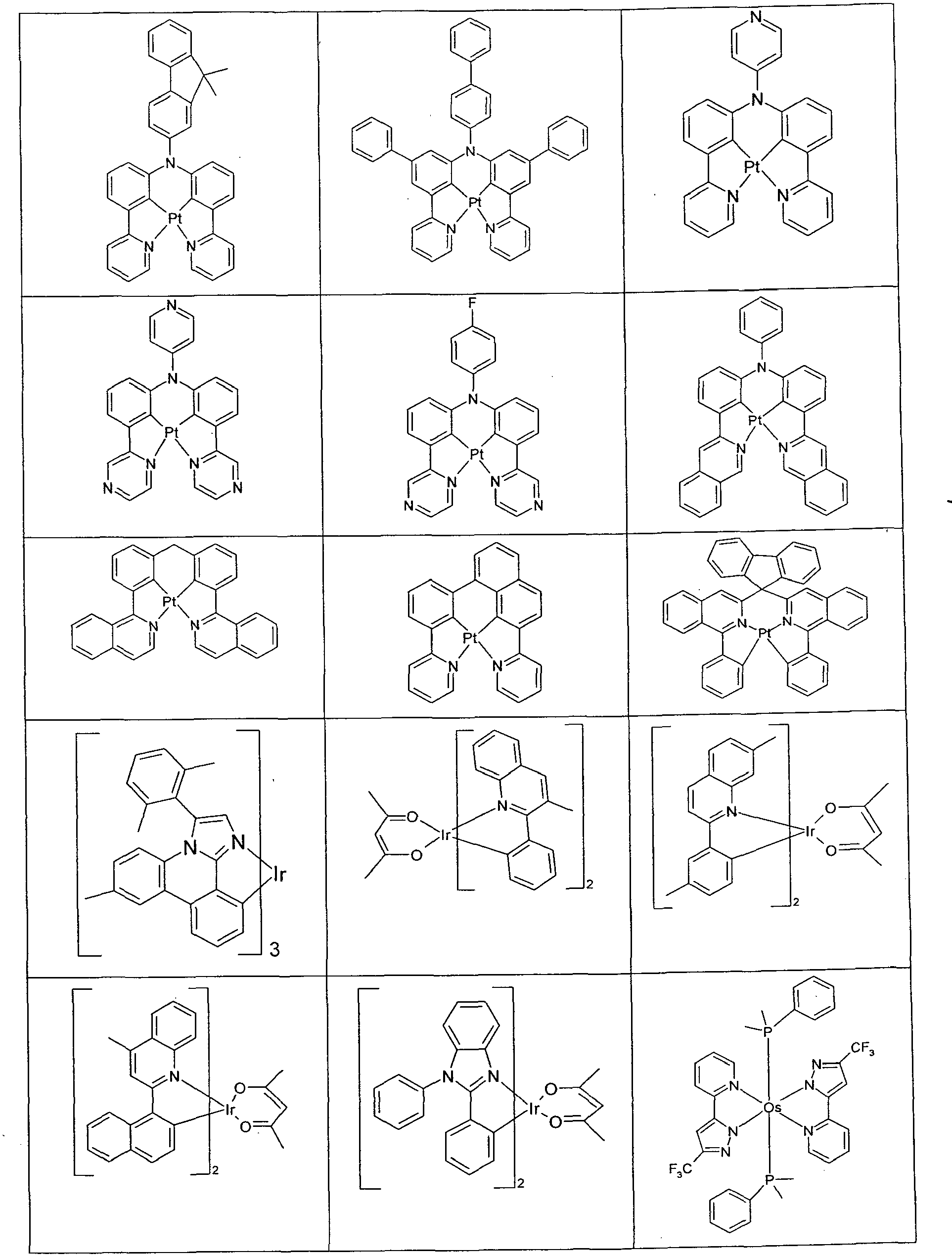

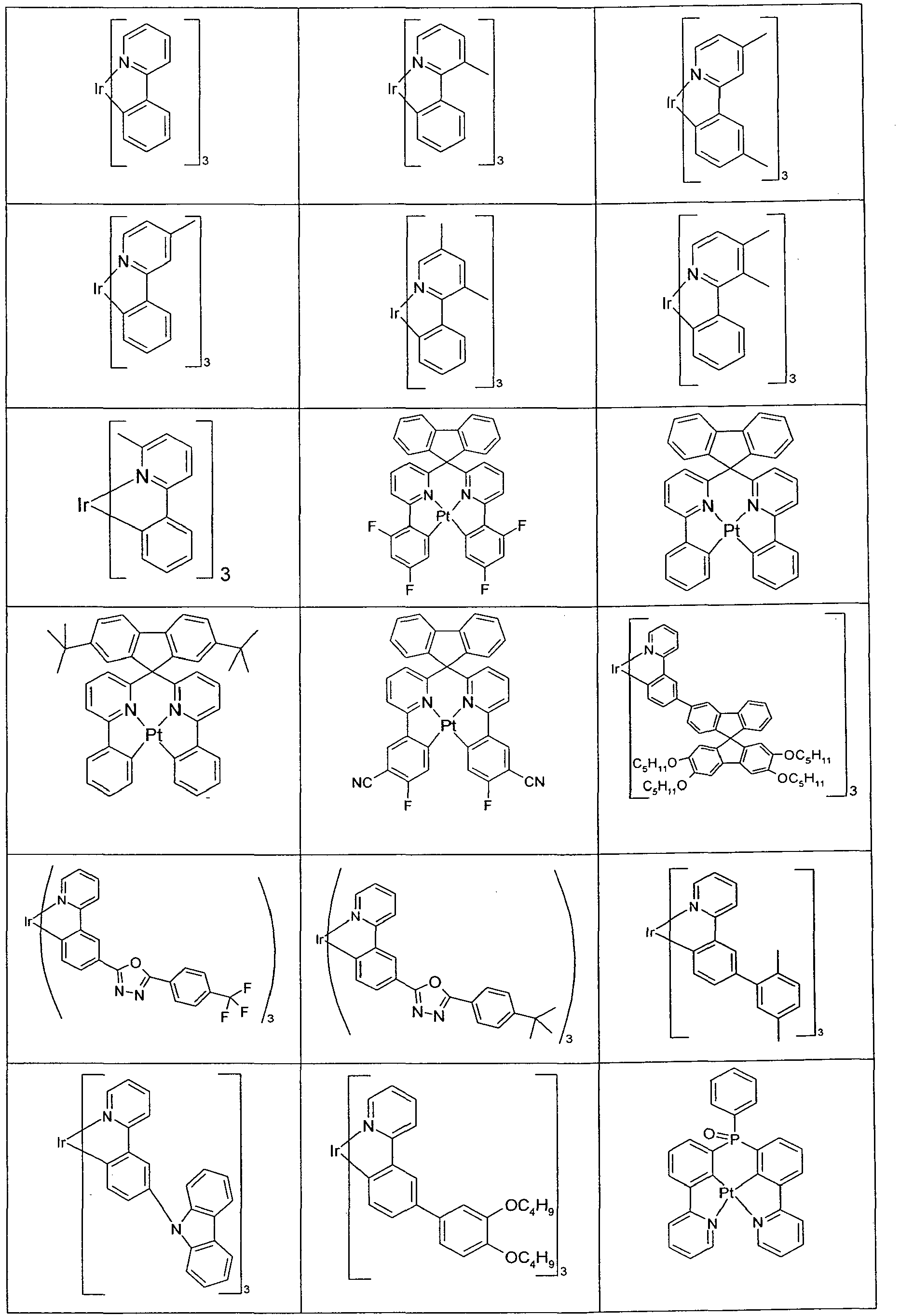

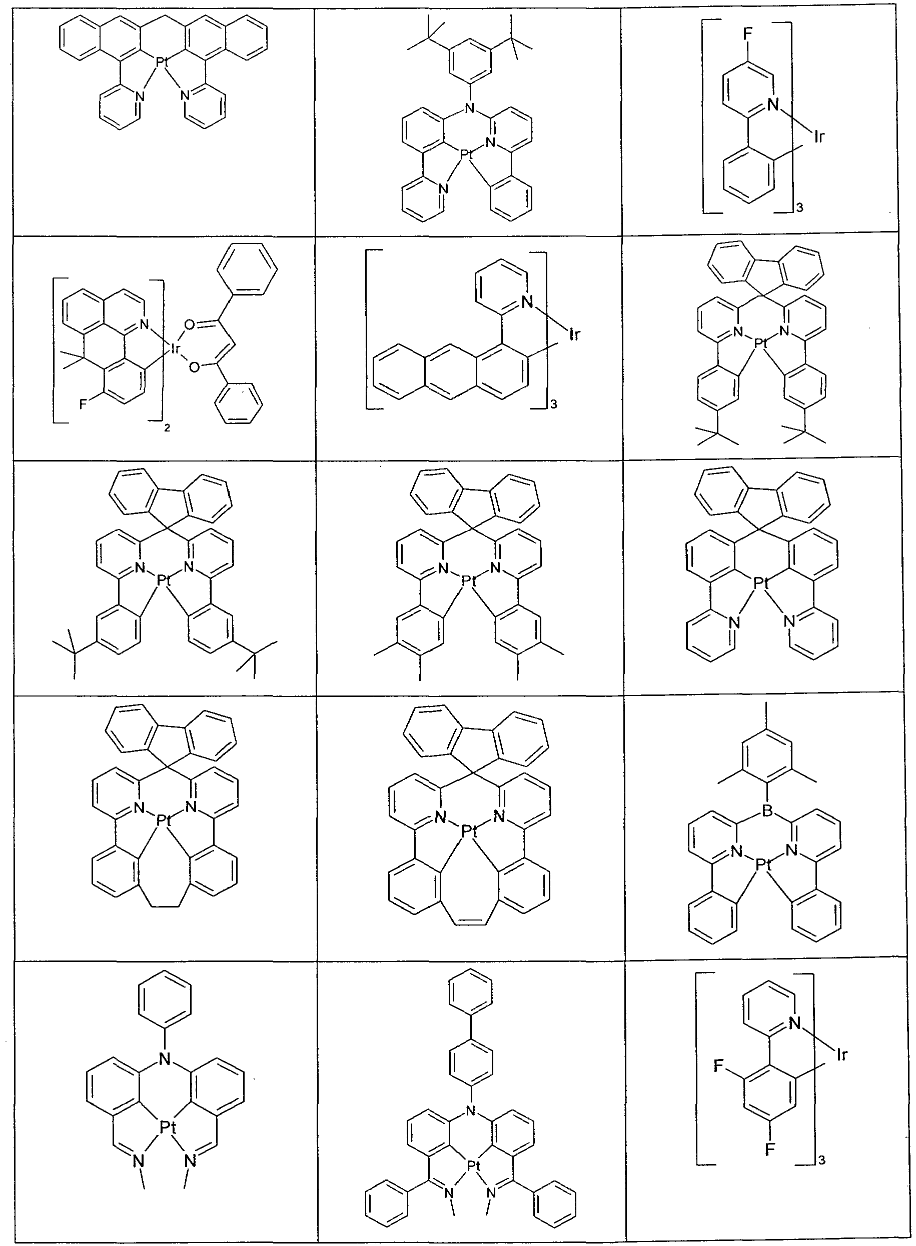

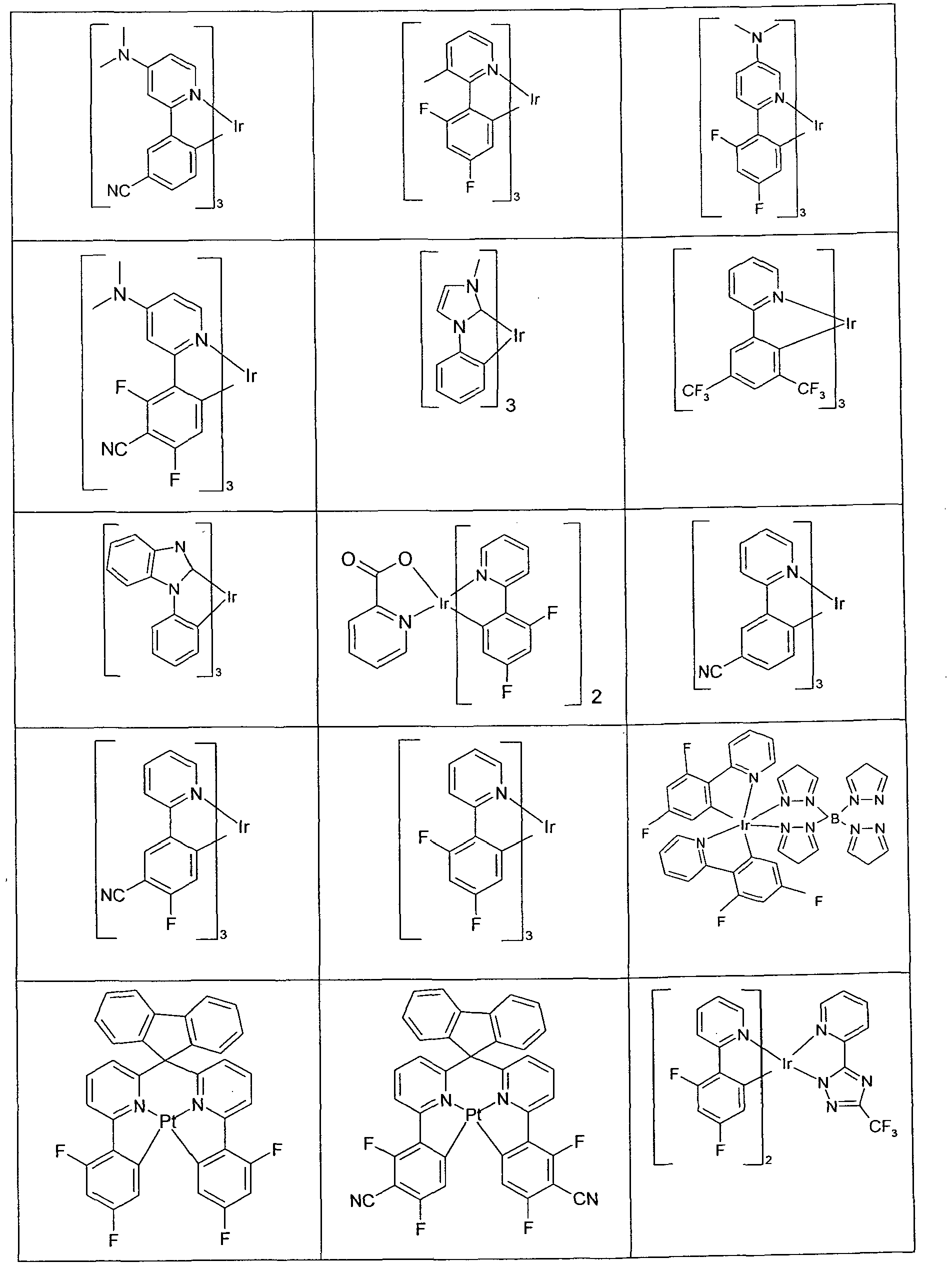

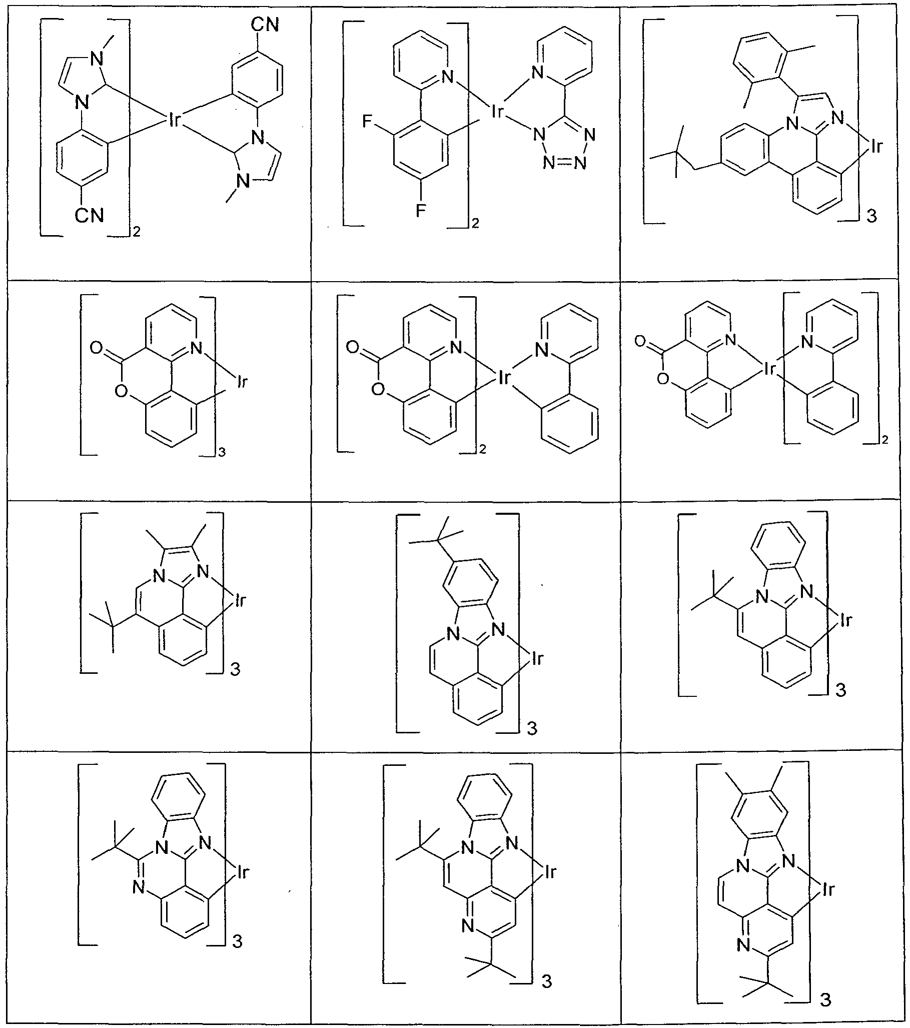

Bevorzugte fluoreszierende Emitter sind ausgewählt aus der Klasse der Arylamine. Unter einem Arylamin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und der noch nicht offengelegten EP 12004426.8 offenbarten Pyren-Arylamine. Ebenfalls bevorzugt sind die in der noch nicht offengelegten EP 12006239.3 offenbarten Benzoindenofluoren-Amine und die in der noch nicht offengelegten EP 13000012.8 offenbarten Benzofluoren-Amine.

Als Matrixmaterialien, bevorzugt für fluoreszierende Emitter, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende Emitter sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAlQ.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

Die folgenden Ausführungsbeispiele dienen zur Erläuterung der vorliegenden Erfindung. Sie sind nicht einschränkend auszulegen.

### A) Synthesebeispiele

Die Ausgangsverbindung 10-Brom-benzo[h]chinolin ist literaturbekannt (CAS-Nummer [152583-10-3]). Ein entsprechendes N-heterocyclisches Derivat ist als weitere Ausgangsverbindung unter CAS [66693-82-1] bekannt.

### Benzo[h]chinolin-10-yl-biphenyl-4-yl-(9,9-dimethyl-9H-fluoren-2-yl)-amin

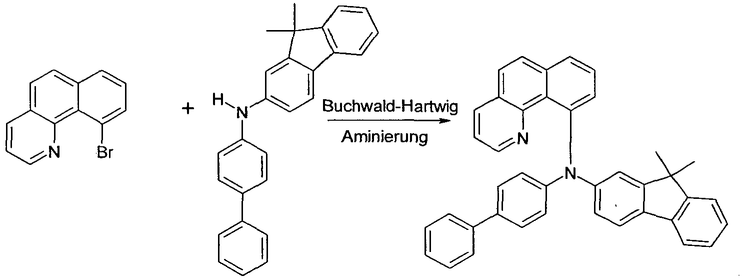

27,1 g Biphenyl-4-yl-(9,9-dimethyl-9H-fluoren-2-yl)-amin (75 mmol) und 19,4g 10-Bromo-benzo[h]chinolin (75 mmol) werden in 500 mL Toluol gelöst. Die Lösung wird entgast und mit N2 gesättigt. Danach wird sie mit 3,0 mL (3,0 mmol) einer Tri-tert-Butylphosphin-Lösung und 0,33 g (1,52 mmol) Palladium(II)acetat versetzt. Anschließend werden 11,2 g Natriumtert-butylat (116 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase zweimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%, die Ausbeute beträgt 28,3 g (70% d. Th.).

Analog dazu werden folgende Verbindungen hergestellt:

| | Edukt 1 | Edukt 2 | Produkt | Ausbeute |
|---|---|---|---|---|
| **1-2** | 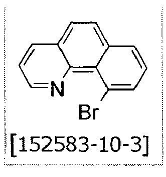 | 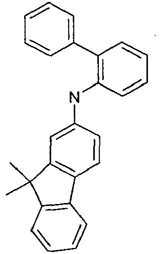 | 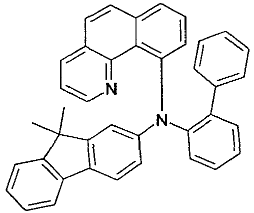 | 68% |
| **1-3** | 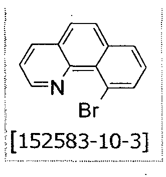 | 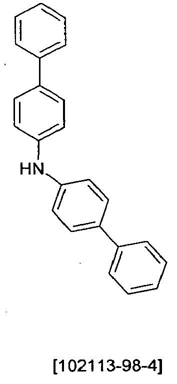 | 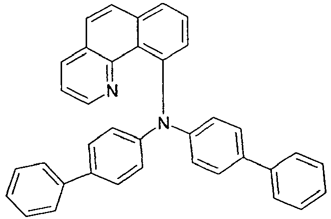 | 78% |
| **1-4** | 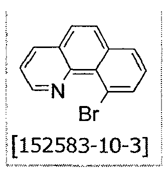 | 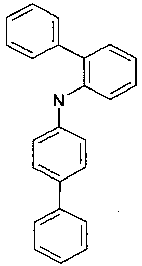 | 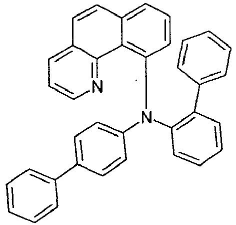 | 74% |
| **1-5** | 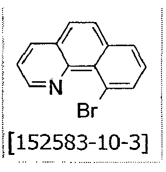 | 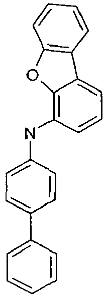 | 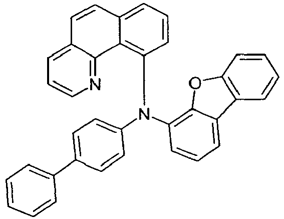 | 78% |
| **1-6** | 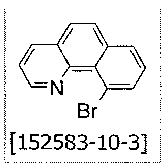 | 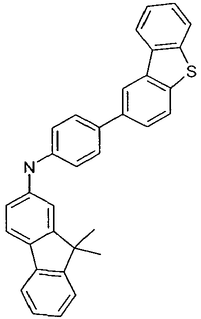 | 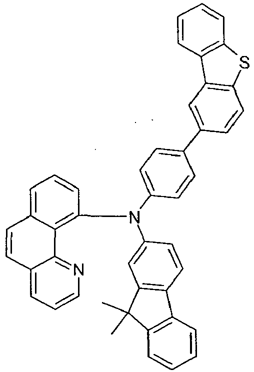 | 62% |
| **1-7** | 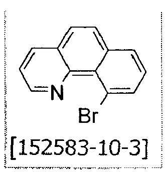 | 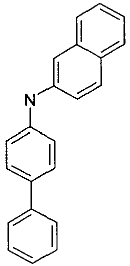 | 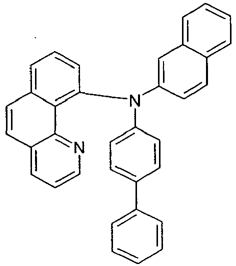 | 75% |
| **1-8** | 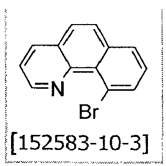 | 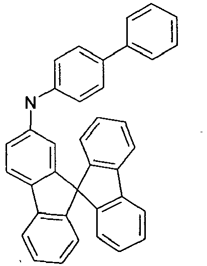 | 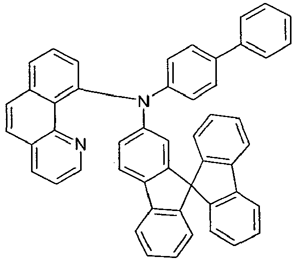 | 68% |
| **1-9** | 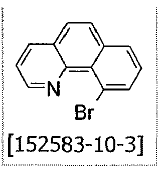 | 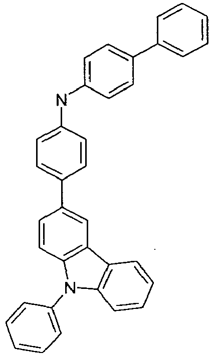 | 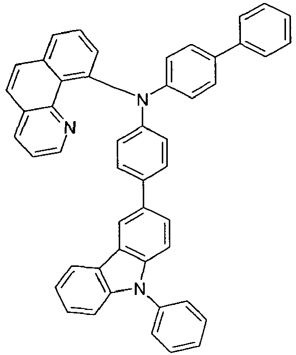 | 71% |
| **1-10** | 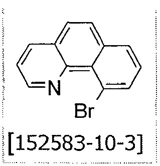 | 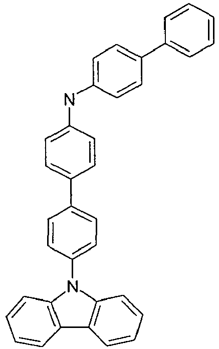 | 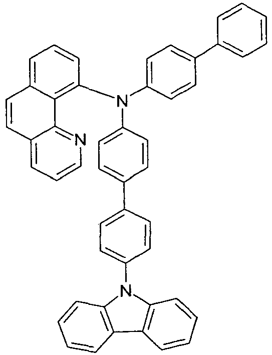 | 75% |
| **1-11** | 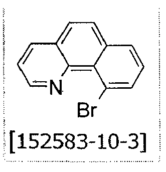 | 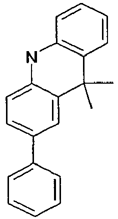 | 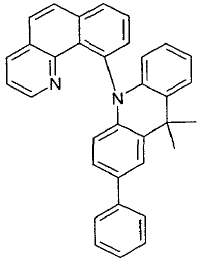 | 70% |
| **1-12** | 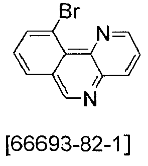 | 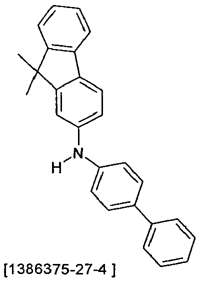 | 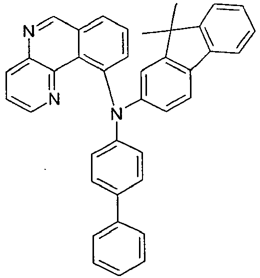 | 56% |
| **1-13** | 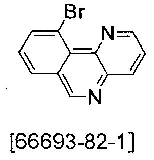 | 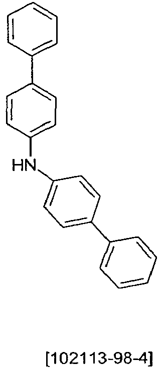 | 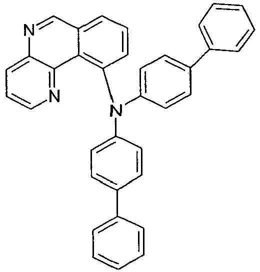 | 63% |
| **1-14** | 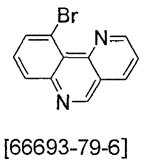 | 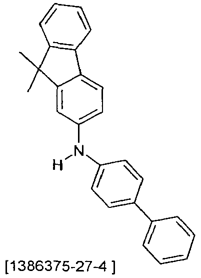 | 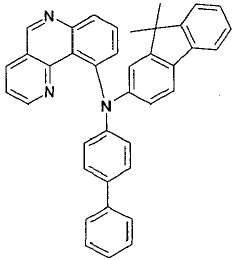 | 58% |
| **1-15** | 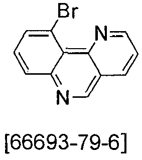 | 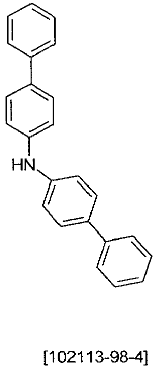 | 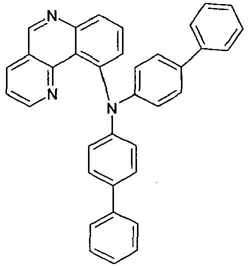 | 67% |
| **1-16** | 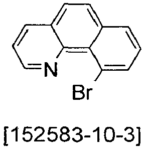 | 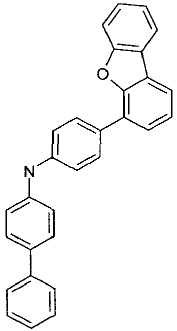 | 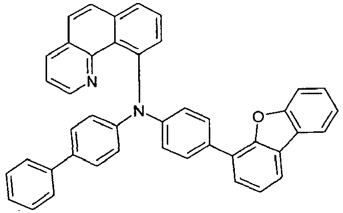 | 78% |

### Beispiel 2-1:

### (4-Benzo[h]chinolin-10-yl-phenyl)-biphenyl-4-yl-(9,9-dimethyl-9H-fluoren-2-yl)-amin

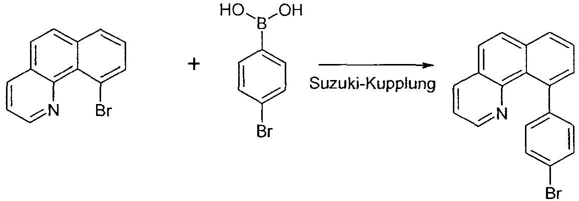

15,47 g (75 mmol) 4-Brom-benzolboronsäure, 19,4 g 10-Bromo-benzo[h]chinolin (75 mmol) und 110 mL einer 2M NaHCO₃ enthaltenden wässrigen Lösung (163 mmol) werden in 500 mL Dimethoxyethan suspendiert. Zu dieser Suspension werden 3,0 g (3,45 mmol) Tetrakis-(triphenyl)phosphin-palladium(0) gegeben, und die Reaktionsmischung wird 22 h unter Rückfluss erhitzt. Nach Erkalten wird die organische Phase abgetrennt, über Kieselgel filtriert, viermal mit 400 mL Wasser gewaschen und anschließend zur Trockene eingeengt. Nach Filtration des Rohprodukts über Kieselgel mit Heptan/Toluol (10:1) erhält man 39 g (71 %) 10-(4-Bromo-phenyl)-benzo[h]chinolin.

Analog dazu werden folgende Verbindungen hergestellt:

| | Edukt 1 | Edukt 2 | Produkt | Ausbeute |
|---|---|---|---|---|
| Int-2-2 | 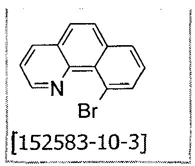 | 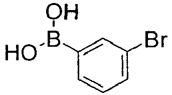 | 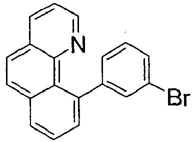 | 58% |
| Int-2-3 | 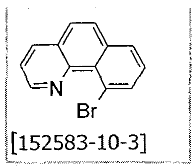 | 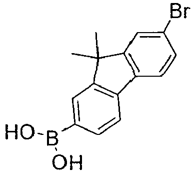 | 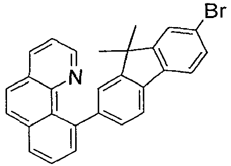 | 49% |
| Int-2-4 | 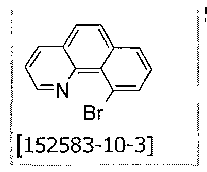 | 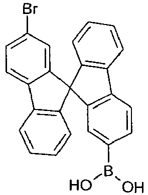 | 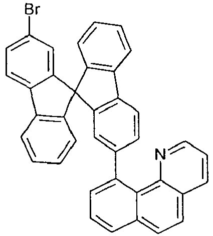 | 51% |
| Int-2-5 | 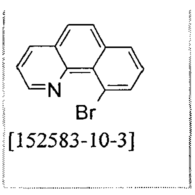 | 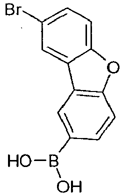 | 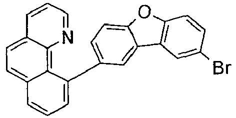 | 56% |
| Int-2-6 | 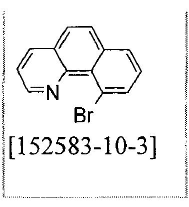 | 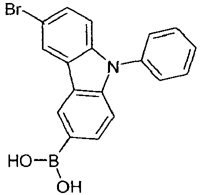 | 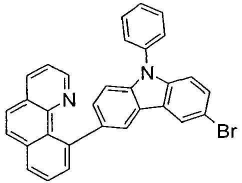 | 56% |
| Int-2-7 | 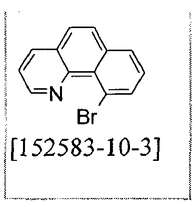 | 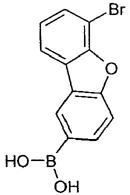 | 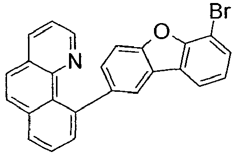 | 54% |
| Int-2-8 | 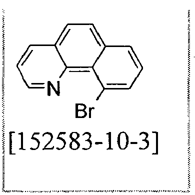 | 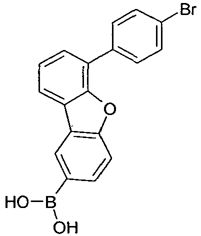 | 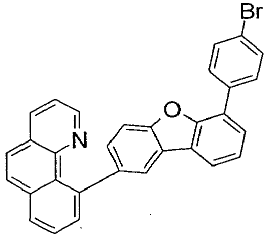 | 49% |

### Beispiel 3-1:

### (4-Benzo[h]chinolin-10-yl-phenyl)-biphenyl-4-yl-(9,9-dimethyl-9H-fluoren-2-yl)-amin

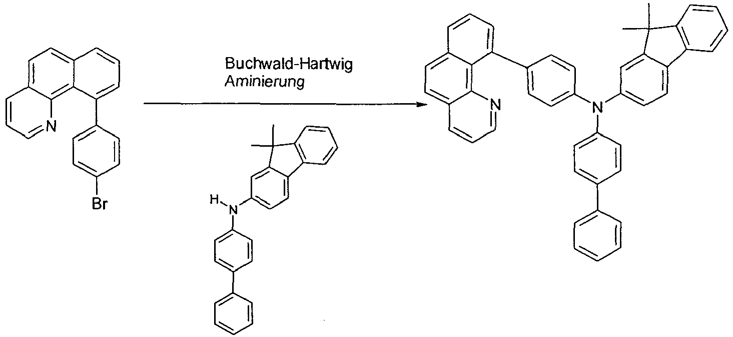

16,27 g Biphenyl-2-yl-(9,9-dimethyl-9H-fluoren-2-yl)-amin (45 mmol) und 15,0 g Produkt aus Beispiel 2-1 (45 mol) werden in 300 mL Toluol gelöst. Die Lösung wird entgast und mit N₂ gesättigt. Danach wird sie mit 2,4 g (2,9 mmol) einer Tri-tert-Butylphosphin-Lösung und 0,20 g (0,9 mmol) Palladium(II)acetat versetzt. Anschließend werden 6,68 g Natrium-tert-butylat (67,5 mmol) zugegeben. Die Reaktionsmischung wird 16 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohprodukts über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 20 g (60% d. Th.).

Analog dazu werden folgende Verbindungen hergestellt:

| | Edukt 1 | Edukt 2 | Produkt | Ausbeute |
|---|---|---|---|---|
| **3-2** | 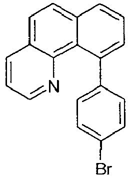 | 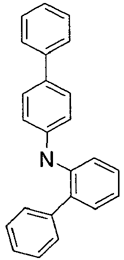 | 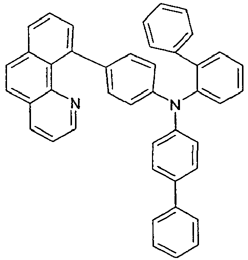 | 65% |
| **3-3** | 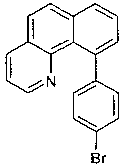 | 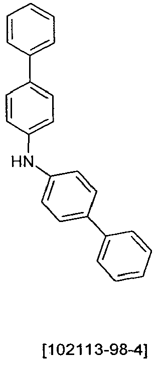 | 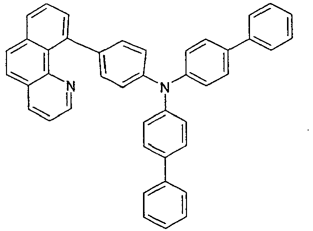 | 75% |
| **3-4** | 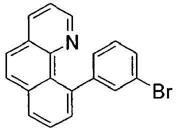 | 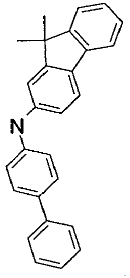 | 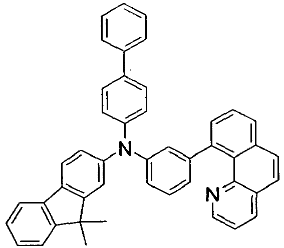 | 65% |
| **3-5** | 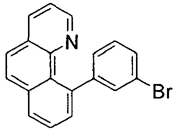 | 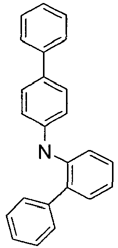 | 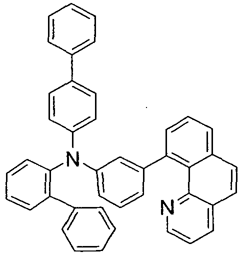 | 55% |
| **3-6** | 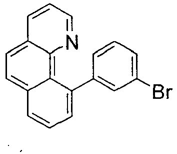 | 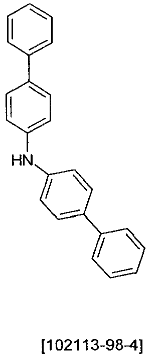 | 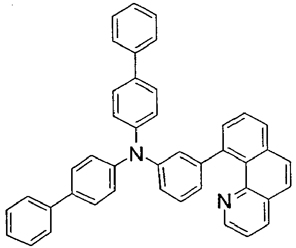 | 74% |
| **3-7** | 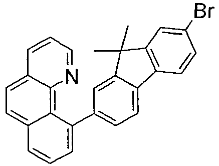 | 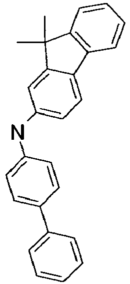 | 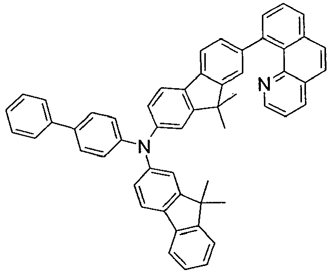 | 62% |
| **3-8** | 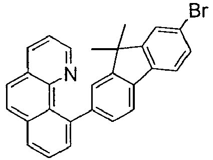 | 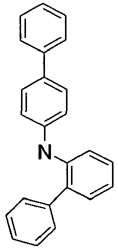 | 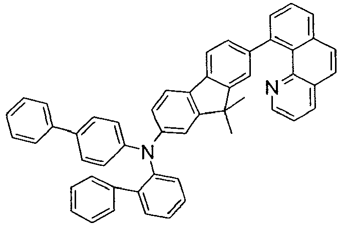 | 55% |
| **3-9** | 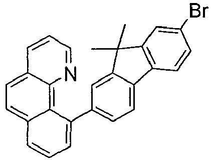 | 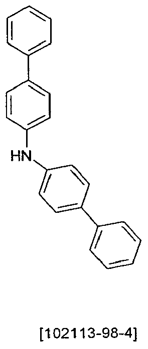 | 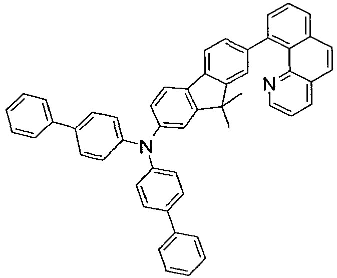 | 64% |
| **3-10** | 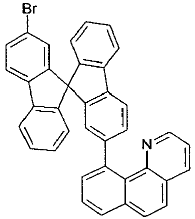 | 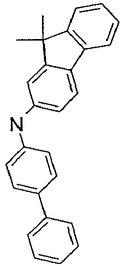 | 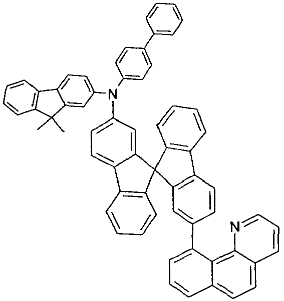 | 52% |
| **3-11** | 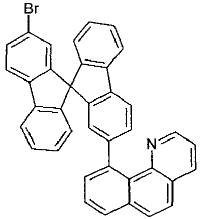 | 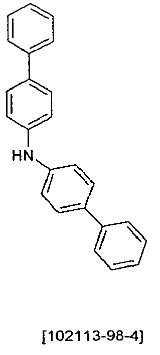 | 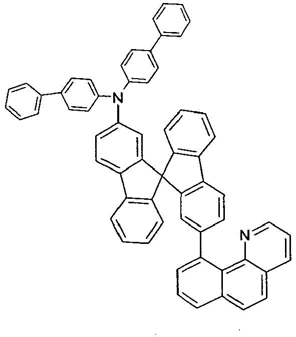 | 44% |
| **3-12** | 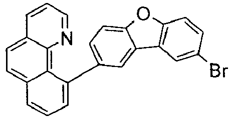 | 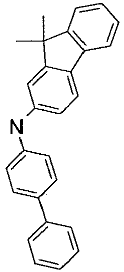 | 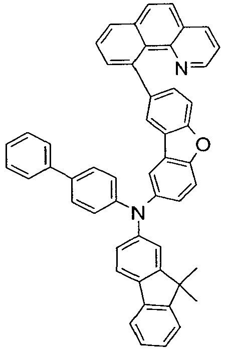 | 62% |
| **3-13** | 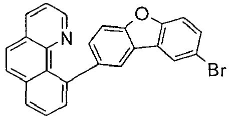 | 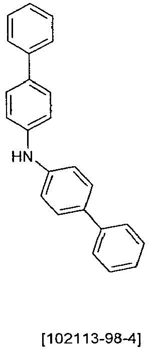 | 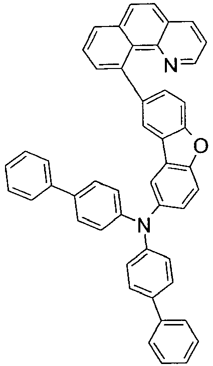 | 64% |
| **3-14** | 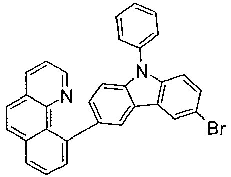 | 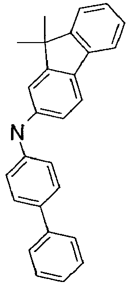 | 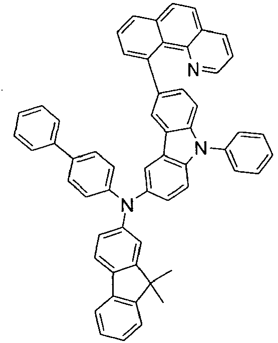 | 62% |
| **3-15** | 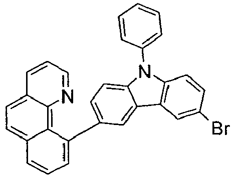 | 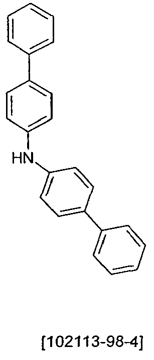 | 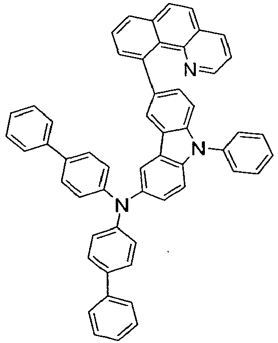 | 64% |
| **3-16** | 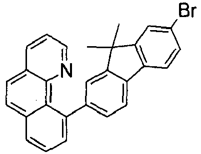 | 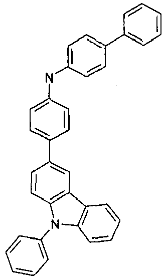 | 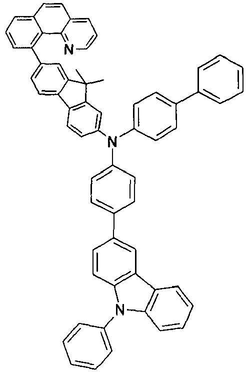 | 76% |
| **3-17** | 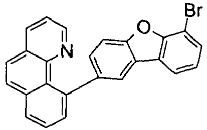 | 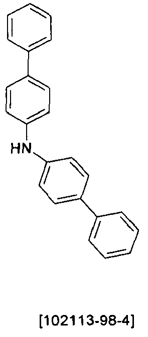 | 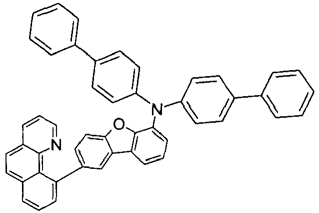 | 64% |
| **3-18*** Nicht gemäß Ansprüchen | 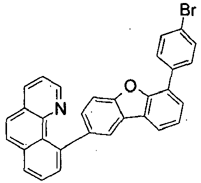 | 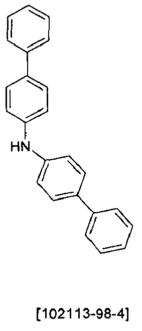 | 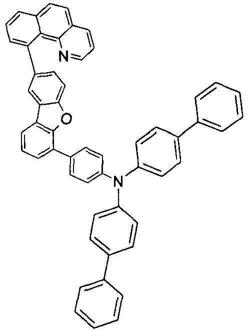 | 64% |

### Beispiel 4-1:

### 10-Benzo[h]chinolin-10-yl-12,12-dimethyl-10,12-dihydro-10-aza-indeno[2,1-b] fluoren

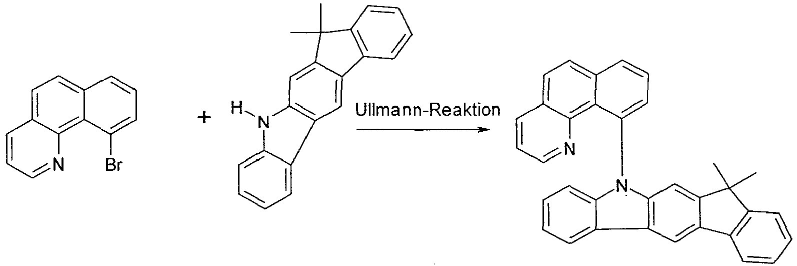

30 g (116 mmol) 10-Brom-benzo[h]chinolin, 32 g (116 mmol) 2,12-Dimethyl-10-phenyl-10,12-dihydro-10-aza-indeno[2,1-b]fluoren, 32g (232 mmol) Kaliumcarbonat, 2,6 g (11,6 mmol)1,3-Di(2-pyridyl)-1,3-propandion und 2,2 (11,6 mmol) Kupferiodid werden in 1000 mL DMF vorgelegt. Die Lösung wird entgast, mit N₂ gesättigt und 60 h auf 100°C erhitzt. Danach werden Wasser und Toluol zugegeben, die Phasen getrennt und die organische Phase zweimal mit Wasser gewaschen und über Na₂SO₄ getrocknet und einrotiert. Nach Filtration des Rohprodukts über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 37 g (71% d. Th.).

Analog dazu werden folgende Verbindungen hergestellt:

| | Edukt 1 | Edukt 2 | Produkt | Ausbeute |
|---|---|---|---|---|
| **4-2** | 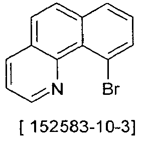 | 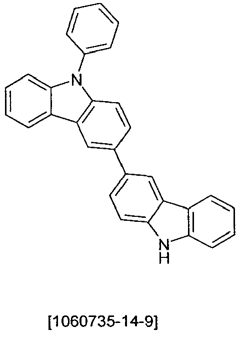 | 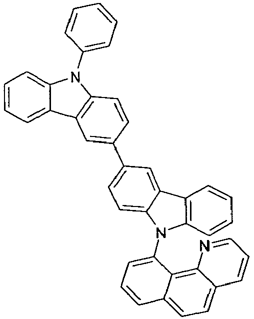 | 64% |
| **4-3** | 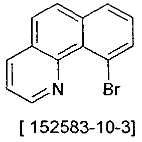 | 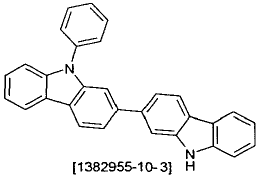 | 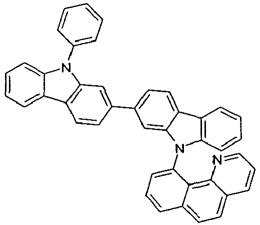 | 60% |
| **4-4** | 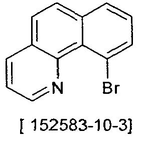 | 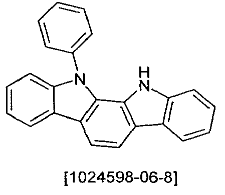 | 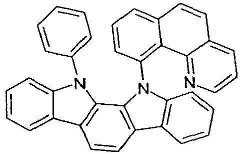 | 69% |
| **4-5** | 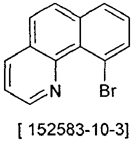 | 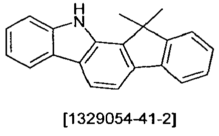 | 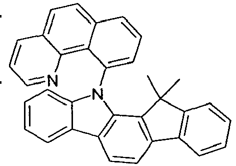 | 73% |
| **4-6** | 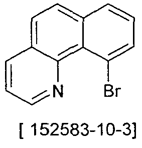 | 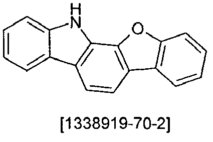 | 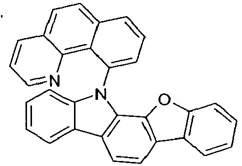 | 67% |
| **4-7** | 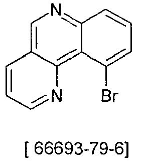 | 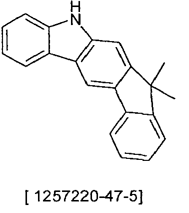 | 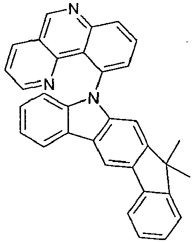 | 67% |
| **4-8** | 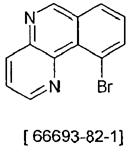 | 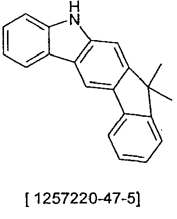 | 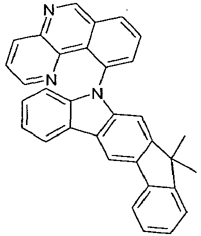 | 63% |
| **4-9** | 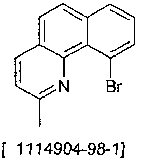 | 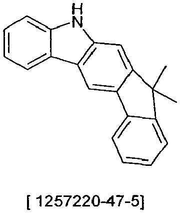 | 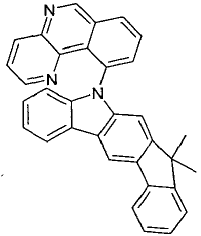 | 62% |
| **4-10** | 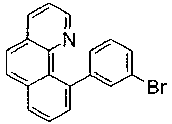 | 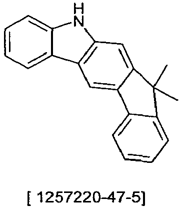 | 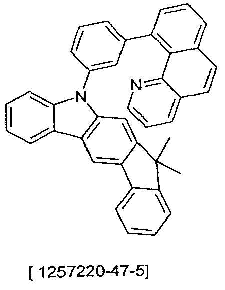 | 72% |
| **4-11** | 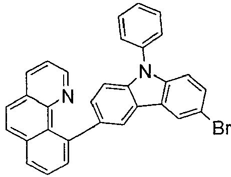 | 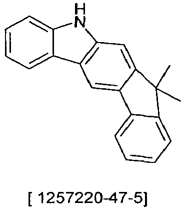 | 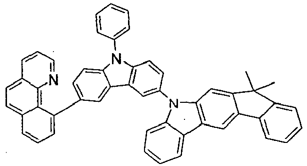 | 59% |

Analog dazu werden folgende Verbindungen mit 58 mmol Carbazol-Derivat hergestellt:

| | Edukt 1 | Edukt 2 | Produkt | Ausbeute |
|---|---|---|---|---|
| **4-12** | 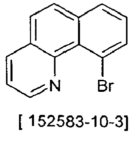 | 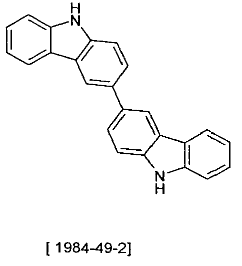 | 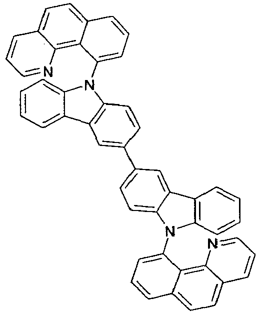 | 67% |
| **4-13** | 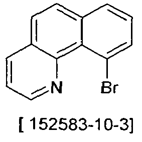 | 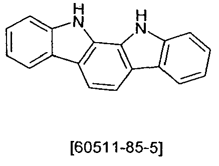 | 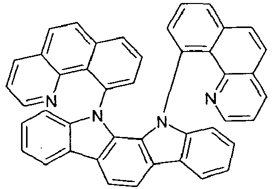 | 72% |
| **4-14** | 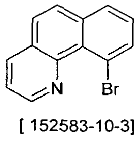 | 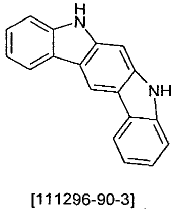 | 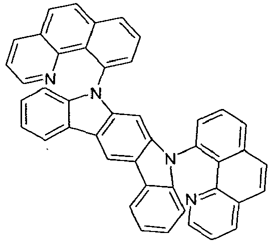 | 78% |
| **4-15** | 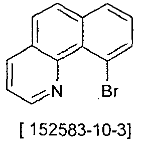 | 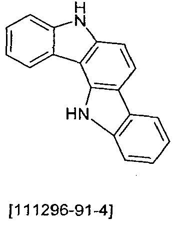 | 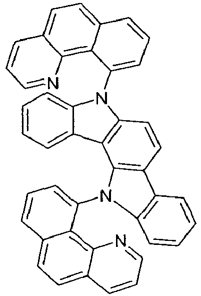 | 70% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden erfindungsgemäßen Beispielen E1 bis E6 und im Referenzbeispiel V1 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht HIL1 / Lochtransportschicht HTL / p-dotierte Lochtransportschicht HIL2 / Lochtransportschicht EBL / Emissionsschicht EML / Elektronentransportschicht ETL / Elektroneninjektionsschicht EIL und abschließend eine Kathode. Die Kathode wird durch eine 100 nm dicke Aluminiumschicht gebildet. Die zur Herstellung der OLEDs benötigten Materialien sind in Tabelle 1 gezeigt, der Aufbau der verschiedenen hergestellten elektronischen Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:TEG(10%) bedeutet hierbei, dass das Material H1 in einem Volumenanteil von 90% und TEG in einem Anteil von 10% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 2 mA/cm² bezeichnet die externe Quanteneffizienz bei einer Stromdichte von 2 mA/cm². LD80 @ 10000 cd/m², angegeben in Stunden, ist die Zeit, bis zu der die OLED bei konstantem Strom auf 80 % der Anfangsintensität, also auf 8000 cd/m², abgefallen ist.

| Tabelle 1: Strukturen der verwendeten Materialien | | |
|---|---|---|
| 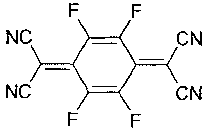 | 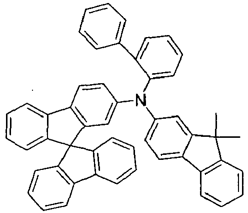 | 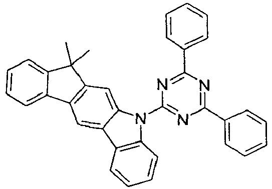 |
| F4TCNQ | HIM | H1 |
| 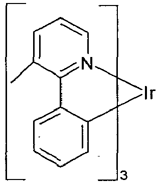 | 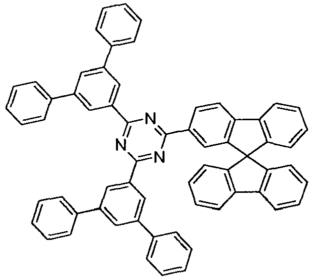 | 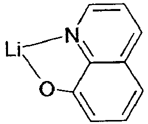 |
| TEG | ETM | LiQ |
| 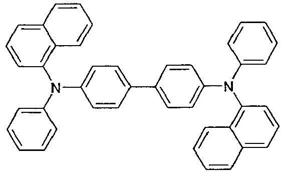 | 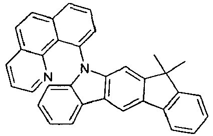 | 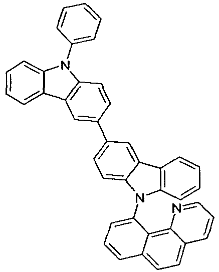 |
| NPB | HTM1 | HTM2 |
| 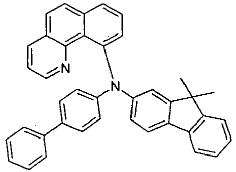 | 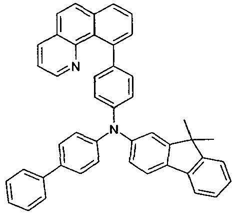 | 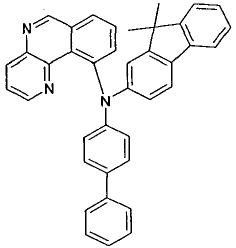 |
| HTM3 | HTM4 | HTM5 |
| 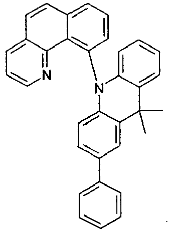 | | |
| HTM6 | | |

| Tabelle 2: Aufbau der OLEDs | | | | | | | |
|---|---|---|---|---|---|---|---|
| ***Bsp.*** | ***HIL1*** | ***HTL*** | ***HIL2*** | ***EBL*** | ***EML*** | ***ETL*** | ***EIL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM1:F4TCNQ(3%) 20 nm* | *HIM1 210 nm* | *NPB:F4TCNQ(3%) 20 nm* | *NPB 20 nm* | *H1:TEG(10%) 30 nm* | *ETM(50%):LiQ(50%) 40 nm* | *LiQ 1 nm* |
| *E1* | *HIM1:F4TCNQ(3%) 20 nm* | *HIM1 210 nm* | *HTM1:F4TCNQ(3%) 20 nm* | *HTM1 20 nm* | *H1:TEG(10%) 30 nm* | *ETM(50%):LiQ(50%) 40 nm* | *LiQ 1 nm* |
| *E2* | *HTM2:F4TCNQ(3%) 20 nm* | *HTM2 210 nm* | *NPB:F4TCNQ(3%) 20 nm* | *NPB 20 nm* | *H1:TEG(10%) 30 nm* | *ETM(50%):LIQ(50%) 40 nm* | *LiQ 1 nm* |
| *E3* | *HTM3:F4TCNQ(3%) 20 nm* | *HTM3 210 nm* | *NPB:F4TCNQ(3%) 20 nm* | *NPB 20 nm* | *H1:TEG(10%) 30 nm* | *ETM(50%):LiQ(50%) 40 nm* | *LiQ 1 nm* |
| *E4* | *HTM4:F4TCNQ(3%) 20 nm* | *HTM4 210 nm* | *NPB:F4TCNQ(3%) 20 nm* | *NPB 20 nm* | *H1:TEG(10%) 30 nm* | *ETM(50%):LiQ(50%) 40 nm* | *LiQ 1 nm* |
| *E5* | *HTM5:F4TCNQ(3%) 20 nm* | *HTM5 210 nm* | *NPB:F4TCNQ(3%) 20 nm* | *NPB 20 nm* | *H1:TEG(10%) 30 nm* | *ETM(50%):LiQ(50%) 40 nm* | *LiQ 1 nm* |
| *E6* | *HIM1:F4TCNQ(3%) 20 nm* | *HIM1 210 nm* | *HTM6:F4TCNQ(3%) 20 nm* | *HTM6 20 nm* | *H1:TEG(10%) 30 nm* | *ETM(50%):LiQ(50%) 40 nm* | *LiQ 1 nm* |

Die folgenden Beispiele zeigen die Verwendung der erfindungsgemäßen Verbindungen HTM1 bis HTM6 als Lochtransportmaterialien in einer phosphoreszierenden OLED. Die emittierende Schicht ist dabei aus den Materialien TEG1 als Emittermaterial und H1 als Matrixmaterial aufgebaut.

### Device-Beispiele 1 und 2

Es wurde eine grün phosphoreszierende Referenzvorrichtung V1 hergestellt und mit den erfindungsgemäßen Vorrichtungen E1 und E2 verglichen. Die Referenzvorrichtung V1 hat bei einer Stromdichte von 2 mA/cm² eine externe Quanteneffizienz von 11.7 % und eine Lebensdauer (LD80 @ 10000 cd/m²) von 90 h. Verglichen damit hat die erfindungsgemäße Vorrichtung E1 eine bessere externe Quanteneffizienz von 15.5 % und eine bessere LD80 bei 10000 cd/m² von 120 h. Die erfindungsgemäße Vorrichtung E2 hat eine Quanteneffizienz von 15.8 % und eine Lebensdauer von 100 h.

### Device-Beispiele 3 bis 6

Es wurde eine grün phosphoreszierende Referenzvorrichtung V1 hergestellt und mit den erfindungsgemäßen Vorrichtungen E3 bis E6 verglichen. Die Referenzvorrichtung V1 hat bei einer Stromdichte von 2 mA/cm² eine externe Quanteneffizienz von 11.7 %. Verglichen damit haben die erfindungsgemäßen Vorrichtungen E3 - E6 eine bessere externe Quanteneffizienz von 13.3 % (E3), 14.2 % (E4), 13.7 % (E5) und 12.8 % (E6).

Die Beispiele zeigen, dass die erfindungsgemäßen Verbindungen sehr gut zur Verwendung in OLEDs geeignet sind, insbesondere zur Verwendung als lochtransportierende Materialien. Die Verwendung ist jedoch nicht auf diese Funktion beschränkt. Die Materialien eignen sich beispielsweise auch zur Verwendung als Matrixmaterialien in der emittierenden Schicht.

## Patentansprüche

1. Verbindung einer Formel (I) 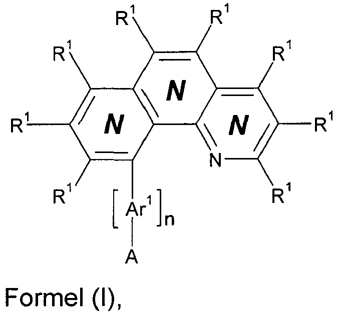 wobei gilt:
A ist gewählt aus Gruppen der Formeln (A-1) oder (A-2) 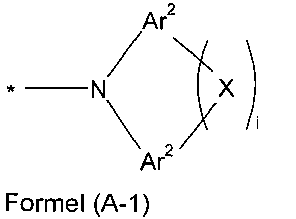 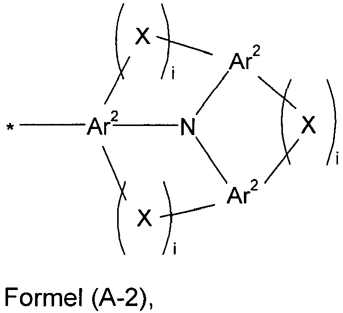 die über die mit * markierte Bindung gebunden sind,
Ar¹ ist bei jedem Auftreten gleich oder verschieden gewählt aus Benzol, Pyridin, Pyrimidin, Triazin, Naphthalin, Chinolin, Fluoren, Spirobifluoren, Indenofluoren, Carbazol, Indenocarbazol, Indolocarbazol, Dibenzofuran und Dibenzothiophen, die jeweils mit einem oder mehreren Resten R¹ substituiert sein können;
Ar² ist bei jedem Auftreten gleich oder verschieden gewählt aus Benzol, Biphenyl, Terphenyl, Quaterphenyl, Pyridin, Pyrimidin, Pyrazin, Pyridazin, Triazin, Naphthalin, Chinolin, Fluoren, Spirobifluoren, Indenofluoren, Carbazol, Indenocarbazol, Indolocarbazol, Dibenzofuran und Dibenzothiophen, die jeweils mit einem oder mehreren Resten R¹ substituiert sein können;
X ist bei jedem Auftreten gleich oder verschieden eine Einfachbindung oder eine Gruppe gewählt aus BR², C(R²)₂, - C(R²)₂-C(R²)₂-, -C(R²)=C(R²)-, -C(R²)₂-O-, -C(R²)₂-NR²-, Si(R²)₂, C=O, NR², PR², P(=O)R², O, S, S=O oder SO₂;
R¹ ist bei jedem Auftreten gleich oder verschieden H, D, F, C(=O)R³, CN, Si(R³)₃, N(R³)₂, P(=O)(R³)₂, OR³, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches Ringsystem mit 5 bis 30 aromatischen Ringatomen, das mit einem oder mehreren Resten R³ substituiert sein kann, oder ein heteroaromatisches Ringsystem mit 5 bis 30 aromatischen Ringatomen, das mit einem oder mehreren Resten R³ substituiert sein kann, und das aus Gruppen gewählt aus Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin, Benzothiadiazol und Indenocarbazol besteht; zwei oder mehr Reste R¹ können miteinander verknüpft sein und einen Ring bilden;
R² ist bei jedem Auftreten gleich oder verschieden H, D, F, C(=O)R³, CN, Si(R³)₃, N(R³)₂, P(=O)(R³)₂, OR³, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das mit einem oder mehreren Resten R³ substituiert sein kann; zwei oder mehr Reste R² können miteinander verknüpft sein und einen Ring bilden;
R³ ist bei jedem Auftreten gleich oder verschieden H, D, F, C(=O)R⁴, CN, Si(R⁴)₃, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch-R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das mit einem oder mehreren Resten R⁴ substituiert sein kann; zwei oder mehr Reste R³ können miteinander verknüpft sein und einen Ring bilden;
R⁴ ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch D, F oder CN ersetzt sein können; zwei oder mehr Substituenten R⁴ können miteinander verknüpft sein und einen Ring bilden.
n ist 0, 1, 2 oder 3;
i ist bei jedem Auftreten gleich oder verschieden 0 oder 1; und
in den Benzolringen, in deren Mitte ein N gezeichnet ist, können jeweils ein oder mehrere Ringglieder -CR¹= durch -N= ersetzt sein.

2. Verbindung nach Anspruch 1, **dadurch gekennzeichnet, dass** in den Benzolringen, in deren Mitte ein N gezeichnet ist, keines oder genau ein oder zwei Ringglieder -CR¹= durch -N= ersetzt sind.

3. Verbindung nach Anspruch 1 oder 2, **dadurch gekennzeichnet, dass** in allen drei Benzolringen in Formel (I), in deren Mitte ein N gezeichnet ist, kein Ringglied -CR¹= durch N ersetzt ist.

4. Verbindung nach einem oder mehreren der Ansprüche 1 bis 3, **dadurch gekennzeichnet, dass** n gleich 0 ist.

5. Verbindung nach einem oder mehreren der Ansprüche 1 bis 4, **dadurch gekennzeichnet, dass** für Gruppen der Formel (A-2) keine, genau eine oder genau zwei Indices i gleich 1 sind, und die anderen Indices i gleich 0 sind.

6. Verbindung nach einem oder mehreren der Ansprüche 1 bis 5, **dadurch gekennzeichnet, dass** X bei jedem Auftreten gleich oder verschieden gewählt ist aus einer Einfachbindung oder einer Gruppe gewählt aus C(R²)₂, C=O, NR², O und S.

7. Verbindung nach einem oder mehreren der Ansprüche 1 bis 6, **dadurch gekennzeichnet, dass** sie als Substituent R¹, R², R³ und R⁴ keine kondensierte Aryl- oder Heteroarylgruppe mit mehr als 14 aromatischen Ringatomen aufweist.

8. Verbindung nach einem oder mehreren der Ansprüche 1 bis 7, **dadurch gekennzeichnet, dass** sie zusätzlich zur Gruppe A keine weitere Arylaminogruppe, keine weitere verbrückte Arylaminogruppe und keine weitere Carbazolgruppe umfasst.

9. Verbindung nach einem oder mehreren der Ansprüche 1 bis 8, **dadurch gekennzeichnet, dass** sie einer der Formeln (I-1) bis (I-3) entspricht
| | |
|---|---|
| 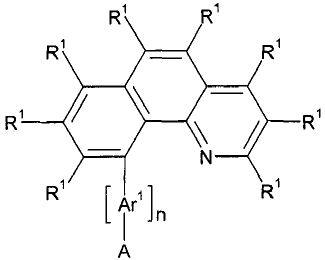 | 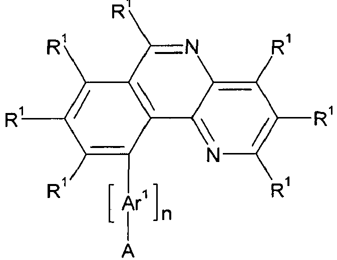 |
| Formel (I-1) | Formel (I-2) |
| | |
| 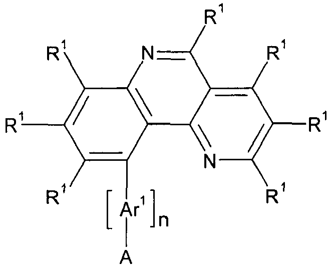 | |
| Formel (I-3) | |
wobei die auftretenden Gruppen und Indices gemäß einem oder mehreren der Ansprüche 1 bis 8 definiert sind.

10. Verfahren zur Herstellung einer Verbindung nach einem oder mehreren der Ansprüche 1 bis 9, **dadurch gekennzeichnet, dass** eine Verbindung einer Formel (Int-I) 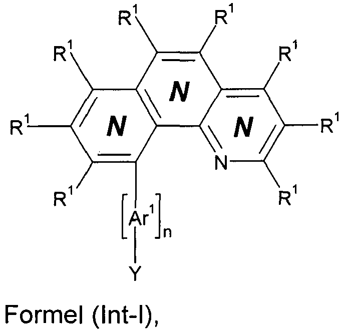 in einer Kupplungsreaktion weiter umgesetzt wird,
wobei die auftretenden variablen Gruppen und Indices wie für die Verbindung der Formel (I) nach einem oder mehreren der Ansprüche 1 bis 9 definiert sind, und wobei in den Benzolringen, in deren Mitte ein N gezeichnet ist, jeweils ein oder mehrere Ringglieder -CR¹= durch -N= ersetzt sein können, und wobei Y eine beliebige reaktive Gruppe darstellt.

11. Oligomer, Polymer oder Dendrimer enthaltend eine oder mehrere Verbindungen nach einem oder mehreren der Ansprüche 1 bis 9, wobei die Bindungen zum Polymer, Oligomer oder Dendrimer an beliebigen, in Formel (I) mit R¹ oder R² substituierten Positionen lokalisiert sein können.

12. Formulierung, enthaltend mindestens eine Verbindung nach einem oder mehreren der Ansprüche 1 bis 9 sowie mindestens ein Lösungsmittel.

13. Verwendung einer Verbindung nach einem oder mehreren der Ansprüche 1 bis 9 in einer elektronischen Vorrichtung.

14. Elektronische Vorrichtung, enthaltend Anode, Kathode sowie mindestens eine organische Schicht, wobei die organische Schicht mindestens eine Verbindung nach einem oder mehreren der Ansprüche 1 bis 9 enthält.

15. Elektronische Vorrichtung nach Anspruch 14, **dadurch gekennzeichnet, dass** sie ausgewählt ist aus der Gruppe bestehend aus organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, organischen lichtemittierenden elektrochemischen Zellen, organischen Laserdioden und organischen Elektrolumineszenzvorrichtungen.

16. Organische Elektrolumineszenzvorrichtung nach Anspruch 15, **dadurch gekennzeichnet, dass** die Verbindung nach einem oder mehreren der Ansprüche 1 bis 9 als Matrixmaterial in einer emittierenden Schicht oder als Lochtransportmaterial in einer lochtransportierenden Schicht vorliegt.

## Claims

1. Compound of a formula (I) 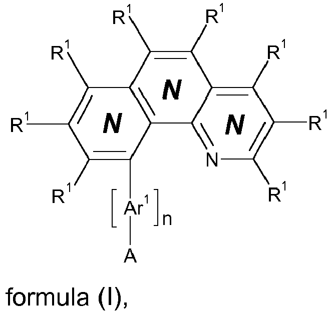 where:
A is selected from groups of the formulae (A-1) or (A-2) 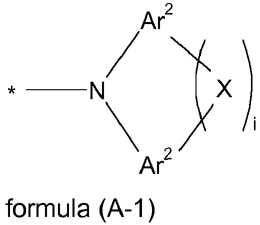 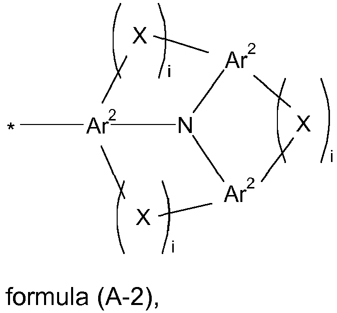 which are bonded via the bond marked with *,
Ar¹ is selected on each occurrence, identically or differently, from benzene, pyridine, pyrimidine, triazine, naphthalene, quinoline, fluorene, spirobifluorene, indenofluorene, carbazole, indenocarbazole, indolocarbazole, dibenzofuran and dibenzothiophene, which may in each case be substituted by one or more radicals R¹;
Ar² is selected on each occurrence, identically or differently, from benzene, biphenyl, terphenyl, quaterphenyl, pyridine, pyra-zine, pyridazine, pyrimidine, triazine, naphthalene, quinoline, fluorene, spirobifluorene, indenofluorene, carbazole, indenocarbazole, indolocarbazole, dibenzofuran and dibenzothiophene, which may in each case be substituted by one or more radicals R¹;
X is on each occurrence, identically or differently, a single bond or a group selected from BR², C(R²)₂, -C(R²)₂-C(R²)₂-, -C(R²)=C(R²)-, -C(R²)₂-O-, -C(R²)₂-NR²-, Si(R²)₂, C=O, NR², PR², P(=O)R², O, S, S=O or SO₂;
R¹ is on each occurrence, identically or differently, H, D, F, C(=O)R³, CN, Si(R³)₃, N(R³)₂, P(=O)(R³)₂, OR³, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic ring system having 5 to 30 aromatic ring atoms, which may be substituted by one or more radicals R³, or a heteroaromatic ring system having 5 to 30 aromatic ring atoms, which may be substituted by one or more radicals R³ and which consists of groups selected from benzene, naphthalene, anthracene, phenanthrene, pyrene, dihydropyrene, chrysene, perylene, triphenylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, phenothiazine, phenoxazine, pyrazole, indazole, imidazole, benzimidazole, naphthimidazole, phenanthrimidazole, pyridimidazole, pyrazinylimidazole, quinoxalinylimidazole, oxazole, benzoxazole, naphthoxazole, arithroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine, benzothiadiazol and indenocarbazole; two or more radicals R¹ may be linked to one another and form a ring;
R¹ is on each occurrence, identically or differently, H, D, F, C(=O)R³, CN, Si(R³)₃, N(R³)₂, P(=O)(R³)₂, OR³, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may be substituted by one or more radicals R³; two or more radicals R² may be linked to one another and form a ring;
R³ is on each occurrence, identically or differently, H, D, F, C(=O)R⁴, CN, Si(R⁴)₃, N(R⁴)₂, P(=O)(R⁴)₂, OR⁴, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may be substituted by one or more radicals R⁴; two or more radicals R³ may be linked to one another and form a ring;
R⁴ is on each occurrence, identically or differently, H, D, F, CN or an aliphatic, aromatic or heteroaromatic organic radical having 1 to 20 C atoms, in which, in addition, one or more H atoms may be replaced by D, F or CN; two or more substituents R⁴ may be linked to one another and form a ring.
n is 0, 1, 2 or 3;
i is on each occurrence, identically or differently, 0 or 1; and
in the benzene rings in the centre of which an N is drawn, in each case one or more ring members -CR¹= may be replaced by -N=.

2. Compound according to Claim 1, **characterised in that**, in the benzene rings in the centre of which an N is drawn, no or precisely one or two ring members -CR¹= may be replaced by -N=.

3. Compound according to Claim 1 or 2, **characterised in that**, in all three benzene rings in formula (I) in the centre of which an N is drawn, no ring member -CR¹= is replaced by N.

4. Compound according to one or more of Claims 1 to 3, **characterised in that** n is equal to 0.

5. Compound according to one or more of Claims 1 to 4, **characterised in that**, for groups of the formula (A-2), no, precisely one or precisely two indices i are equal to 1, and the other indices i are equal to 0.

6. Compound according to one or more of Claims 1 to 5, **characterised in that** X is selected on each occurrence, identically or differently, from a single bond or a group selected from C(R²)₂, C=O, NR², O and S.

7. Compound according to one or more of Claims 1 to 6, **characterised in that** it does not contain a condensed aryl or heteroaryl group having more than 14 aromatic ring atoms as substituent R¹, R², R³ or R⁴.

8. Compound according to one or more of Claims 1 to 7, **characterised in that** it does not contain a further arylamino group, a further bridged arylamino group or a further carbazole group in addition to the group A.

9. Compound according to one or more of Claims 1 to 8, **characterised in that** it corresponds to one of the formulae (I-1) to (I-3)
| | |
|---|---|
| 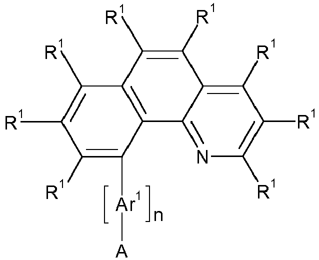 | 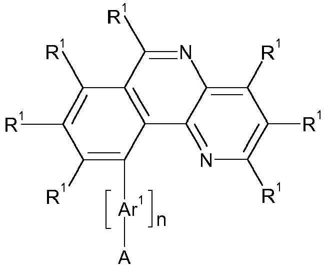 |
| formula (I-1) | formula (I-2) |
| | |
| 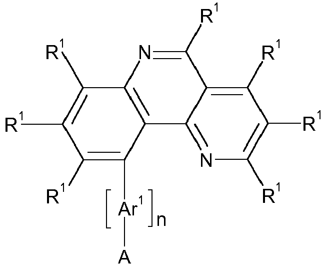 | |
| formula (I-3) | |
where the groups and indices occurring are defined in accordance with one or more of Claims 1 to 8.

10. Process for the preparation of a compound according to one or more of Claims 1 to 9, **characterised in that** a compound of a formula (Int-I) 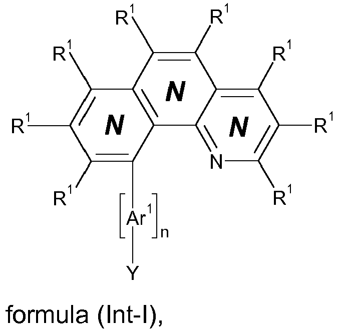 is reacted further in a coupling reaction, where the variable groups and indices occurring are defined as for the compound of the formula (I) according to one or more of Claims 1 to 9, and where, in the benzene rings in the centre of which an N is drawn, in each case one or more ring members -CR¹= may be replaced by -N=, and where Y represents any desired reactive group.

11. Oligomer, polymer or dendrimer containing one or more compounds according to one or more of Claims 1 to 9, where the bonds to the polymer, oligomer or dendrimer may be localised at any desired positions in formula (I) that are substituted by R¹ or R².

12. Formulation comprising at least one compound according to one or more of Claims 1 to 9 and at least one solvent.

13. Use of a compound according to one or more of Claims 1 to 9 in an electronic device.

14. Electronic device comprising anode, cathode and at least one organic layer, where the organic layer comprises at least one compound according to one or more of Claims 1 to 9.

15. Electronic device according to Claim 14, **characterised in that** it is selected from the group consisting of organic integrated circuits, organic field-effect transistors, organic thin-film transistors, organic light-emitting transistors, organic solar cells, organic optical detectors, organic photoreceptors, organic field-quench devices, organic light-emitting electrochemical cells, organic laser diodes and organic electroluminescent devices.

16. Organic electroluminescent device according to Claim 15, **characterised in that** the compound according to one or more of Claims 1 to 9 is present as matrix material in an emitting layer or as hole-transport material in a hole-transporting layer.

## Revendications

1. Composé d'une formule (I) : 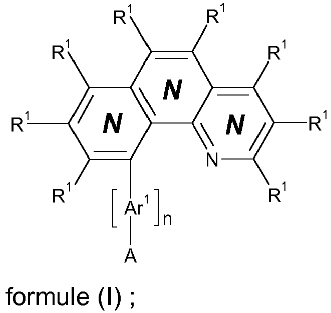 dans laquelle :
A est sélectionné parmi des groupes des formules (A-1) ou (A-2) : 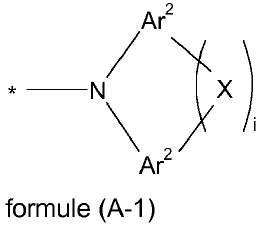 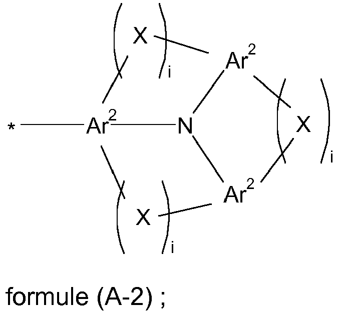 qui sont liés via la liaison qui est repérée à l'aide de * ;
Ar¹ est sélectionné pour chaque occurrence, de manière identique ou différente, parmi benzène, pyridine, pyrimidine, triazine, naphtalène, quinoline, fluorène, spirobifluorène, indénofluorène, carbazole, indénocarbazole, indolocarbazole, dibenzofurane et dibenzothiophène, lesquels peuvent dans chaque cas être substitués par un radical ou par plusieurs radicaux R¹ ;
Ar² est sélectionné pour chaque occurrence, de manière identique ou différente, parmi benzène, biphényle, terphényle, quaterphényle, pyridine, pyrazine, pyridazine, pyrimidine, triazine, naphtalène, quinoline, fluorène, spirobifluorène, indénofluorène, carbazole, indénocarbazole, indolocarbazole, dibenzofurane et dibenzothiophène, lesquels peuvent dans chaque cas être substitués par un radical ou par plusieurs radicaux R¹ ;
X est pour chaque occurrence, de manière identique ou différente, une liaison simple ou un groupe qui est sélectionné parmi BR², C(R²)₂, -C(R²)₂-C(R²)₂-, -C(R²)=C(R²)-, -C(R²)₂-O-, -C(R²)₂-NR²-, Si(R²)₂, C=O, NR², PR², P(=O)R², O, S, S=O ou SO₂,
R¹ est pour chaque occurrence, de manière identique ou différente, H, D, F, C(=O)R³, CN, Si(R³)₃, N(R³)₂, P(=O)(R³)₂, OR³, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique qui comporte de 5 à 30 atomes de cycle aromatique, lequel peut être substitué par un radical ou par plusieurs radicaux R³, ou un système de cycle hétéroaromatique qui comporte de 5 à 30 atomes de cycle aromatique, lequel peut être substitué par un radical ou par plusieurs radicaux R³ et lequel est constitué par des groupes qui sont sélectionnés parmi benzène, naphtalène, anthracène, phénanthrène, pyrène, dihydropyrène, chrysène, pérylène, triphénylène, fluoranthène, benzanthracène, benzophénanthrène, tétracène, pentacène, benzopyrène, furane, benzofurane, isobenzofurane, dibenzofurane, thiophène, benzothiophène, isobenzothiophène, dibenzothiophène, pyrrole, indole, isoindole, carbazole, pyridine, quinoline, isoquinoline, acridine, phénanthridine, phénothiazine, phénoxazine, pyrazole, indazole, imidazole, benzimidazole, naphthimidazole, phénanthrimidazole, pyridimidazole, pyrazinylimidazole, quinoxalinylimidazole, oxazole, benzoxazole, naphthoxazole, arithroxazole, phénanthroxazole, isoxazole, 1,2-thiazole, 1,3-thiazole, benzothiazole, pyridazine, benzopyridazine, pyrimidine, benzopyrimidine, quinoxaline, pyrazine, phénazine, naphtyridine, azacarbazole, benzocarboline, phénanthroline, 1,2,3-triazole, 1,2,4-triazole, benzotriazole, 1,2,3-oxadiazole, 1,2,4-oxa-diazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tétrazole, 1,2,4,5-tétra-zine, 1,2,3,4-tétrazine, 1,2,3,5-tétrazine, purine, ptéridine, indolizine, benzothiadiazole et indénocarbazole ; deux radicaux R¹ ou plus peuvent être liés l'un à l'autre ou les uns aux autres et peuvent former un cycle ;
R¹ est pour chaque occurrence, de manière identique ou différente, H, D, F, C(=O)R3, CN, Si(R³)3, N(R³)2, P(=O)(R³)2, OR³, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupes CH₂ dans les groupes qui ont été mentionnés ci-avant peut/peuvent être remplacé(s) par -R³C=CR³-, -C≡C-, Si(R³)2, C=O, C=NR³, -C(=O)O-, -C(=O)NR³-, NR³, P(=O)(R³), -O-, -S-, SO ou SO₂, ou un système de cycle aromatique ou hétéroaromatique qui comporte de 5 à 30 atomes de cycle aromatique, lequel peut être substitué par un radical ou par plusieurs radicaux R³; deux radicaux R² ou plus peuvent être liés l'un à l'autre ou les uns aux autres et peuvent former un cycle ;
R³ est pour chaque occurrence, de manière identique ou différente, H, D, F, C(=O)R⁴, CN, Si(R⁴)₃, N(R⁴)₂, P(=O)(R⁴)₂, OR⁴, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)2, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique qui comporte de 5 à 30 atomes de cycle aromatique, lequel peut être substitué par un radical ou par plusieurs radicaux R⁴; deux radicaux R³ ou plus peuvent être liés l'un à l'autre ou les uns aux autres et peuvent former un cycle ;
R⁴ est pour chaque occurrence, de manière identique ou différente, H, D, F, CN ou un radical organique aliphatique, aromatique ou hétéroaromatique qui comporte de 1 à 20 atome(s) de C, où, en outre, un ou plusieurs atome(s) de H peut/peuvent être remplacé(s) par D, F ou CN ; deux substituants R⁴ ou plus peuvent être liés l'un à l'autre ou les uns aux autres et peuvent former un cycle ;
n est 0, 1, 2 ou 3 ;
i est pour chaque occurrence, de manière identique ou différente, 0 ou 1 ; et
dans les cycles benzène à partir du centre desquels un N est tiré, dans chaque cas un ou plusieurs élément(s) de cycle -CR¹= peut/ peuvent être remplacé(s) par -N=.

2. Composé selon la revendication 1, **caractérisé en ce que**, dans les cycles benzène à partir du centre desquels un N est tiré, aucun élément de cycle -CR¹= ne peut être remplacé par -N= ou de façon précise, un ou deux élément(s) de cycle -CR¹= peut/peuvent être remplacé(s) par -N=.

3. Composé selon la revendication 1 ou 2, **caractérisé en ce que**, dans l'ensemble des trois cycles benzène dans la formule (I) à partir du centre desquels un N est tiré, aucun élément de cycle -CR¹= n'est remplacé par N.

4. Composé selon une ou plusieurs des revendications 1 à 3, **caractérisé en ce que** n est égal à 0.

5. Composé selon une ou plusieurs des revendications 1 à 4, **caractérisé en ce que**, pour des groupes de la formule (A-2), aucun indice i n'est égal à 1, de façon précise un ou de façon précise deux indice(s) i est égal/sont égaux à 1, et les autres indices i sont égaux à 0.

6. Composé selon une ou plusieurs des revendications 1 à 5, **caractérisé en ce que** X est sélectionné pour chaque occurrence, de manière identique ou différente, parmi une liaison simple ou un groupe qui est sélectionné parmi C(R²)₂, C=O, NR², O et S.

7. Composé selon une ou plusieurs des revendications 1 à 6, **caractérisé en ce qu'**il ne contient pas de groupe aryle ou hétéroaryle condensé comportant plus de 14 atomes de cycle aromatique en tant que substituant R¹, R², R³ ou R⁴.

8. Composé selon une ou plusieurs des revendications 1 à 7, **caractérisé en ce qu'**il ne contient pas un autre groupe arylamino, un autre groupe arylamino ponté ou un autre groupe carbazole en plus du groupe A.

9. Composé selon une ou plusieurs des revendications 1 à 8, **caractérisé en ce qu'**il correspond à l'une des formules (I-1) à (I-3) :
| | |
|---|---|
| 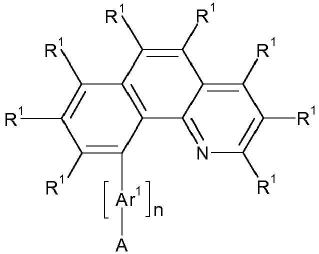 | 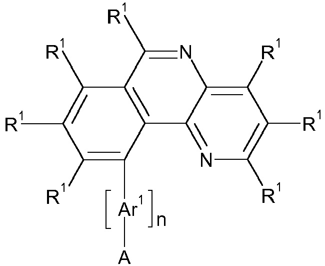 |
| formule (I-1) | formule (I-2) |
| 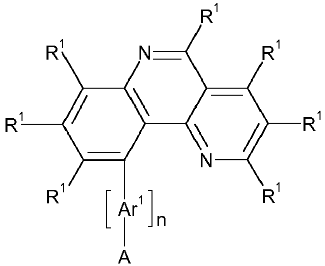 | |
| formule (I-3) | |
dans lesquelles les groupes et les indices rencontrés sont définis conformément à une ou plusieurs des revendications 1 à 8.

10. Procédé pour la préparation d'un composé selon une ou plusieurs des revendications 1 à 9, **caractérisé en ce que** le composé d'une formule (Int-I) : 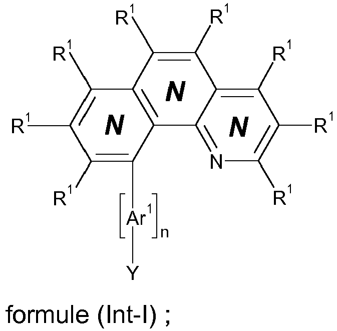 est amené à réagir en outre selon une réaction de couplage ;
où les groupes variables et les indices afférents rencontrés sont définis tels que pour le composé de la formule (I) conformément à une ou plusieurs des revendications 1 à 9, et où, dans les cycles benzène à partir du centre desquels un N est tiré, dans chaque cas un ou plusieurs élément(s) de cycle -CR¹= peut/peuvent être remplacé(s) par -N=, et où Y représente un quelconque groupe réactif souhaité.

11. Oligomère, polymère ou dendrimère contenant un ou plusieurs composé(s) selon une ou plusieurs des revendications 1 à 9, où les liaisons sur le polymère, l'oligomère ou le dendrimère peuvent être situées au niveau de n'importe quelles positions souhaitées dans la formule (I) qui sont substituées par R¹ ou R².

12. Formulation comprenant au moins un composé selon une ou plusieurs des revendications 1 à 9 et au moins un solvant.

13. Utilisation d'un composé selon une ou plusieurs des revendications 1 à 9 dans un dispositif électronique.

14. Dispositif électronique comprenant une anode, une cathode et au moins une couche organique, dans lequel la couche organique comprend au moins un composé selon une ou plusieurs des revendications 1 à 9.

15. Dispositif électronique selon la revendication 14, **caractérisé en ce qu'**il est sélectionné parmi le groupe qui est constitué par les circuits intégrés organiques, les transistors à effet de champ organiques, les transistors à film mince organiques, les transistors à émission de lumière ou luminescents organiques, les cellules solaires organiques, les détecteurs optiques organiques, les photorécepteurs organiques, les dispositifs à extinction de champ organiques, les cellules électrochimiques à émission de lumière ou luminescentes organiques, les diodes laser organiques et les dispositifs électroluminescents organiques.

16. Dispositif électroluminescent organique selon la revendication 15, **caractérisé en ce que** le composé selon une ou plusieurs des revendications 1 à 9 est présent en tant que matériau de matrice dans une couche d'émission ou en tant que matériau de transport de trous dans une couche de transport de trous.
